(19) Europäisches Patentamt
European Patent Office
Office européen des brevets

(11) Numéro de publication: **0 282 365 B1**

# (12) FASCICULE DE BREVET EUROPEEN

(45) Date de publication de fascicule du brevet: **12.05.93**

(51) Int. Cl.⁵: **C07D 513/04**, C07D 519/00, A61K 31/54, //(C07D513/04, 279:00,205:00),(C07D519/00, 513:00,495:00),(C07D519/00, 513:00,455:00)

(21) Numéro de dépôt: **88400239.5**

(22) Date de dépôt: **02.02.88**

Le dossier contient des informations techniques présentées postérieurement au dépôt de la demande et ne figurant pas dans le présent fascicule.

## (54) Dérivés de l'acide I–déthia 2–thia céphalosporanique, leur procédé de préparation, leur application comme médicaments et les compositions pharmaceutiques les contenant.

(30) Priorité: **06.02.87 FR 8701457**

(43) Date de publication de la demande: **14.09.88 Bulletin 88/37**

(45) Mention de la délivrance du brevet: **12.05.93 Bulletin 93/19**

(84) Etats contractants désignés: **AT BE CH DE ES FR GB IT LI NL SE**

(56) Documents cités:
**EP–A– 153 229**
**EP–A– 182 301**
**EP–A– 214 020**
**FR–A– 2 505 840**
**EP–A– 176 329**
**EP–A– 186 586**
**FR–A– 2 324 639**

(73) Titulaire: **ROUSSEL–UCLAF**
**35, Boulevard des Invalides**
**F–75007 Paris(FR)**

(72) Inventeur: **Costerousse, Germain**
**10, rue des Réservoirs**
**F–94410 Saint Maurice(FR)**
Inventeur: **Gouin d'Ambrieres, Solange**
**17, rue Lis Franc**
**F–75020 Paris(FR)**
Inventeur: **Teutsch, Jean–Georges**
**Résidence Lavoisier Bât 3 3, Rue Lavoisier**
**F–93500 Pantin(FR)**

(74) Mandataire: **Bourgouin, André et al**
**Département des Brevets ROUSSEL UCLAF**
**111, route de Noisy B.P. no 9**
**F–93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

## Description

Dans le brevet français n° 2 559 486 déposé le 13 février 1984, et correspondant au brevet européen EP 0 153 299, la société demanderesse a décrit de nouveaux dérivés de l'acide 1−déthia, 2−thia céphalosporanique et leurs sels, leur procédé de préparation et leur application comme médicaments.

Les produits décrits dans ce brevet répondent à la formule générale :

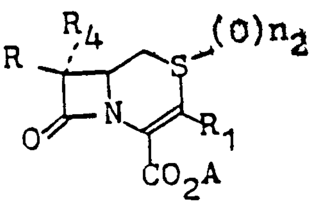

dans laquelle R représente soit un radical

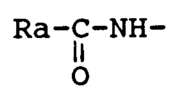

dans lequel Ra représente un radical organique, soit un radical

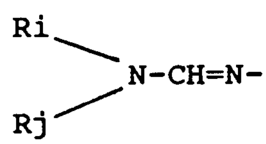

dans lequel Ri et Rj identiques ou différents représentent un atome d'hydrogène ou un radical hydrocarboné aliphatique ou aromatique, un radical hétérocyclique ou Ri et Rj forment avec l'atome d'azote auquel ils sont liés, un radical cyclique éventuellement substitué, soit un radical Rb−NH− dans lequel Rb représente un radical aryle carbocyclique ou hétérocyclique éventuellement substitué, $R_1$ représente, soit :

a) un radical $-Z-R_2$, dans lequel $R_2$ est un radical alkyle, alkényle ou alkynyle éventuellement substitué ou interrompu par un hétéroatome et Z représente un atome de soufre éventuellement oxydé, de sélénium, d'oxygène ou −NH−,

b) un radical $Za-R_3$, dans lequel $R_3$ représente un radical aryle carbocyclique ou hétérocyclique éventuellement substitué ou un ammonium quaternaire éventuellement substitué et Za représente un radical méthylène, un atome de soufre, de sélénium, d'oxygène ou −NH−, soit Za représente une simple liaison, soit Za représente un radical $-CH_2-S-$,

c) un radical alkyle, alkényle ou alkynyle ayant de 2 à 8 atomes de carbone éventuellement substitué ou interrompu par un hétéroatome,

d) un atome d'halogène, un radical nitrile, azido, thiocyanato ou isothiocyanato,

e) un radical azidométhyle, amino ou amino mono ou disubstitué méthyle, thiocyanato méthyle, isothiocyanatométhyle, carbamoyloxyméthyle,

$R_4$ représente un atome d'hydrogène ou un radical méthoxy,

A représente un atome d'hydrogène, un équivalent de métal alcalin, alcalino−terreux, de magnésium, d'ammonium ou d'une base organique aminée ou A représente un groupement ester ou $CO_2A$ représente un groupement $CO_2^{\ominus}$,

$n_2$ représente un entier égal à 0, 1 ou 2.

Les produits répondant à la formule générale ci−dessus peuvent se présenter sous forme racémique ou optiquement active.

La société demanderesse a maintenant découvert qu'une classe particulière de produits présente des propriétés pharmacologiques particulièrement intéressantes.

C'est ainsi que les produits de la présente demande sont plus actifs que les produits du brevet européen EP 0 153 299.

Des produits comportant également un noyau 2-thiocéphème sont également décrits dans les brevets français BF 2,324,639 et 2,505,840 et européen EP 0.214.029. Cependant les produits décrits dans ces brevets sont plus éloignés des produits de la présente demande que ne le sont les produits du brevet EP 0.153.299.

La présente demande a ainsi pour objet les produits répondant à la formule générale (I') :

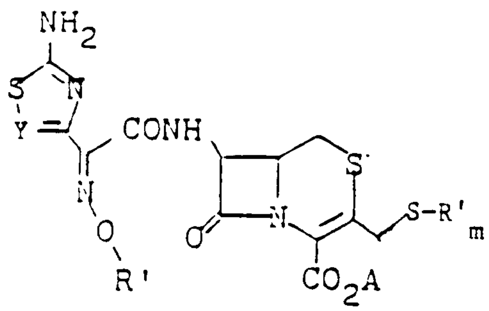

(I')

isomère syn

dans laquelle R' représente un atome d'hydrogène ou un radical alkyle, alkényle, alkynyle ou cycloalkyle ayant au plus 6 atomes de carbone, éventuellement substitué par les radicaux halogène, cyano, carbamoyle, nitro, amino, hydroxy, mercapto, alkylthio, oxo, alkoxy, carboxyle libre, estérifié ou salifié, $R'_m$ représente soit un radical pyridinium éventuellement substitué par un ou plusieurs radicaux choisis parmi les radicaux alkyle ayant de 1 à 4 atomes de carbone, méthoxy, éthoxy, les atomes d'halogènes, les radicaux carboxy alkyle éventuellement estérifiés, le radical carbamoyle, les radicaux thioacétylalkyle, thiocarbamoylalkyle, haloalkyle, cyanoalkyle, alkényle ayant au plus 4 atomes de carbone, alkylthio alkyle et thioalkyle, ledit radical pyridinium pouvant éventuellement être partiellement hydrogéné

soit un radical quinoléinyle, isoquinoléinyle, cinnolinyle, quinazolinyle, pyridino [1,2-a] pyrimidinyle

soit un radical choisi parmi les radicaux :

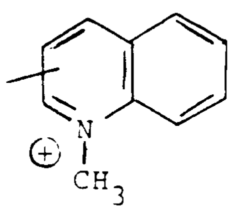 ; 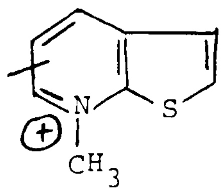 ; 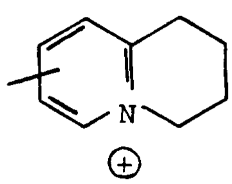 ; 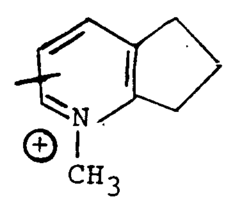

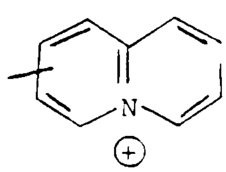

ces radicaux étant liés à l'atome de soufre par un atome de carbone, Y représente un radical méthine ou un atome d'azote, A représente un atome d'hydrogène, un équivalent de métal alcalin, alcalino-terreux, de magnésium, d'ammonium ou d'une base organique aminée ou A représente un groupement ester ou $CO_2A$ représente un groupement $CO_2^{\ominus}$, ainsi que les sels des produits de formule (I') avec les acides minéraux ou organiques.

Parmi les valeurs de R', on peut citer les valeurs hydrogène, alkyle, alkényle, alkynyle et notamment les valeurs $-CH_3$, $-CH_2-CH_3$, $-CHF_2$, $-CH_2-CF_3$, $-CH_2CN$, $-CH_2-CONH_2$, $-CH_2CO_2H$, $-CH(CH_3)-CO_2H$, $-C(CH_3)_2-CO_2H$,

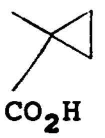, 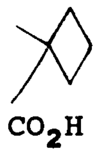, 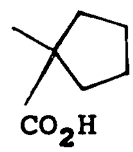,

$-CH_2-CH=CH_2$, $-CH_2-C(=CH_2)-CO_2H$, $-CH_2-CH=CH-CO_2H$, $-CH=CH-CH(CH_3)-CO_2H$,

$-CH_2-C{\equiv}CH$, $-CH_2-CH_2-NH_2$, $-CH_2-CF_2-CO_2H$.

Parmi les valeur de R', on préfère les valeurs méthyle, hydrogène, éthyle, allyle, 1-méthyl 1-carboxyéthyle, carboxyméthyle ou difluorométhyle.

Le radical pyridinium, partiellement hydrogéné, que représente $R'_m$ est éventuellement substitué par un ou plusieurs radicaux choisis parmi les radicaux alkyle ayant de 1 à 4 atomes de carbone tel que méthyle, éthyle, propyle, isopropyle, butyle, sec-butyle, tert-butyle, les radicaux méthoxy, éthoxy, les atomes d'halogène tel que brome, chlore, iode ou fluor, les radicaux carboxy alkyle éventuellement estérifié ou le radical carbamoyle, les radicaux thioacétylalkyle, thiocarbamoylalkyle, haloalkyle, cyanoalkyle, alkényle ayant au plus 4 atomes de carbone tel que allyle, alkylthioalkyle, thioalkyle.

Parmi les valeurs de $R'_m$ on peut citer outre la valeur pyridinium, les valeurs quinoléinyle, isoquinoléinyle, cinnolinyle, quinazolinyle, pyridino[1,2-a]pyrimidinyle, ainsi que les dérivés partiellement hydrogénés tels que les dihydro ou tétrahydropyridyle.

Parmi les valeurs de $R'_m$, les radicaux suivants constituent des valeurs préférées :

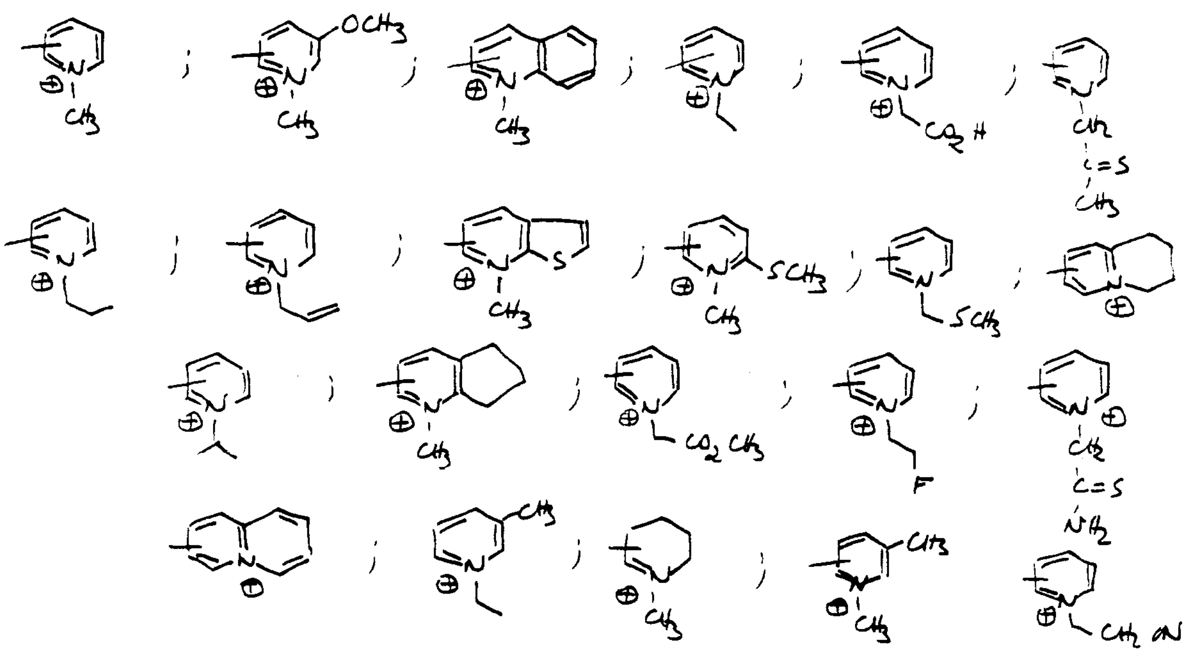

Parmi les valeurs de A, on peut citer un équivalent de sodium, de potassium, de lithium, de calcium, de magnésium, d'ammonium ou d'une base organique. On peut citer, parmi les bases organiques, la méthylamine, la propylamine, la triméthylamine, la diéthylamine, la triéthylamine, la N,N-diméthyléthanolamine, le tris(hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements esters facilement clivables que peut représenter A, les groupements méthoxyméthyle, éthoxyméthyle, isopropyloxyméthyle, alpha-méthoxyéthyle, alpha-éthoxyéthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butyl carbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1 – hexadécnoyloxyéthyle, 1 – propionyloxypropyle, 1 – méthoxycarbonyoxyéthyle, méthoxy – carbonyloxyméthyle, 1 – acétyloxybutyle, 1 – acétyloxyhexyle, 1 – acétyloxyheptyle, phtalidyle, 5,6 – dimé – thoxyphtalidyle, tert – butylcarbonylméthyle, allyle, 2 – chloroallyle, méthoxycarbonylméthyle, benzyle ou tert – butyle.

On peut encore citer entre autres restes de groupements esters que peut représenter A, les groupe – ments méthoxyéthoxyméthyle, diméthylaminoéthyle, cyanométhyle, tert – butyloxycarbonyméthyle, 2,2 – éthylènedioxyéthyle, cyanoéthyle, 2,2 – diméthoxyéthyle, 2 – chloroéthoxyméthyle, 2 – hydroxyéthoxyéthyle, 2,3 – époxypropyle, 3 – diméthylamino, 2 – hydroxypropyle, 2 – hydroxyéthyle, 2 – méthylaminoéthoxymé – thyle, 2 – aminoéthoxyméthyle, 3 – méthoxy 2,4 – thiadiazol 5 – yle, 2 – tétrahydropyranyle, 2 – méthoxyprop – 2 – yle, 1 – hydroxyprop – 2 – yle, isopropyle, carbamoylméthyle, chlorométhyle, 2 – chloroé – thyle, acétylméthyle, 2 – méthylthioéthyle ou thiocyanatométhyle.

On peut encore citer entre autres restes de groupements esters que peut représenter A, les groupe – ments 2 – chloro 1 – acétyloxyéthyle, 2 – bromo 1 – acétyloxyéthyle, 2 – fluoro 1 – acétyloxyéthyle, 2 – mé – thoxy 1 – acétyloxyéthyle, 2 – méthyl 1 – acétyloxypropyle, 2 – acétyloxyprop – 2 – yle, 1 – méthoxyacéty – loxyéthyle, 1 – acétylcarbonyloxyéthyle, 1 – hydroxyacétyloxyéthyle, 1 – formyl carbonyloxyéthyle, 1 – (2 – thiényl) carbonyloxyéthyle, 1 – (2 – furyl) carbonyl oxyéthyle, 1 – (5 – nitro 2 – furyl) carbonyloxyéthyle, 1 – (2 – pyrrolyl) carbonyl oxyéthyle, 1 – (propionyloxycarbonyloxy) éthyle, 1 – (propyloxycarbonyloxy) éthyle, 1 – (isopropyloxycarbonyloxy) éthyle, 1 – (méthoxyéthoxycarbonyloxy) éthyle, 1 – (allyloxycarbonyloxy) éthyle, 1 – (méthoxyéthoxycarbonyloxy) éthyle, 1 – (allyloxycarbonyloxy) éthyle, 1 – (2,3 – époxy) propy – loxycarbonyloxy éthyle, 1 – (2 – furyl) méthyloxycarbonyloxy éthyle, 1 – (2 – fluoro) éthyloxycarbonyloxy éthyle, 1 – (méthoxycarbonyloxy) propyle, (2 – méthoxycarbonyloxy) prop – 2 – yle, (méthoxycarbonyloxy) chlorométhyle, 1 – (méthoxycarbonyloxy) 2 – chloroéthyle, 1 – (méthoxycarbonyloxy) 2 – méthoxyéthyle, 1 – (méthoxycarbonyloxy) 1 – allyle. A peut également représenter le groupement :

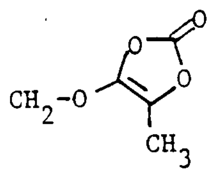

Les produits de formule (I) peuvent également se présenter sous forme de sels d'acides organiques ou minéraux.

Parmi les acides avec lesquels on peut salifier le ou les groupements amino ou le groupement ammonium quaternaire des produits (I), on peut citer entre autres, les acides acétique, trifluoroacétique, maléïque, tartrique, méthanesulfonique, benzène sulfonique, p – toluène sulfonique, trifluorométhanesulfoni – que, formique, phosphorique, sulfurique, chlorhydrique, bromhydrique, iodhydrique.

Parmi les valeurs de A, on peut notamment citer les groupements esters de formule :

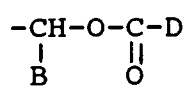

dans lesquels B représente un atome d'hydrogène ou un radical alkyle linéaire ou ramifié éventuellement substitué, renfermant de 1 à 5 atomes de carbone et D représente un radical alcoyle ou alcoxy linéaire ou ramifié, éventuellement substitué, renfermant de 1 à 15 atomes de carbone et notamment de 1 à 5 atomes de carbone, et plus particulièrement les groupements esters dans lesquels B représente un atome d'hydrogène ou un radical méthyle ou éthyle et D représente un radical méthyle, éthyle, méthoxy ouu éthoxy.

Parmi les valeurs de A, on peut encore citer le radical

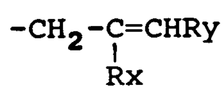

dans lequel Rx représente les valeurs hydrogène, alkyle, notamment méthyle, éthyle, halogène, notamment chlore, et Ry représente les valeurs hydrogène, halogène, aryle, notamment phényle, éventuellement substitué par méthyle, méthoxy ou halogène, ou Ry représente la valeur alkyle éventuellement substitué par acyloxy, par alcoxycarbonyl, par halogène.

Parmi les valeurs de A, on peut encore citer le radical

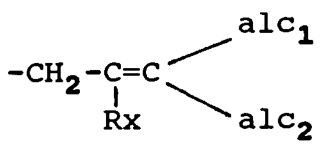

,

dans lequel Rx est défini comme ci – dessus et $alc_1$ et $alc_2$, identiques ou différents, représentent un radical alkyle renfermant de 1 à 4 atomes de carbone.

Parmi les produits précités de formule (I'), on préfère les produits dans lesquels R' représente un atome d'hydrogène, ou un radical méthyle ou isopropyle éventuellement substitué par un radical carboxy, carbomoyle, nitrile, méthylthio ou méthoxy, un radical fluorométhyle, difluorométhyle ou trifluorométhyle, un radical phényle, un radical cyclobutyle, ou cyclopropyle éventuellement substitué par un radical carboxy, un radical alkyle éventuellement substitué par un atome de chlore ou de brome, un radical propényle éventuellement substitué par un atome d'halogène tel qu'un atome de brome, un radical cyclopropyle méthyle.

L'invention a plus spécialement pour objet les produits de formule (I") :

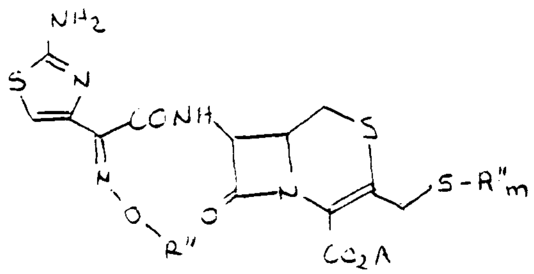

(I")

dans laquelle R" représente un radical méthyle, fluorométhyle ou difluorométhyle, $R"_m$ représente un radical

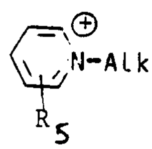

dans lequel Alk représente un radical alkyle ayant de 1 à 4 atomes de carbone, $R_5$ représente un atome d'hydrogène, un radical alkyle ou alkylthio ayant de 1 à 4 atomes de carbone, ledit radical $R"_m$ étant lié à l'atome de soufre par les positions 2 ou 4 et A a la signification indiquée ci – dessus.

Parmi les produits préférés, on peut citer ceux décrits ci – après dans les exemples et notamment les produits suivants :

- Le (6S) (7S) (Z) 4 – [[[7 – [[(2 – amino 4 – thiazolyl) [(difluorométhoxy) imino] acétyl amino] 2 – carboxy 8 – oxo 4 – thia 1 – azabicyclo[4.2.0.]oct – 2 – èn – 3 – yl] méthyl] thio] 1 – méthyl pyridinium,
- Le (6S) (7S) (Z) 4 – [[[7 – [[(2 – amino 4 – thiazolyl) [fluorométhoxy) imino] acétyl] amino] 2 – carboxy 8 – oxo 4 – thia 1 – azabicyclo[4.2.0.]oct – 2 – èn – 3 – yl] méthyl] thio] 1 – méthyl pyridinium,
- Le (6S) (7S) (Z) 2 – [[[7 – [[(2 – amino 4 – thiazolyl) [fluorométhoxy) imino] acétyl] amino] 2 – carboxy 8 – oxo 4 – thia 1 – azabicyclo[4.2.0.]oct – 2 – èn – 3 – yl] méthyl] thio] 1 – méthyl pyridinium,
- Le 4 – [[7 – [[2 – (2 – amino 4 – thiazolyl 2 – (méthoxyimino) acétyl] amino] 2 – carboxy 8 – oxo 4 – thia 1 – azabicyclo[4.2.0.]oct – 2 – èn – 3 – yl] méthyl] thio] 1 – éthyl 3 – méthyl pyridinium (6S) (7S) (Z),

- Le 4–[[[7–[[2–(2–amino 4–thiazolyl 2–(méthoxyimino) acétyl] amino] 2–carboxy 8–oxo 4–thia 1–azabicyclo[4.2.0.]oct–2–èn–3–yl] méthyl] thio] 1,3–diméthyl pyridinium (6S) (7S) (Z),
- Le 4–[[[7–[[2–(2–amino 4–thiazolyl) (méthoxyimino) acétyl] amino] 2–carboxy 8–oxo 4–thia 1–azabicyclo[4.2.0.]oct–2–èn–3–yl] méthyl] thio] 1–méthyl pyridinium (6S) (7S) (Z),
- Le 4–[[[7–[[2–(2–amino 4–thiazolyl) (méthoxyimino) acétyl] amino] 2–carboxy 8–oxo 4–thia 1–azabicyclo[4.2.0.]oct–2–èn–3–yl] méthyl] thio] 1–méthyl 5,6–dihydro pyridinium (6S) (7S) (Z),

sous forme de sel interne ou de sels avec les acides minéraux ou organiques.

L'invention a tout particulièrement pour objet :

- Le (6S) (7S) (Z) 4–[[[7–[[(2–amino 4–thiazolyl [(difluorométhoxy) imino] acétyl amino] 2–carboxy 8–oxo 4–thia 1–azabicyclo[4.2.0.]oct–2–èn–3–yl] méthyl] thio] 1–méthyl pyridinium, sous forme de sel interne ou de sels avec les acides minéraux ou organiques.

L'invention a également pour objet un procédé de préparation des produits de formule générale (I') telle que définie ci–dessus caractérisé en ce que l'on traite un produit de formule (VI') :

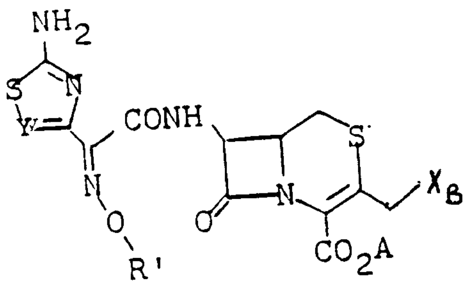

(VI')

dans laquelle Y, A et R' ont la signification indiquée à la revendication 1 et $X_B$ représente un atome d'halogène ou un pseudo halogène par un produit de formule (VIII) :

$R_{AM}$ CS (VIII)

dans laquelle $R_{AM}$ représente le reste du radical $R'_m$ dans lequel l'atome d'azote est lié par des liaisons simples et non polaires et soumet le produit obtenu si nécessaire ou si désiré, à l'une quelconque ou à plusieurs des réactions suivantes, dans un ordre quelconque :

a) coupure par hydrolyse, hydrogénolyse ou par action de la thiourée de tout ou partie du ou des groupements protecteurs;

b) estérification ou salification par une base du ou des groupements carboxy ou sulfo;

c) salification par un acide du ou des groupements amino;

d) dédoublement de la molécule pour obtenir un produit optiquement actif;

e) oxydation de l'atome de soufre en position 2 du cycle isocéphème.

Le substituant X peut représenter un atome d'halogène tel que chlore ou iode, il peut également représenter un pseudo halogène tel que tosyloxy. Dans le produit de formule (VIII), $R_{AM}$ représente le reste du radical $R'_m$ dans lequel l'atome de carbone par lequel ledit radical $R'_m$ est lié à l'atome de soufre est extrait pour la commodité de l'écriture. Dans le radical $R_{AM}$, l'atome d'azote est lié par des liaisons simples et est non polaire.

Par exemple, dans le cas où $R'_m$ représente un radical 2 ou 4–méthyl pyridinium :

$R_{AM}$ représente les radicaux

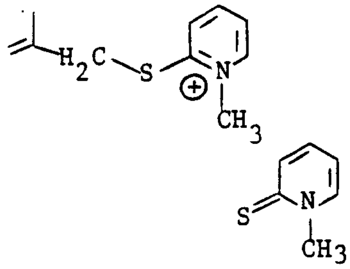

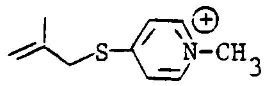

ou

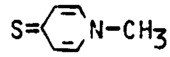

L'action des produits de formule (VIII) sur les produits de formule (VI') est effectuée de préférence à température ambiante dans un solvant tel que l'acétonitrile ou le chlorure de méthylène.

Lorsque les produits obtenus comportent des groupements protecteurs que l'on souhaite éliminer, on traite lesdits produits par un ou plusieurs agents d'hydrolyse ou d'hydrogénolyse ou la thiourée.

La nature des réactifs à mettre en jeu dans tous ces cas est bien connue de l'homme de métier. Des exemples de telles réactions sont donnés plus loin dans la partie expérimentale.

On donne ci-après une émunération non exhaustive des moyens pouvant être mis en oeuvre pour éliminer les différents groupements.

On utilise préférentiellement l'hydrolyse acide pour éliminer les groupements alkoxy et cycloalkoxycarbonyle éventuellement substitués, tels que tert-pentyloxycarbonyle ou tert-butyloxycarbonyle, les groupements aralcoxycarbonyle éventuellement substitués tels que benzyloxycarbonyle, les groupements trityle, benzhydryle, tert-butyle ou 4-méthoxybenzyle.

L'acide que l'on utilise de préférence peut être choisi dans le groupe constitué par les acides chlorhydrique, benzène sulfonique ou para-toluène sulfonique, formique ou trifluoroacétique. On peut cependant utiliser d'autres acides minéraux ou organiques.

L'hydrolyse basique est utilisée préférentiellement pour éliminer les groupements acyle tels que trifluoroacétyle.

La base que l'on utilise de préférence est une base minérale telle que l'hydroxyde de sodium ou de potassium. On peut également utiliser la magnésie, la baryte ou un carbonate ou carbonate acide de métal alcalin tel que les carbonates et carbonates acides de sodium ou de potassium ou d'autres bases.

On peut également utiliser l'acétate de sodium ou de potassium.

L'hydrolyse utilisant l'hydrazine est utilisée de préférence pour éliminer des groupes tels que phtaloyle.

Certains groupements peuvent également être éliminés par le système zinc-acide acétique (pour le groupement trichloroéthyle), les groupements benzhydryle benzyloxycarbonyle sont éliminés de préférence par l'hydrogène, en présence d'un catalyseur.

Le groupement chloroacétyle est éliminé par action de la thiourée en milieu neutre ou acide selon le type de réaction décrit par MASAKI J.A.C.S., 90, 4508, (1968).

On peut également utiliser d'autres méthodes de déprotection connues dans la littérature.

Parmi les groupes préférés, on peut citer les groupements formyle, acétyle, éthoxycarbonyle, mésyle, trifluoroacétyle, chloroacétyle, trityle. On préfère particulièrement les radicaux trityle et chloroacétyle.

L'acide que l'on utilise de préférence est l'acide trifluoroacétique.

La salification des produits peut être effectuée selon les méthodes usuelles.

La salification peut, par exemple, être obtenue par action sur un produit sous forme acide ou sur un solvat, par exemple le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate tri-sodique. On peut également faire appel à des sels d'acides organiques.

Comme sels d'acides organiques, on peut mentionner, par exemple, les sels de sodium d'acides carboxyliques aliphatiques, linéaires ou ramifiés, saturés ou insaturés de 1 à 18 et de préférence de 2 à 10 atomes de carbone. Ces radicaux aliphatiques peuvent être interrompus par un ou plusieurs hétéroatomes tels que l'oxygène ou le soufre ou substitués par des radicaux aryle, comme par exemple : phényle, thiényle, furyle, ou par un ou plusieurs radicaux hydroxyle ou par un ou plusieurs atomes d'halogène tel que fluor, chlore ou brome, préférentiellement chlore, par un ou plusieurs radicaux carboxyliques ou

alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques des acides aromatiques suffisamment solubles comme par exemple des acides benzoïques substitués, de préférence, par des radicaux alkyles inférieurs.

Comme exemples de tels acides organiques, on peut mentionner : les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthylique de l'acide adipique, les acides hexanoïque, heptanoïque, décanoïque, oléïque, stéarique, palmitique, 3-hydroxypropionique, 3-méthoxypropionique, 3-méthylthiobutyrique, 4-chlorobutyrique, 4-phénylbutyrique, 3-phénoxybutyrique, 4-éthylbenzoïque, 1-propylbenzoïque.

On utilise cependant de préférence comme sels de sodium l'acétate de sodium, le 2-éthyl hexanoate de sodium ou le diéthyl acétate de sodium.

La salification peut également être obtenus par action d'une base organique comme la triéthylamine, la diéthylamine, la triméthylamine, la propylamine, la N,N-diméthyl-éthanolamine, le tris(hydroxyméthyl) amino méthane, la méthylamine, l'éthanolamine, la pyridine, la picoline, la dicyclohexylamine, la morpholine et la benzylamine.

Elle peut également être obtenue par action de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opère en général en faisant réagir l'acide de formule (I') avec un dérivé de formule :

$Z-R_s$

dans laquelle Z représente un radical hydroxyle ou un atome d'halogène tel que le chlore, le brome, l'iode et $R_s$ désigne le groupement ester à introduire, groupement dont une liste non exhaustive figure ci-dessus. Dans certains cas, il peut être avantageux d'effectuer une estérification sur un produit dont l'amine est bloquée avant d'enlever le groupement protecteur de l'amine.

L'éventuel dédoublement des composés de formule générale (I) peut être effectué au moyen d'un acide organique carboxylique ou sulfonique optiquement actif comme l'acide tartrique, dibenzoyltartrique, camphosulfonique ou glutamine, la décomposition du sel ainsi obtenue étant effectuée au moyen d'une base minérale telle que le bicarbonate de sodium ou d'une base organique telle qu'une amine tertiaire comme la triéthylamine. De plus, on peut éventuellement utiliser une base optiquement active.

L'oxydation éventuelle des produits de formule (I) peut être effectuée à l'aide d'oxygène, de péroxydes, d'hydropéroxydes, de péracides ou d'eau oxygénée. La réaction est avantageusement sensibilisée à la lumière. Ces réactifs peuvent être en mélange avec des acides organiques ou minéraux. On utilise de préférence l'acide métachloro perbenzoïque. Les conditions réactionnelles sont connues de l'homme de métier. On trouve de telles conditions exposées par exemple dans le brevet français 2.387.234.

Les produits de formule générale (I') ainsi que leurs sels pharmaceutiquement acceptables, possèdent une très bonne activité antibiotique sur les bactéries gram (+) telles que les staphylocoques, les streptocoques et, notamment, sur les staphylocoques phénicillino-résistants. Leur efficacité sur les bactéries gram (-), notamment, sur les bactéries coliformes, les klebsiella, les salmonella et les proteus est particulièrement remarquable.

Ces propriétés rendent aptes lesdits produits à être utilisés comme médicaments dans le traitement des affectations à germes sensibles et, notamment, dans celui des staphylococcies, telles que septicémies à staphycocoques, staphylococcies malignes de la face ou cutanée, pyodermites, plaies septiques ou suppurantes, anthrax, phlegmons, érésipèles, staphylococcies aigües primitives ou post grippales, bronchopneumonies, suppuration pulmonaire.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

La présente invention a donc également pour objet, à titre de médicaments et notamment de médicaments antibiotiques, les produits de formule (I'), tels que définis ci-dessus, ainsi que leurs sels d'acide, pharmaceutiquement acceptables.

Parmi ces produits l'invention a particulièrement pour objet à titre de médicaments et notamment de médicaments antibiotiques les produits précités de formule (I") :

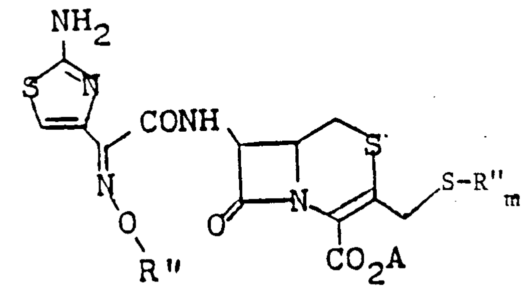

(I")

dans laquelle R" représente un radical méthyle, fluorométhyle ou difluorométhyle, $R''_m$ représente un radical

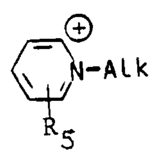

dans lequel Alk représente un radical alkyle ayant de 1 à 4 atomes de carbone, $R_5$ représente un atome d'hydrogène, un radical alkyle ou alkylthio ayant de 1 à 4 atomes de carbone, ledit radical $R''_m$ étant lié à l'atome de soufre par les positions 2 ou 4 et A a la signification indiquée ci-dessus.

L'invention a tout particulièrement pour objet, à titre de médicament et notamment à titre de médicaments antibiotiques, les produits décrits ci-après dans les exemples et notamment :

- Le (6S) (7S) (Z) 4-[[[7-[[(2-amino 4-thiazolyl) [(difluorométhoxy) imino] acétyl amino] 2-carboxy 8-oxo 4-thia 1-azabicyclo[4.2.0.]oct-2-èn-3-yl] méthyl] thio] 1-méthyl pyridinium,
- Le (6S) (7S) (Z) 4-[[[7-[[(2-amino 4-thiazolyl) [fluorométhoxy) imino] acétyl] amino] 2-carboxy 8-oxo 4-thia 1-azabicyclo[4.2.0.]oct-2-èn-3-yl] méthyl] thio] 1-méthyl pyridinium,
- Le (6S) (7S) (Z) 2-[[[7-[[(2-amino 4-thiazolyl) [fluorométhoxy) imino] acétyl] amino] 2-carboxy 8-oxo 4-thia 1-azabicyclo[4.2.0.]oct-2-èn-3-yl] méthyl] thio] 1-méthyl pyridinium,
- Le 4-[[7-[[2-(2-amino 4-thiazolyl 2-(méthoxyimino) acétyl] amino] 2-carboxy 8-oxo 4-thia 1-azabicyclo[4.2.0.]oct-2-èn-3-yl] méthyl] thio] 1-éthyl 3-méthyl pyridinium (6S) (7S) (Z),
- Le 4-[[[7-[[2-(2-amino 4-thiazolyl 2-(méthoxyimino) acétyl] amino] 2-carboxy 8-oxo 4-thia 1-azabicyclo[4.2.0.]oct-2-èn-3-yl] méthyl] thio] 1,3-diméthyl pyridinium (6S) (7S) (Z),
- Le 4-[[[7-[[2-(2-amino 4-thiazolyl) (méthoxyimino) acétyl] amino] 2-carboxy 8-oxo 4-thia 1-azabicyclo[4.2.0.]oct-2-èn-3-yl] méthyl] thio] 1-méthyl pyridinium (6S) (7S) (Z),
- Le 4-[[[7-[[2-(2-amino 4-thiazolyl) (méthoxyimino) acétyl] amino] 2-carboxy 8-oxo 4-thia 1-azabicyclo[4.2.0.]oct-2-èn-3-yl] méthyl] thio] 1-méthyl 5,6-dihydro pyridinium (6S) (7S) (Z),

sous forme de sel interne ou de sels avec les acides minéraux ou organiques.

L'invention s'étend aux compositions pharmaceutiques renfermant comme principe actif au moins un des médicaments définis ci-dessus.

Ces compositions peuvent être administrées par voie buccale, rectale, parentérale, intramusculaire ou par voie locale en application topique sur la peau et les muqueuses.

En particulier, les produits de formule (I') dans laquelle A représente un groupement ester clivable tel que l'ester de propionyloxyméthyle peuvent être administrés par voie orale.

Elles peuvent être solides ou liquide et se présenter sous les formes pharmaceutiques couramment utilisées en médecine humaine comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des

excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent, notamment, se présenter sous forme d'une poudre destinées à être dissoute extemporanément dans un véhicule approprié, par exemple de l'eau stérile apyrogène.

La dose administrée est variables selon l'affection traitée, le sujet en cause, la voie d'administration et le produit considéré. Elle peut être, par exemple, comprise entre 0,500 g et 1 g trois fois par jour, par voie intramusculaire pour les produits des exemples 2 ou 3.

Les produits de formule (I') peuvent également être utilisés comme désinfectants des intruments chirurgicaux.

Les produits de formule (VI') utilisés au départ du procédé de préparation des produits de formule (I') et dans lesquels $n_1$ représente le nombre 0 peuvent être préparés selon le procédé décrit dans le brevet français 2.559.486.

Les produits de formule (VIII) peuvent être préparés selon les méthodes usuelles à partir des produits $R_{AM}CO$ correspondants.

Les exemples suivants illustrent l'invention sans toutefois la limiter.

**Exemple 1 : Iodure (60%), trifluoroacétate (40%) de 2-[[3-[7-[[(2-amino-4-thiazolyl) [-(fluorométhoxy) imino] acétyl] amino] 2-carboxy 8-oxo 4-thia 1-azabicyclo [4.2.0.] oct-2-èn-3-yl] 2 (E) propényl] thio] 1-méthyl pyridinium (trifluoroacétate)**.

1) Echange Hydroxyle-iode.

On mélange sous atmosphère inerte 200 mg de 3-hydroxy 2 (E) propényl 7-[2-[2-triphényl méthylamino 4-thiazolyl] [2-(difluorométhoxy) imino] acétyl] amino] 8-oxo 4-thia 1-azabicyclo [4.2.0.] oct-2-èn-3-yl] 2-carboxylate de 1,1-diméthyléthyl (6S) (7S) (Z), 4 cm3 de chlorure de méthylène, 195 mg d'iodure de tétrabutylammonium, 123 ul de 2,6-lutidine, introduit à -70¤C en 10 minutes environ 1,8 cm3 de solution de trifluorométhane sulfonate à 5% dans le chlorure de méthylène, agite à -70¤C pendant 15 minutes, concentre à sec par distillation sous pression réduite à -70¤C puis à 20¤C, chromatographie le résidu sur silice en éluant avec un mélange de chlorure de méthylène et d'acétate d'éthyle (90/10) et obtient 170 mg de produit attendu.

2) Echange iode-base.

On mélange sous atmosphère inerte 170 mg de dérivé iodé obtenu au paragraphe 1 précédent, 24 mg de 1-méthyl 2-thio pyridine, 3 cm3 d'acétonitrile, agite à 20¤C pendant 30 minutes, concentre à sec par distillation sous pression réduite, chromatographie le résidu sur silice sous pression d'azote en éluant avec un mélange de chlorure de méthylène et de méthanol (90/10) et obtient 114 mg de produit attendu.

Le 3-hydroxy 2 (E) propényl 7-[[[2-(2-tritylamino 4-thiazolyl) 2-(difluorométhoxyimino) acétyl] amino] 8-oxo 4-thia 1-azabicyclo [4.2.0.] oct-2-èn-3-yl] 2-carboxylate de 1,1-diméthyléthyle (6S) (7S) (Z) a été préparé d'une manière analogue à celle décrite ci-dessous pour le "méthoxyimino" en partant du dérivé correspondant.

**Préparation du 7-[[2-(2-triphénylméthylaminothiazol-4-yl) 2 (Z) méthoxy imino acétyl] amino] 3-[(3-hydroxy propèn-1(E)-yl) 8-oxo 4-thia 1-azabicyclo [4.2.0.] oct-2-èn-2-carboxylate de (1,1-diméthyléthyle) et, 7-[[2-(2-triphénylméthylaminothiazol-4-yl) 2 (Z) méthoxy imino acétyl] amino] 3-[(3-hydroxy propèn-1(Z)-yl) 8-oxo 4-thia 1-azabicyclo [4.2.0.] oct-2-èn-2-carboxylate de (1,1-diméthyléthyle)**.

**Stade A** : Chlorure de [7-[[(2-triphénylméthylaminothiazol-4-yl) 2 (Z) méthoxy imino) acétyl] amino] 2-[(1,1-diméthyléthyl) oxycarbonyl 8-oxo 4-thia 1-azabicyclo [4.2.0.] oct-2-èn-3-yl] méthyl tri-phényl phosphonium (6S, 7S).

On dissout 2, 182 g de 7-[[2-(2-triphénylméthylaminothiazol-4-yl) 2 (Z) méthoxy imino acétyl] amino] 3-chlorométhyl 8-oxo 4-thia 1-azabicyclo [4.2.0.] oct-2-èn-2-carboxylate de 1,1-diméthyléthyle (6S, 7S), 1,679 g de triphényl phosphine dans 24 cm3 de tétrahydrofuranne, ajoute 14,1 g de silice, distille le tétrahydrofuranne pendant 2 heures, refroidit, agite pendant 26 heures à 20¤C, chromatographie sur silice en éluant par un mélange de dichlorométhane et de méthanol (90/10) et obtient 1,89 g de produit recherché.

**Stade B :** 7–//2–(2–triphénylméthylamino thiazol–4–yl/2(Z) méthoxy imino acétyl/amino 3–/3–/(1,1–diméthyl éthyl)diméthyl silyl/oxy/ propèn–1–yl/ 8–oxo 4–thia 1–azabicyclo /4.2.0/ oct–2–èn 2–carboxylate de (1,1–diméthyl) éthyl 6S,7S.

On mélange 1,89 g de chlorhydrate de 7–//2–(2–triphénylméthylamino thiazol–4–yl/ 2(Z) méthoxy iminoacétyl/amino/ 3–méthyl triphényl phosphonium 8–oxo 4–thia 1–azabicyclo /4.2.0/ oct–2–èn 2–carboxylate de (1,1–diméthyl) éthyle, 30 cm3 de dichlorométhane, 0,68 cm3 de 2/1,1–diméthyléthyl diméthylsilyl oxy/ acétaldéhyde, 0,53 cm3 de triéthylamine, agite à + 20¤C pendant 14 heures, chromatographie la solution sur silice en éluant par un mélange de dichlorométhane et d'acétate d'éthyle (9/1) et isole 1,305 g de produit attendu contenant un mélange d'isomère E (2/3) et Z (1/3).

Spectre de RMN ($CDCl_3$)

0,90 – 0,92 ppm SitBu ; 1,50 – 1,53 ppm $CO_2$tBu ; 2,95 – 3,11 ppm 20 $CH_2S$ ; 4,05 ppm $OCH_3$ ; 4,31 – 4,37 ppm $CH_2O$ 4,11 ppm $H_6$ ; 5,4 à 5,5 ppm $H_7$ 5,75 – 5,87 ppm et 6,11 – 6,24 ppm Z(1/3) ; 6,17 – 6,34 ppm et 6,97 – 7,16 ppm E(2/3) ; 6,50 – 6,56 ppm $H_5$ thiazole syn ; 7,34 ppm Ø $_3$.

**Stade C :** 7–//2–(2–triphénylméthylamino thiazol–4–yl) 2(Z)méthoxy imino acétyl/ amino/ 3–/(3–hydroxy propèn–1(E) yl) 8–oxo 4–thia 1–azabicyclo /4.2.0/ oct–2–èn 2–carboxylate de (1,1–diméthyl éthyle) et,

7–//2–(2–triphénylméthylamino thiazol–4–yl) 2(Z) méthoxy imino acétyl/ amino/ 3–/3–hydroxy propèn–1 (Z) yl/ 8–oxo 4–thia 1–azabicyclo /4.2.0/ oct–2–èn 2–carboxylate de (1,1–diméthyl éthyle).

On dissout 1,305 g de produit obtenu au stade B de l'exemple 1 dans 15 cm3 d'acétone, ajoute 2 cm3 de solution aqueuse normale d'acide chlorhydrique, agite pendant 2 heures et 30 minutes, concentre à sec par distillation sous pression réduite, ajoute de l'eau bicarbonatée, extrait au dichlorométhane, concentre à sec par distillation sous pression réduite, chromatographie le résidu sur silice en éluant par un mélange de dichlorométhane et d'acétate d'éthyle (1/1) et isole :
536 mg d'isomère E et,
369 mg d'isomère Z du produit attendu.

Isomère (E).

Spectre U.V. : éthanol.

| | | |
|---|---|---|
| Max. | 231 nm $E_1^1$ | = 497 $\epsilon$ = 32 200. |
| Inflexion | 258 nm $E_1^1$ | = 290 ; |
| Inflexion | 264 nm $E_1^1$ | = 264 ; |
| Inflexion | 270 nm $E_1^1$ | = 240 ; |
| Max. | 320 nm $E_1^1$ | = 245 ; $\epsilon$ 18 100. |

Isomère Z.

Spectre U.V. (éthanol)

Inflexion 230 nm $E_1^1$ = 411 ; Inflexion 260 nm $E_1^1$ = 220 ;
Inflexion 265 nm $E_1^1$ = 198 ; Inflexion 271 nm $E_1^1$ = 176 ;
Max. 308 nm $E_1^1$ = 194$\epsilon$ = 14 300.

2) Détritylation – déterbutylation.

On mélange sous atmosphère inerte 144 mg de produit obtenu au paragraphe 2 précédent, 1,8 cm3 d'une solution aqueuse d'acide formique à 66%, agite à 60¤C pendant 1 heure et 30 minutes, refroidit,

élimine par filtration l'insoluble formé, le lave à l'eau, ajoute au filtrat 2 cm3 d'éthanol, concentre à sec par distillation sous pression réduite, ajoute au résidu 1 cm3 d'eau et 1 cm3 d'éthanol, concentre à nouveau à sec, ajoute au résidu 5 cm3 d'acétate d'éthyle, concentre, isole par essorage le précipité formé, le lave, le sèche, obtient 125 mg de produit brut, ajoute 5 cm3 d'acide trifluoroacétique, 50 µl d'eau, agite à 20¤C pendant 20 minutes, ajoute lentement 5 cm3 d'éther, agite, isole par essorage le précipité formé, le lave, le sèche et obtient 59 mg de produit attendu.

Spectre U.V. (éthanol + HCl 0,1N)

| | | | |
|---|---|---|---|
| Max. | 218 nm $E_1^1$ | = 374 | $\epsilon$ = 30 244; |
| Inflexion | 247 nm $E_1^1$ | = 261 ; | |
| Inflexion | 260 nm $E_1^1$ | = 247 | $\epsilon$ = 20 000 ; |
| Inflexion | 278 nm $E_1^1$ | = 240 ; | |
| Max. | 317 nm $E_1^1$ | = 217 | $\epsilon$ = 17 500. |

**Exemple 2 : (6S) (7S) (Z) trifluoroacétate de 4−[[[7−[[(2−amino 4−thiazolyl) [(difluorométhoxy) imino] acétyl amino] 2−carboxy 8−oxo 4−thia 1−azabicyclo [4.2.0.] oct−2−èn−3−yl] méthyl] thio] 1−méthyl pyridinium (trifluoroacétate)**.

1) Condensation avec la base.

On mélange sous atmosphère inerte 150 mg de 3−iodo méthyl de 7−[3−[7−[2−triphénylméthylamino−4−thiazolyl] [(difluorométhyloxy) imino] acétyl] amino] 8−oxo 4−thia 1−azabicyclo [4.2.0.] oct−2−èn−3−yl] 2−carboxylate de 1,1−diméthyl éthyle (6S) (7S) (Z) (préparé comme le méthoxyimino décrit ci−dessous), 33 mg de 4−thio 1−méthyl pyridine, 2 cm3 d'acétonitrile, agite à 20¤C pendant 1 heure, concentre à sec par distillation sous pression réduite, chromatographie le résidu sur silice, sous pression d'azote en éluant par un mélange de chlorure de méthylène et de méthanol (90/10) et obtient 72 mg de produit attendu.

2) Détritylation, déterbutylation.

On mélange sous atmosphère inerte 72 mg de produit obtenu au paragraphe 1 précédent, 0,3 cm3 d'acide trifluoroacétique, 30 µl d'eau, agite à 20¤C pendant 2 heures, ajoute lentement 3 cm3 d'éther, agite, isole par essorage le précipité formé, le lave, le sèche et obtient 45 mg du produit attendu.

Spectre UV (éthanol)

| | | | |
|---|---|---|---|
| Max. | 225 nm $E_1^1$ | = 375 | $\epsilon$ = 29 900 ; |
| Max. | 307 nm $E_1^1$ | = 353 | $\epsilon$ = 28 200. |

Spectre UV (éthanol + HCl 0,1N)

| | | | |
|---|---|---|---|
| Max. | 223 nm $E_1^1$ | = 296 | $\epsilon$ = 23 600 ; |
| Max. | 257 nm $E_1^1$ | = 195 | $\epsilon$ = 15 600 ; |
| Max. | 302 nm $E_1^1$ | = 375 | $\epsilon$ = 29 900. |

**Préparation du 2−[[7−(2−triphénylméthylamino 4−thiazolyl) 2−(méthoxyimino) acétyl] amino] 2−terbutoxy carbonyl 8−oxo 4−thia 1−azabicyclo [4.2.0.] oct−2−èn−3−yl] méthyl iodo.**

On dissout 3 g de 2−[[7−(2−triphénylméthylamino 4−thiazolyl) 2−(méthoxyimino) acétyl] amino] 2−terbutoxycarbonyl 8−oxo 4−thia 1−aza bicyclo [4.2.0.] oct−2−èn−3−yl] méthyl] hydroxy dans 30 cm3 de chlorure de méthylène, ajoute 2,021 g d'iodure de tétrabutylammonium, 1,96 cm3 de 2,6−lutidine, refroidit à −70¤C, introduit 8,7 cm3 de solution d'anhydride trifluorométhyle sulfonique titrant 1 cm3 pour 10 cm3, agite pendant 15 minutes à −70¤C, amène la température à 0¤C, verse le mélange réactionnel dans l'eau, décante, extrait au chlorure de méthylène, concentre à sec par distillation sous pression réduite, dissout le résidu dans l'acétate d'éthyle, ajoute 10 cm3 de solution aqueuse 0,2 N de thiosulfate de sodium, agite, décante, extrait à l'acétate d'éthyle, concentre à sec par distillation sous pression réduite, empâte à l'éther isopropylique, concentre à sec et obtient 2,400 g de produit recherché.

**Exemple 3 : (6S) (7S) (Z) trifluoroacétate de 4−[[[7−[[(2−amino 4−thiazolyl) [(fluorométhoxy) imino] acétyl] amino] 2−carboxy 8−oxo 4−thia 1−azabicyclo [4.2.0.] oct−2−èn−3−yl] méthyl] thio] 1−méthyl pyridinium (trifluoroacétate).**

1) Condensation avec la base.

On mélange sous atmosphère inerte 150 mg de 3−iodo méthyl de 7−[3−[7−[(2−triphénylméthylamino 4−thiazolyl] [(2−fluorométhoxy) imino] acétyl] amino] 8−oxo 4−thia 1−azabicyclo [4.2.0.] oct−2−èn−3−yl] 2−carboxylate de 1,1−diméthyléthyle (6S) (7S) (Z) (préparé comme le méthoxyimino), 33 mg de 4−thio 1−méthylpyridine, 2 cm3 d'acétonitrile agite à 20¤C pendant 1 heure, concentre à sec par distillation sous pression réduite, chromatographie le résidu sur silice en éluant par un mélange de chlorure de méthylène et de méthanol (96/4) et obtient 110 mg de produit attendu.

2) Détritylation, déterbutylation.

On mélange sous atmosphère inerte 110 mg de produit obtenu au paragraphe 1 précédent, 0,440 cm3 d'acide trifluoroacétique, 44 µl d'eau, agite à 20¤C pendant 2 heures, ajoute lentement 10 cm3 d'éther, agite, isole par essorage le précipité formé, le lave, le sèche et obtient 64 mg de produit attendu.

Spectre UV (éthanol + HCl 0,1N)

| | | |
|---|---|---|
| Max. | 224 nm $E_1^1$ | = 316 $\epsilon$ = 24 700 ; |
| Inflexion | 260 nm $E_1^1$ | = 213 ; |
| Max. | 303 nm $E_1^1$ | = 379 $\epsilon$ = 29 600. |

**Exemple 4: Sel interne de 2−[[[7−[[2−(2−amino 4−thiazolyl) 2−(méthoxy imino) acétyl] amino] 2−carboxy 8−oxo 4−thia 1−azabicyclo [4.2.0.] oct−2−èn−3−yl] méthyl] thio] 1−méthyl pyridinium (6 R,S) (7 R,S) (Z).**

**Stade A :** Iodure de 7−[[[2−[(2−tritylaminothiazol−4−yl) 2−(méthoxy imino) acétyl] amino] 8−oxo 2−(1,1−diméthyl) éthyloxy carbonyl 4−thia 1−azabibyclo [4.2.0.] oct−2−èn−3−yl] méthyl] thio] 1−méthyl pyridinium (6 R,S) (7 R,S) (Z).

1) 3−Thiol méthyl 7−[2−[(2−triphénylméthylamino 4−thiazolyl) (méthoxy) imino] acétyl amino] 8−oxo 4−thia 1−azabicyclo [4.2.0.] oct−2−èn−3−yl] 2−carboxylate de 1,1−diméthyléthyle (6 R,S) (7 R,S) (Z) − Non isolé.

On mélange sous atmosphère inerte 368 mg de 1−(2−hydroxyéthyl) 4,6−diphényl pyridine 2−thione, 50 cm3 d'acétonitrile, agite, ajoute en une fois 984 mg de 3−iodo méthyl 7−[2−[2−(triphénylméthylamino 4−thiazolyl) (méthoxy) imino] acétyl] amino 8−oxo 4−thia 1−azabicyclo [4.2.0.] oct−2−èn−3yl] 2−carboxylate de 1,1−diméthyléthyle (6 R,S) (7 R,S) (Z), agite pendant 1 heure à 20¤C et obtient une solution de thiol recherché.

2) Condensation avec le chloropyridinium. Obtention de l'iodure de 7−[[[2−[[2−tritylaminothiazol−4−yl] 2−(méthoxyimino) acétyl] amino] 8−oxo 2−(1,1−diméthyl) éthyloxycarbonyl 4−thia 1−azabicyclo [4.2.0.] oct−2−èn−3−yl] méthyl] thio] 1−méthyl pyridinium (6 R,S) (7 R,S) (Z).

A la solution de thiol obtenu au paragraphe 1 du stade A, on ajoute en une fois 306 mg d'iodure de 2−chloro 1−méthyl pyridinium puis 0,2 cm3 de diisopropylamine, agite pendant 1 heure à 20¤C, concentre à sec par distillation sous pression réduite, chromatographie le résidu sur silice en éluant par un mélange de chlorure de méthylène et de méthanol (9/1). Au moment de la mise en solution et de la fixation sur colonne, on note une cristallisation importante d'iodure de 2,3−dihydro 5,7−diphényl oxazolo [3,2−a] pyridinium, on isole par essorage le précipité formé, chromatographie les liqueurs mères dans les conditions précédemment indiquées et obtient 720 mg de produit recherché.

**Stade B :** Sel interne de 2−[[[7−[[2−(2−amino 4−thiazolyl) 2−(méthoxy imino) acétyl] amino] 2−carboxy 8−oxo 4−thia 1−azabicyclo [4.2.0.] oct−2−èn−3−yl] méthyl] thio] 1−méthyl pyridinium (6 R,S) (7 R,S) (Z).

1) Détritylation et déterbutylation. Obtention du mélange 1 : 1 d'iodure et de trifluoroacétate de 2 – [[[7 – [ – [2 – (2 – amino 4 – thiazolyl) 2 – (méthoxy imino) acétyl] amino] 2 – carboxy 8 – oxo 4 – thia 1 – azabicyclo [4.2.0.] oct – 2 – èn – 3 – yl] méthyl] thio] 1 – méthyl pyridinium (6 R,S) (7 R,S) (Z).

On mélange sous atmosphère inerte 460 mg de produit obtenu au stade A précédent, ajoute 2 cm3 d'acide trifluoroacétique à 10% d'eau, agite à 20¤C pendant 1 heure, ajoute goutte à goutte 20 cm3 d'éther, agite à 20¤C pendant 1 heure, isole par essorage le précipité formé, le lave, le sèche et obtient 254 mg de produit recherché F = 150¤C peu net.

2) Obtention du sel interne.

On effectue une chromatographie "H.P.L.C." sur silice greffée RP 18. Après mise en solution dans un mélange de 1,5 cm3 de carbonate de triéthylamine et de 1,5 cm3 d'acétonitrile, on élue par des mélanges successifs d'eau distillée contenant de 5 à 20% d'acétonitrile. La majorité du produit est recueillie dans la fraction éluée par l'eau distillée à 10% d'acétonitrile. On lyophilyse et obtient 90 mg de sel interne recherché. F ≃ 250¤C avec décomposition.

<u>Spectre UV</u> (éthanol + HCl 0,1N)

Inflexion 225 nm $E_1^1$ = 296 ;
Max. 258 nm $E_1^1$ = 304 $\epsilon$ = 15 800 ;
Inflexion 288 nm $E_1^1$ = 304 ;
Max. 295 nm $E_1^1$ = 307 $\epsilon$ = 16 000 ;
Max. 310 nm $E_1^1$ = $\epsilon$ = 15 600.

La 1 – (2 – hydroxyéthyl) 4,6 – diphényl pyridin – 2 – thione utilisée au stade A de l'exemple peut être préparé comme suit :

**<u>Stade A</u> :** 4,6 – diphényl 2H – pyran – 2 – one.

On mélange sous atmosphère inerte 60 g de benzoylacétate d'éthyle, 33 cm3 d'acide sulfurique concentré, agite à 20¤C sous atmosphère inerte, pendant 2 semaines, verse le mélange réactionnel sur de la glace, extrait à l'éther, lave à l'eau, concentre à sec par distillation sous pression réduite, ajoute 30 cm3 d'éther au résidu, refroidit à 0¤C, agite, isole par essorage le précipité formé, le lave, le sèche. Les liqueurs mères amenées à sec cristallisent. On ajoute au résidu 10 cm3 d'éther, isole par essorage le précipité formé, le lave à l'éther, le sèche. On réunit le premier et le deuxième jet et obtient 11 g de produit recherché. F = 138¤C.

**<u>Stade B</u> :** 4,6 – diphényl 2H – pyran – 2 – thione.

On mélange sous atmosphère inerte 2,1 g de 4,6 – diphényl 2H – pyran – 2 – one obtenu au stade A, 45 cm3 de benzène, 7,5 g de pentasulfure de phosphore, porte à reflux sous atmosphère inerte, maintient le reflux durant 5 heures, décante à chaud la solution surnageante, la concentre à sec par distillation sous pression réduite, renouvelle cette opération à chaud sur le milieu réactionnel 4 fois avec à chaque fois 30 cm3 de benzène, les concentrats sont réunis et séchés sous bon vide, on chromatographie le produit obtenu (3 g) sur silice en éluant au chlorure de méthylène et obtient 2 g de produit recherché. F = 122 – 123¤C.

**<u>Stade C</u> :** 1 – (2 – hydroxyéthyl) 4,6 – diphényl pyridin – 2 – thione.

On mélange sous azote 2 g de 4,6 – diphényl 2H – pyran – 2 – thione, 40 cm3 de méthanol, porte au reflux, ajoute en une fois une solution de 560 mg d'éthanolamine dans 2 cm3 de méthanol, maintient le reflux pendant 5 heures, refroidit, concentre à sec par distillation sous pression réduite, chromatographie le résidu (2,4 g) sur silice en éluant par un mélange de chlorure de méthylène et d'acétate d'éthyle (80/20) et obtient 1,6 g de produit recherché.

Spectre UV (éthanol)

Max. 278 nm $E_1^1$ = 885 $\epsilon$ = 27 200 ;
Max. 390 nm $E_1^1$ = 263 $\epsilon$ = 8 100.

**Exemple 5 : Sel interne de 2−[[[7−[[2−(2−amino 4−thiazolyl) 2−méthoxy imino] acétyl] amino] 2−carboxy 8−oxo 4−thia 1−azabicyclo [4.2.0.] oct−2−èn−3−yl] méthyl] thio] 1−méthyl pyridinium (6 S) (7 S) (Z).**

**Stade A :** 3−iodo méthyl 7−[[2−[2−(triphénylméthyl) amino 4−thiazolyl] 2−(méthoxyimino) acétyl] amino] 8−oxo 4−thia 1−azabicyclo [4.2.0.] oct−2−èn−2−carboxylate de (1,1−diméthyl) éthyle (6S) (7S) (Z).

On mélange 2 g de 3−hydroxyméthyl 7−[[2−[2−(triphénylméthyl) amino 4−thiazolyl] 2−(méthoxyimino) acétyl] amino] 8−oxo 4−thia 1−azabicyclo [4.2.0.] oct−2−èn 2−carboxylate de (1,1−diméthyl) éthyle (6S) (7S) (Z), 20 cm3 de chlorure de méthylène, 1,3 g d'iodure de tétrabutylammonium, 1,3 cm3 de 2,6−lutidine, ajoute à −70¤C en 15 minutes environ, 9 cm3 d'une solution à 10% d'anhydride trifluorométhane sulfonique dans le chlorure de méthylène, plonge durant 15 minutes dans un bain de glace, ajoute 200 cm3 de solution aqueuse 0,1 N d'acide chlorhydrique, agite, décante, extrait au chlorure de méthylène, concentre à sec par distillation sous pression réduite, chromatographie le résidu (4,36 g) sur silice en éluant par un gradient de 1 à 10% d'acétate d'éthyle dans le chlorure de méthylène et obtient 1,324 g de produit recherché.
$[alpha]_D$ = +13¤ (c = 0,5% chloroforme) F = 150¤C peu net.

**Stade B :** Iodure de 7−[[[2−[[(2−tritylaminothiazol−4−yl) 2−(méthoxy imino) acétyl] amino] 8−oxo 2−(1,1−diméthyl) éthoxycarbonyl 4−thia 1−azabicyclo [4.2.0.] oct−2−èn−3−yl] méthyl] thio] 1−méthyl pyridinium (6S) (7S) (Z).

On mélange sous atmosphère inerte 820 mg de dérivé iodé obtenu au stade A précédent, 20 cm3 d'acétonitrile, ajoute en une fois 125 mg de N−méthyl 2−pyridine thione, agite pendant 1 heure à 20¤C, concentre à sec par distillation sous pression réduite, sèche sous bon vide, chromatographie le résidu (930 mg) sur silice en éluant par un mélange de chlorure de méthylène et de méthanol (9/1) et obtient 800 mg.
$[alpha]_D$ = −16¤ (c = 0,7% chloroforme).

**Stade C :** Sel interne de 2−[[[7−[[2−(2−amino 4−thiazolyl) 2−méthoxy imino] acétyl] amino] 2−carboxy 8−oxo 4−thia 1−azabicyclo [4.2.0.] oct−2−èn−3−yl] méthyl] thio] 1−méthyl pyridinium (6S) (7S) (Z).

1) Déterbutylation, détritylation. Obtention du mélange 1 : 1 d'iodure et du trifluoroacétate de 2−[[[7−[[2−(2−amino 4−thiazolyl) 2−(méthoxyimino) acétyl] amino] 2−carboxy 8−oxo 4−thia 1−azabicyclo [4.2.0.] oct−2−èn−3−yl] méthyl] thio] 1−méthyl pyridinium (6S) (7S) (Z).

On mélange à 0¤C 700 mg de produit obtenu au stade B précédent, 3 cm3 d'acide trifluoroacétique à 10% d'eau, agite pendant 1 heure à 20¤C, ajoute goutte à goutte 20 cm3 d'éther, agite à 20¤C pendant 1 heure jusqu'à concrétisation complète du précipité, isole par essorage l'insoluble formé, le lave, le sèche sous vide et obtient 580 mg de produit recherché. F = 150−160¤C avec décomposition.

2) Obtention du sel interne.

On mélange 580 mg du produit obtenu au paragraphe 1 du stade C, 3 cm3 d'acétonitrile, 3 cm3 de carbonate de triéthylamine, fixe la solution obtenue sur une colonne "H.P.L.C." et élue par un mélange d'eau et d'acétonitrile à gradient variable de 5 à 10% et obtient 100 mg de produit recherché. F = 190−200¤C avec décomposition.
$[alpha]_D$ = +28° (c = 0,4% dans l'eau + quelques gouttes d'HCL 0,1 N).

Spectre UV (éthanol + HCl 0,1N)

Inflexion 240 nm $E_1^1$ = 344 ;
Inflexion 265 nm $E_1^1$ = 337 ;
Max. 284 nm $E_1^1$ = 374 $\epsilon$ = 19 500 ;
Inflexion 290 nm $E_1^1$ = 365 ;
Inflexion 307 nm $E_1^1$ = 306 ;
Inflexion 318 nm $E_1^1$ = 276.

La 1 – méthyl 2 – pyridine thione utilisée au départ du stade B de l'exemple peut être préparée comme suit.

On mélange sous atmosphère inerte 1 g de 1 – méthyl 2 – pyridone, 20 cm3 de benzène, ajoute en une fois, sous vive agitation 2 g de 2,4 – bis – (4 – méthoxyphényl) 2,4 – dithioxo 1,3,2,4 – dithiadiphosphétane (réactif de Lawesson), agite pendant 2 heures au reflux, refroidit, filtre, lave au benzène, concentre le filtrat benzénique à sec par distillation sous pression réduite, chromatographie le résidu (2,8 g) sur silice en éluant par le mélange de chlorure de méthylène et d'acétate d'éthyle (8/2) et obtient 900 mg de produit recherché. F = 98¤C.

Spectre UV (éthanol)

Inflexion 220 nm $E_1^1$ = 440 ;
Max. 284 nm $E_1^1$ = 1100 $\epsilon$ = 13 800 ;
Max. 354 nm $E_1^1$ = 569 $\epsilon$ = 7 100.

**Exemple 6 : 7 – [[2 – (2 – amino 4 – thiazolyl) 2 – méthoxyimino acétyl] amino] 3 – [(1 – oxyde pyridin – 2 – yl) thiométhyl] 8 – oxo 4 – thia 1 – azabicyclo [4.2.0.] oct – 2 – èn 2 – carboxylique (6 R,S) (7 R,S) (Z).**

**Stade A :** 7 – [[2 – (2 – triphénylméthylamino – 4 – thiazolyl) 2 – (méthoxyimino acétyl] amino] 3 – [(1 – oxyde pyridin – 2 – yl) thiométhyl] 8 – oxo 4 – thia 1 – azabicyclo [4.2.0.] oct – 2 – èn 2 – carboxylate de (1,1 – diméthyl) éthyle (6 R,S) (7 R,S) (Z).

On mélange 330 mg de 3 – iodo méthyl [[[7 – [[2 – (2 – triphénylméthyl) amino 4 – thiazolyl] 2 – méthoxyimino] acétyl] amino] 8 – oxo 4 – thia 1 – azabicyclo [4.2.0.] oct – 2 – èn 2 – carboxylate de (1,1 – diméthyl) éthyle (6 R,S) (7 R,S) (Z), 1,3 cm3 d'acétonitrile, 61 mg de 2 – mercapto pyridine N – oxyde, 67 mg de carbonate de potassium, agite à 20¤C pendant 1 heure et 30 minutes, concentre à sec par distillation sous pression réduite, chromatographie résidu sur silice en éluant par un mélange de chlorure de méthylène et d'acétone (6/4) et obtient 212 mg de produit recherché.

**Stade B :** Acide 7 – [[2 – (2 – amino 4 – thiazolyl) 2 – méthoxyimino acétyl] amino] 3 – [(1 – oxyde pyridin – 2 – yl) thiométhyl] 8 – oxo 4 – thia 1 – azabicyclo [4.2.0.] oct – 2 – èn 2 – carboxylique (6 R,S) (7 R,S) (Z).

On mélange 200 mg de produit obtenu au stade A précédent, 2 cm3 d'acide formique à 33% d'eau, agite à 20¤C pendant 1 heure, puis pendant 5 heures à 50¤C, refroidit, élimine par filtration le triphénylméthyl carbinol formé, concentre le filtrat à sec par distillation sous pressio réduite, ajoute de l'acétate d'éthyle, concentre à sec et répète cette opération en plusieurs fois, ajoute de l'acétate d'éthyle, agite le précipité dans l'acétate d'éthyle, essore, lave, sèche et obtient 114 mg de produit recherché. F = 220¤C.

Spectre UV (éthanol)

Max. 240 nm $E_1^1$ = 725 $\epsilon$ = 37 900 ;
Max. 300 nm $E_1^1$ = 311 $\epsilon$ = 16 300 ;
Inflexion 224 et 270 nm.

Spectre UV (éthanol + HCl 0,1N)

Max. 242 nm $E_1^1$ = 645 $\epsilon$ = 33 700 ;
Inflexion 264 nm $E_1^1$ = 405 ;

Inflexion 280 nm $E_1^1$ = 371 ;
Inflexion 290 nm $E_1^1$ = 340 ;
Inflexion 307 nm $E_1^1$ = 288.

**Exemple 7 : Iodure de 2-[[[7-[[2-(2-amino 4-thiazolyl) 2-méthoxyimino) acétyl] amino] 2-carboxy 8-oxo 4-thia 1-azabicyclo [4.2.0.] oct-2-èn-3-yl] méthyl] thio] 1-méthyl 3-méthoxy pyridinium (6 R,S) (7 R,S) (Z).**

**Stade A :** Iodure de 7-[[[2-[[(2-tritylaminothiazol-4-yl) 2-(méthoxyimino) acétyl] amino] 8-oxo 2-(1,1-diméthyl) éthyloxycarbonyl 4-thia 1-azabibyclo [4.2.0.] oct-2-èn-3-yl] méthyl] thio] 1-méthyl 3-méthoxy pyridinium (6 R,S) (7 R,S) (Z).

On mélange sous atmosphère inerte 164 mg de 3-iodométhyl 7-[2-[2-(triphénylméthylamino 4-thiazolyl) [(méthoxy) imino] acétyl] amino 8-oxo 4-thia 1-azabicyclo [4.2.0.] oct-2-èn-3-yl] 2-carboxylate de 1,1-diméthyl éthyle (6 R,S) (7 R,S) (Z), 5 cm3 d'acétonitrile, ajoute en une fois 31 mg de N-méthyl 3-méthoxy 2-thiopyridone, agite à 20¤C pendant 2 heures, concentre à sec par distillation sous pression réduite, sèche sous bon vide, chromatographie le résidu sur silice en éluant avec un mélange de chlorure de méthylène et de méthanol (9/1) et obtient 145 mg de produit recherché. F = 170-180¤C avec décomposition.

**Stade B :** Iodure de 2-[[7-[[2-(2-amino 4-thiazolyl) 2-(méthoxyimino) acétyl] amino] 2-carboxy 8-oxo 4-thia 1-azabicyclo [4.2.0.] oct-2-èn-3-yl] méthyl] thio] 1-méthyl 3-méthoxy pyridinium (6 R,S) (7 R,S) (Z).

On mélange sous atmosphère inerte 110 mg de produit obtenu au stade A précédent, 2 cm3 de solution aqueuse d'acide formique à 66%, agite pendant 2 heures et 30 minutes à 60¤C, refroidit, élimine par filtration le triphénylcarbinol formé, concentre le filtrat à sec par distillation sous pression réduite, on ajoute de l'acétate d'éthyle, concentre à sec et répète la même opération à plusieurs reprises, on ajoute de l'acétate d'éthyle, isole par essorage le précipité formé, le lave à l'éther, le sèche sous bon vide et obtient 75 mg de produit recherché. F = 190-200¤C avec décomposition.

Spectre UV (éthanol)

Max. 215 nm $E_1^1$ = 555 $\epsilon$ = 37 700 ;
Max. 294 nm $E_1^1$ = 232 $\epsilon$ = 15 700 ;
Inflexion 335 nm $E_1^1$ = 117 $\epsilon$ = 7 900.

Spectre UV (éthanol + HCl 0,1N)

Max. 214 nm $E_1^1$ = 497 $\epsilon$ = 33 700 ;
Inflexion. 263 nm $E_1^1$ = 244 ;
Max. 285 nm $E_1^1$ = 270 $\epsilon$ = 18 300 ;
Max. 338 nm $E_1^1$ = 138 $\epsilon$ = 9 350.

La N-méthyl 3-méthoxy 2-thiopyridone utilisée au stade A de l'exemple peut être préparée comme suit.

On mélange sous atmosphère inerte 2,1 g de N-méthyl 3-méthoxy 2-pyridone, 50 cm3 de benzène, ajoute en une fois 3 g de 2,4 bis-(4-méthoxy phényl) 2,4-dithioxo 1,3,2,4-dithiadiphosphétane (réactif de Lawesson), agite au reflux pendant 3 heures, refroidit, concentre à sec par distillation sous pression réduite, chromatographie le résidu sur silice en éluant par un mélange de chlorure de méthylène et de méthanol (9/1) et obtient 1,2 g de produit recherché. F = 130-132¤C.

**Exemple 8 : Sel interne de 2-[[[7-[[2-(2-amino 4-thiazolyl) 2-méthoxy imino] acétyl] amino] 2-carboxy 8-oxo 4-thia 1-azabicyclo [4.2.0.] oct-2-èn-3-yl] méthyl] thio] 1-méthyl quinolinium (6S) (7S) (Z).**

**Stade A :** Iodure de 7-[[[2-[[(2-tritylaminothiazol-4-yl) 2-(méthoxy imino) acétyl] amino] 8-oxo 2-(1,1-diméthyl) éthoxycarbonyl-4-thia 1-azabicyclo [4.2.0.] oct-2-èn-3-yl] méthyl] thio] 1-méthyl quinolinium (6S) (7S) (Z).

On mélange sous atmosphère inerte 328 mg de iodométhyl 7 – [2 – [2 – (triphénylméthyl) amino – 4 – thiazolyl] [(méthoxy) imino] acétyl] amino 8 – oxo 4 – thia 1 – azabicyclo [4.2.0.] oct – 2 – èn – 3 – yl] 2 – carboxylate de 1,1 – diméthyl éthyle (6S) (7S) (Z), 10 cm3 d'acétonitrile, ajoute à 20¤C en une fois, 70 mg de 1 – méthyl 2 – quinoline thione, agite à 20¤C pendant 1 heure, élimine l'acétonitrile par distillation sous pression réduite, sèche sous bon vide et obtient 400 mg de produit recherché, utilisé tel quel pour le stade suivant.

**Stade B** : Sel interne de 2 – [[[7 – [[2 – (2 – amino 4 – thiazolyl) 2 – méthoxy imino] acétyl] amino] 2 – carboxy 8 – oxo 4 – thia 1 – azabicyclo [4.2.0.] oct – 2 – èn – 3 – yl] méthyl] thio] 1 – méthyl quinolinium (6S) (7S) (Z).

1) Détritylation, déterbutylation.

On mélange sous atmosphère inerte 360 mg de composé obtenu au stade A précédent, 6 cm3 de solution aqueuse à 60% d'acide formique, agite pendant 3 heures à 60¤C, refroidit, élimine par filtration le triphényl carbinol formé, concentre à sec par distillation sous pression réduite, obtient 300 mg de produit incomplètement débloqué , les introduit dans 1 cm3 d'acide trifluoroacétique à 10% d'eau, agite pendant 1 heure à 20¤C, ajoute goutte à goutte 15 cm3 d'éther, agite, isole par essorage le précipité formé, le lave, le sèche et obtient 260 mg de produit recherché, détritylé et déterbutylé.

2) Sel interne.

On mélange sous atmosphère inerte 1,5 cm3 de carbonate de triéthylamine, 1,5 cm3 d'acétonitrile, obtient une solution que l'on injecte dans une colonne de 65 cm sur 25 mm de diamètre chargée en lychoprop RP 18, 5/20 microns. Après élution du mélange à gradient variable d'eau – acétonitrile, on isole les pics de 5 à 10% (impuretés), puis élue à l'eau à 15% à l'acétonitrile, à l'eau à 20¤C d'acétonitrile et les lyophylyse. On obtient 49 mg. F = 190 – 200¤C.

Spectre UV (éthanol)

| | | |
|---|---|---|
| Max. | 216 nm $E_1^1$ | = 930 $\epsilon$ = 53 100 ; |
| Inflexion | 231 nm $E_1^1$ | = 402 ; |
| Inflexion | 236 nm $E_1^1$ | = 382 ; |
| Max. | 276 nm $E_1^1$ | = 489 $\epsilon$ = 27 900 ; |
| Inflexion | 292 nm $E_1^1$ | = 319 ; |
| Inflexion | 302 nm $E_1^1$ | = 247 ; |
| Max. | 376 nm $E_1^1$ | = 186 $\epsilon$ = 10 600. |

Spectre UV (éthanol + HCl 0,1N)

| | | |
|---|---|---|
| Max. | 216 nm $E_1^1$ | = 877 $\epsilon$ = 50 100 ; |
| Max. | 275 nm $E_1^1$ | = 588 $\epsilon$ = 33 600 ; |
| Max. | 375 nm $E_1^1$ | = 189 $\epsilon$ = 10 800. |
| Inflexions | 231, 238, 292, 306 nm. | |

La 1 – méthyl quinolin – 2 – thione utilisée au départ du stade A de l'exemple peut être préparée comme suit.

On mélange sous atmosphère inerte 1 g de 1 – méthyl quinolin – 2 – one, 25 cm3 de benzène, ajoute en une fois 1,27 g de 2,4 – bis – (4 – méthoxyphényl) 2,4 – dithioxo 1,3,2,4 – dithiadiphosphétane (réactif de Lawesson), agite au reflux pendant 1 heure, refroidit, élimine le solvant par distillation sous pression réduite, sèche sous bon vide, chromatographie le résidu sur silice en éluant avec un mélange de chlorure de méthylène et d'acétate d'éthyle (1/1) et obtient 1 g de produit recherché. F = 114¤C.

**Exemple 9 : Trifluoroacétate de 4– [[7– [[2– (2– amino 4– thiazolyl) 2– (méthoxy imino) acétyl] amino] 2– carboxy 8– oxo 4– thia 1– azabicyclo [4.2.0.] oct– 2– èn– 3– yl] méthyl] thio] 1– méthyl quinolinium, trifluoroacétate (6S) (7S) (Z).**

Stade A : Iodure de 7 – [[4 – [[2 – tritylaminothiazol – 4 – yl] 2 – (méthoxy imino) acétyl] amino] 8 – oxo 2 – (1,1 – diméthyl) éthoxycarbonyl – 4 – thia 1 – azabicyclo [4.2.0.] oct – 2 – èn – 3 – yl] méthyl] thio] 1 – méthyl quinolinium (6S) (7S) (Z).

On mélange sous atmosphère d'azote 328 mg de 3 – iodométhyl 7 – [[2 – [(triphénylméthyl) amino – 4 – thiazolyl] 2 – méthoxyimino] acétyl] amino 8 – oxo 4 – thia 1 – azabicyclo [4.2.0.] oct – 2 – èn – 2 – carboxylate de (1,1 – diméthyl) éthyle (6S) (7S) (Z), 10 cm3 d'acétonitrile, ajoute à 20¤C en une fois 70 mg de 1 – méthyl quinolin – 4 – thione, agite à 20¤C pendant 30 minutes, concentre à sec par distillation sous pression réduite, empâte le résidu (405 mg) par 5 cm3 d'éther en agitant pendant 30 minutes à 20¤C, isole par essorage le précipité formé, le lave, le sèche et obtient 390 mg de produit attendu. F ≃ 210¤C avec décomposition.
$[alpha]_D$ = – 76¤ (c = 0,5% chloroforme)

Stade B : Trifluoroacétate de 4 – [[7 – [[2 – (2 – amino 4 – thiazolyl) 2 – méthoxy) imino] acétyl amino] 2 – carboxy 8 – oxo 4 – thia 1 – azabicyclo [4.2.0.] oct – 2 – èn – 3 – yl] méthyl] thio] 1 – méthyl quinolinium, trifluoroacétate (6S) (7S) (Z).

On mélange à 0¤C sous atmosphère inerte 340 mg de produit obtenu au stade A précédent, 1,5 cm3 d'acide trifluoroacétique à 10% d'eau, agite à 20¤C vigoureusement pendant 45 inutes, ajoute goutte à goutte 15 cm3 d'éther, agite à 20¤C pendant 1 heure, isole par essorage le précipité formé, le lave, le sèche sous vide et obtient 258 mg de produit attendu. F≃ 210¤C avec décomposition.
$[alpha]_D$ = – 43¤ (c = 0,5% méthanol).

Spectre UV (éthanol)

| | | | |
|---|---|---|---|
| Max. | 238 nm $E_1^1$ | = 586 | ε = 46 800 ; |
| Max. | 302 nm $E_1^1$ | = 226 | ε = 18 100 ; |
| Inflexion | 313 nm $E_1^1$ | = 198 ; | |
| Max. | 353 nm $E_1^1$ | = 210 | ε = 16 800. |

Spectre UV (éthanol + HCl 0,1N)

| | | | |
|---|---|---|---|
| Max. | 239 nm $E_1^1$ | = 557 | ε = 44 500 ; |
| Inflexion | 289 nm $E_1^1$ | = 230 ; | |
| Max. | 294 nm $E_1^1$ | = 236 | ε = 18 800 ; |
| Inflexion | 305 nm $E_1^1$ | = 228 ; | |
| Inflexion | 314 nm $E_1^1$ | = 221 ; | |
| Max. | 252 nm $E_1^1$ | = 226 | ε = 18 100. |

La 1 – méthyl quinolin – 4 – thione utilisée au départ du stade A peut être préparée comme suit.

On dissout sous atmosphère inerte 1 g de 1 – méthyl 4 – quinolone dans 20 cm3 de benzène, ajoute en une fois sous vive agitation 1,27 g de 2,4 – bis – (4 – méthoxyphényl) 2,4 – dithioxo 1,3,2,4 – dithiadiphos – phétane (réactif de Lawesson), agite pendant 1 heure au reflux, refroidit, concentre à sec par distillation sous pression réduite, chromatographie le résidu (2,5 g) sur silice en éluant avec un mélange de chlorure de méthylène et de méthanol (9/1) et obtient 910 mg de produit attendu. F = 208 – 210¤C.

**Exemple 10 : Sel interne de 2– [[[7– [[(2– amino 4– thiazolyl) (méthoxy imino) acétyl] amino] 2– carboxy 8– oxo 4– thia 1– azabicyclo [4.2.0.] oct– 2– èn– 3– yl] méthyl] thio] 1– éthyl pyridinium (6S) (7S) (Z).**

Stade A : Iodure de 2 – [[[2 – [[(2 – tritylamino – 4 – thiazolyl) (méthoxyimino) acétyl] amino] 8 – oxo 2 – (1,1 – diméthyl) éthyloxycarbonyl – 4 – thia 1 – azabicyclo [4.2.0.] oct – 2 – èn – 3 – yl] méthyl] thio] 1 – éthyl pyridinium (6S) (7S) (Z).

On mélange sous atmosphère inerte 350 mg de 3 – iodométhyl 7 – [[2 – [2 – (triphénylméthyl) amino 4 – thiazolyl] 2 – (méthoxyimino) acétyl] amino] 8 – oxo 4 – thia 1 – azabicyclo [4.2.0.] oct – 2 – èn – 2 – carboxy – late de 1,1 – diméthyl) éthyle (6S) (7S) (Z), 3,5 cm3 d'acétonitrile,71 mg de 1 – éthyl pyridin – 2 – thione, agite à 20¤C pendant 30 minutes, concentre à sec par distillation sous pression réduite, chromatographie le résidu (450 mg) sur silice en éluant par un mélange de chlorure de méthylène et de méthanol (9/1) et

obtient 347 mg de produit recherché.

**Stade B :** Sel interne de 2 – [[[7 – [[2 – (2 – amino 4 – thiazolyl) (méthoxy imino) acétyl] amino] 2 – carboxy 8 – oxo 4 – thia 1 – azabicyclo [4.2.0.] oct – 2 – èn – 3 – yl] méthyl] thio] 1 – éthyl pyridinium (6S) (7S) (Z).

1) Déterbutylation et détritylation.

On mélange 330 mg de produit obtenu au stade A précédent, 1,6 cm3 d'acide trifluoroacétique à 10% d'eau, agite à 20¤C pendant 50 minutes, introduit lentement 7 cm3 d'éther, agite pendant 2 heures, isole par essorage le précipité formé, le lave, le sèche et obtient 250 mg de produit débloqué. F = 200¤C.

2) Obtention du sel interne.

On dissout 240 mg de produit obtenu au paragraphe 1 du stade B dans 1,2 cm3 d'acétonitrile et 1,2 cm3 de solution 1 M de carbonate de triéthylamine. On fixe la solution sur colonne de silice (longueur 65 cm, diamétre 2,7 cm), élue successivement avec 1 litre des mélanges suivants :

| Acétonitrile % | Eau % |
|---|---|
| 5 | 95 |
| 10 | 90 |
| 15 | 85 |
| 20 | 80 |

recueille les fractions contenant le produit attendu, lyophylyse et obtient 115 mg de produit recherché. F = 200¤C.

Spectre UV (éthanol)

Inflexion 224 nm $E_1^1$ = 408 ;
Inflexion 234 nm $E_1^1$ = 376 ;
Max. 296 nm $E_1^1$ = 326 ;

Spectre UV (éthanol + HCl 0,1N)

Inflexion 223 nm $E_1^1$ = 319 ;
Inflexion 270 nm $E_1^1$ = 329 ;
Max. 284 nm $E_1^1$ = 284.

La 1 – éthyl 2 – pyridinethione utilisée au départ du stade A peut être préparée comme suit.

On mélange sous atmosphère inerte 1,23 g de 1 – éthyl 2 – pyridone, 25 cm3 de benzène, ajoute en une fois sous vive agitation 2,02 g de 2,4 – bis – (4 – méthoxyphényl) 2,4 – dithioxo 1,3,2,4 – dithiadiphos – phétane (réactif de Lawesson), agite au reflux pendant 2 heures, refroidit, filtre, amène le filtrat à sec par distillation sous pression réduite, chromatographie le résidu (3,8 g) sur silice en éluant par un mélange de chlorure de méthylène et d'acétate d'éthyle (8/2) et obtient 1,3 g de produit attendu. F≈50¤C.

**Exemple 11 : Sel interne de 4– [[[7– [[2– amino 4– thiazolyl) (méthoxy imino) acétyl] amino] 2– carboxy 8– oxo 4– thia 1– azabicyclo [4.2.0.] oct– 2– èn– 3– yl] méthyl] thio] 1– méthyl pyridinium (6S) (7S) (Z).**

**Stade A :** Iodure de 4 – [[[2 – [[(2 – tritylamino – 4 – thiazolyl) (2 – méthoxy imino) acétyl] amino] 8 – oxo 2 – (1,1 – diméthyl) éthyloxycarbonyl 4 – thia 1 – azabicyclo [4.2.0.] oct – 2 – èn – 3 – yl] méthyl] thio] 1 – méthyl pyridinium (6S) (7S) (Z).

On mélange sous atmosphère inerte 350 mg de 3 – iodométhyl 7 – [[2 – [2 – (triphénylméthyl) amino 4 – thiazolyl] 2 – (méthoxyimino) acétyl] amino] 8 – oxo 4 – thia 1 – azabicyclo [4.2.0.] oct – 2 – èn – 2 – carboxy – late de (1,1 – diméthyl) éthyle (6S) (7S) (Z), 3,5 cm3 d'acétonitrile, 65 mg de 1 – méthyl 4 – pyridine thione,

agite à 20¤C pendant 2 heures, concentre à sec par distillation sous pression réduite, chromatographie le résidu (440 mg) sur silice en éluant avec un mélange de chlorure de méthylène et de méthanol (9/1) et obtient 320 mg de produit recherché.

**Stade B :** Sel interne de 4 – [[[7 – [[(2 – amino 4 – thiazolyl) (méthoxy imino) acétyl] amino] 2 – carboxy 8 – oxo 4 – thia 1 – azabicyclo [4.2.0.] oct – 2 – èn – 3 – yl] méthyl] thio] 1 – méthyl pyridinium (6S) (7S) (Z).

1) Déterbutylation et détritylation.

On mélange sous atmosphère inerte 280 mg de produit obtenu au stade A précédent, 1,4 cm3 d'acide trifluoroacétique à 10% d'eau, agite à 20¤C pendant 50 minutes, ajoute lentement 10 cm3 d'éther, agite pendant 2 heures, isole par essorage le précipité formé, le lave, le sèche et obtient 184 mg de produit débloqué. F = 200¤C

2) Obtention du sel interne.

On dissout les 180 mg de produit obtenu au paragraphe 1 du stade B dans 1 cm3 d'acétonitrile et 1 cm3 de solution aqueuse 1 M de carbonate de triéthylamine. On fixe sur colonne de silice (longueur 67 cm, diamètre 2,5 cm), élue successivement avec 1 litre des mélanges suivants :

| Acétonitrile % | Eau % |
|---|---|
| 5 | 95 |
| 10 | 90 |
| 15 | 85 |
| 20 | 80 |

recueille les fractions contenant le produit attendu, lyophilise et obtient 95 mg de sel interne recherché. F = 200¤C.

Spectre UV (éthanol)

| | | |
|---|---|---|
| Max. | 229 nm $E_1^1$ | = 452 ϵ = 23 500 ; |
| Inflexion | 292 nm $E_1^1$ | = 409 ; |
| Max. | 305 nm $E_1^1$ | = 463 ϵ = 24 100. |

Spectre UV (éthanol + HCl 0,1N)

| | | |
|---|---|---|
| Max. | 229 nm $E_1^1$ | = 394 ϵ = 20 500 ; |
| Inflexion | 276 nm $E_1^1$ | = 341 ; |
| Max. | 300 nm $E_1^1$ | = 511 ϵ = 26 600. |

**Exemple 12 : Mélange 1:1 des iodures et trifluoroacétate de 2–[[7–[[(2–amino 4–thiazolyl) (difluorométhoxyimino) acétyl] amino] 2–carboxy 8–oxo 4–thia 1–azabicyclo [4.2.0.] oct–2–èn–3–yl] méthyl] thio] 1–méthyl pyridinium (6S) (7S) (Z) (trifluoroacétate).**

**Stade A :** Echange de l'iodo et de la 1 – méthylpyridine 2 – thione.

On mélange sous atmosphère inerte 130 mg de 3 – iodométhyl 7 – [[[2 – [2 – triphénylméthylamino – 4 – thiazolyl] [(difluorométhyxy) imino] acétyl] amino] 8 – oxo 4 – thia 1 – azabicyclo [4.2.0.] oct – 2 – èn – 3 – yl] 2 – carboxylate de 1,1 – diméthyléthyle (6S) (7S) (Z), 1,5 cm3 d'acétonitrile, 21 mg de 1 – méthyl 2 – pyridine thione, agite à 20¤C pendant 1 heure, concentre à sec par distillation sous pression réduite, chromatographie le résidu sur silice en éluant sous pression d'azote avec un mélange de chlorure de méthylène et de méthanol (9/1) et obtient 102 mg de produit recherché.

**Stade B :** Déblocage. Obtention du mélange 1:1 des iodures et trifluoroacétate de 2 – [[7 – [[(2 – amino 4 – thiazolyl) (difluorométhoxyimino) acétyl] amino] 2 – carboxy 8 – oxo 4 – thia 1 – azabicyclo [4.2.0.] oct – 2 – èn – 3 – yl] méthyl] thio] 1 – méthyl pyridinium (6S) (7S) (Z) (trifluoroacétate).

On mélange sous atmosphère inerte 102 mg de produit obtenu au stade A précédent, 0,4 cm3 d'acide trifluoroacétique à 10% d'eau, agite à 20¤C pendant 1 heure, ajoute goutte à goutte 6 cm3 d'éther, agite, isole par essorage le précipité formé, le lave, le sèche et obtient 66 mg de produit recherché. Rf. 0,5 en éluant par un mélange d'acétone et d'eau (2/1).

Spectre UV (éthanol)

| | | | |
|---|---|---|---|
| Max. | 221 nm $E^1_1$ | = 373 | $\epsilon$ = 29 800 ; |
| Inflexion | 246 nm $E^1_1$ | = 223 ; | |
| Max. | 307 nm $E^1_1$ | = 247 | $\epsilon$ = 19 700. |

Spectre UV (éthanol + HCl 0,1N)

| | | | |
|---|---|---|---|
| Inflexion | 217 nm $E^1_1$ | = 327 | $\epsilon$ = 26 100 ; |
| Max. | 250 nm $E^1_1$ | = 239 | $\epsilon$ = 19 100 ; |
| Inflexion | 292 nm $E^1_1$ | = 226 ; | |
| Max. | 304 nm $E^1_1$ | = 239 | $\epsilon$ = 19 100. |

**Exemple 13 : Iodure de 2– [[[7– [[(2– amino 4– thiazolyl) (2– propényloxy) imino] acétyl] amino] 2– carboxy 8– oxo 4– thia 1– azabicyclo [4.2.0.] oct– 2– èn– 3– yl] méthyl] thio] 1– méthyl pyridinium (trifluoroacétate) (6S) (7S) (Z).**

1) Echange de l'iodo avec la 1 – méthyl pyridin – 2 – thione.

On mélange sous atmosphère inerte 117 mg de 3 – iodométhyl de 7 – [3 – [7 – [(2 – triphénylméthyl) amino – 4 – thiazolyl] [(2 – propényloxy) imino] acétyl] amino] 8 – oxo 4 – thia 1 – azabicyclo [4.2.0.] oct – 2 – èn – 3 – yl] 2 – carboxylate de 1,1 – diméthyléthyle (6S) (7S) (Z), 1,2 cm3 d'acétonitrile, 18 mg de 1 – méthyl pyridin – 2 – thione, agite à 20 °C pendant 1 heure et 30 minutes, concentre à sec par dsitillation sous pression réduite, chromatographie le résidu sur silice en éluant par un mélange de chlorure de méthylène et de méthanol (90/10) et obtient 118 mg de produit attendu, utilisé tel quel pour la réaction suivante.

2) Détritylation et déterbutylation. Obtention de l'iodure de 2 – [[[7 – [[(2 – amino 4 – thiazolyl) (2 – propény – loxy) imino] acétyl] amino] 2 – carboxy 8 – oxo 4 – thia 1 – azabicyclo [4.2.0.] oct – 2 – èn – 3 – yl] méthyl] thio] 1 – méthyl pyridinium (trifluoroacétate) (6S) (7S) (Z).

On mélange 114 mg de produit obtenu au paragraphe 1 précédent, 0,82 cm3 d'acide trifluoroacétique, 0,05 cm3 d'eau, agite à 20¤C pendnt 2 heures, ajoute lentement 3,5 cm3 d'éther, agite, isole pr essorage le précipité formé, le lave, le sèche sous vide et obtient 58 mg de produit attendu. Rf = 0,5 en éluant avec un mélange d'acétone et d'eau (2/1).

Spectre UV (éthanol)

| | | | |
|---|---|---|---|
| Inflexion | 213 nm $E^1_1$ | = 357 ; | |
| Inflexion | 222 nm $E^1_1$ | = 345 ; | |
| Inflexion | 231 nm $E^1_1$ | = 328 ; | |
| Max. | 305 nm $E^1_1$ | = 245 | $\epsilon$ = 19 300. |

Spectre UV (éthanol + HCl 0,1N)

| | | | |
|---|---|---|---|
| Inflexion | 219 nm $E^1_1$ | = 340 $\times$ 2 ; | |
| Max. | 252 nm $E^1_1$ | = 255 $\times$ 2 | $\epsilon$ = 20 100 ; |
| Max. | 294 nm $E^1_1$ | = 243 $\times$ 2 | $\epsilon$ = 19 200 ; |
| Inflexion | 307 nm $E^1_1$ | = 230 $\times$ 2. | |

**Exemple 14 : Iodure et trifluoroacétate de (6S) (7S) (Z) 2–[[7–[[(2–amino 4–thiazolyl) [–(fluorométhoxy) imino] acétyl] amino] 2–carboxy 8–oxo 4–thia 1–azabicyclo [4 2 0.] oct–2–èn–3–yl] méthyl thio] 1–méthyl pyridinium (trifluoroacétate).**

**Stade A :** Echange de l'hydroxyle par l'iode.

On mélange sous atmosphère inerte 150 mg de 3–hydroxyméthyl 7–[2–[2–triphénylméthylamino 4–thiazolyl] [(difluorométhyl) imino] acétyl] amino] 8–oxo 4–thia 1–azabicyclo [4.2.0.] oct–2–èn–3–yl] 2–carboxylate de 1,1–diméthyléthyle (6S) (7S) (Z), 3 cm3 de chlorure de méthylène, 152 mg de $Bu_4N^{\oplus}I^{\ominus}$, 96 ul de 2,6–lutidine, ajoute en 10 minutes environ, à –70¤C, 1,4 cm3 de solution d'anhydride trifluorométhane sulfonique à 5% en volume dans le chlorure de méthylène, agite pendant 15 minutes, concentre à sec par distillation sous pression réduite, chromatographie le résidu sur silice en éluant avec un mélange de chlorure de méthylène et d'acétate d'éthyle (9/1) et obtient 120 mg de produit iodé recherché utilisé tel quel pour le stade suivant.

**Stade B :** Echange de l'iode par la base 2–thio 1–méthyl pyridine.

On mélange sous atmosphère inerte 120 mg de dérivé iodé obtenu au stade A précédent, 18 mg de 2–thio 1–méthyl pyridine, 4 cm3 d'acétonitrile, agite à 20¤C pendant 1 heure, concentre à sec par distillation sous pression réduite, chromatographie le résidu sur silice en éluant par un mélange de chlorure de méthylène et de méthanol (9/1) et obtient 100 mg de produit recherché. Rf = 0,25 en éluant avec un mélange de chlorure de méthylène et de méthanol (9/1).

**Stade C :** Déblocage. Iodure et trifluoroacétate de (6S) (7S) (Z) 2–[[7–[[(2–amino4–thiazolyl) [–(fluorométhoxy) imino] acétyl] amino] 2–carboxy 8–oxo 4–thia 1–azabicyclo [4.2.0.] oct–2–èn–3–yl] méthyl thio] 1–méthyl pyridinium (trifluoroacétate).

On mélange sous atmosphère inerte 100 mg de produit obtenu au stade B précédent, 0,6 cm3 d'acide trifluoroacétique à 10% dans l'eau, agite à 20¤C pendant 1 heure, ajoute 6 cm3 d'éther, agite pendant 10 minutes à 20¤C, isole par essorage le précipité formé, le lave, le sèche et obtient 65 mg de produit recherché. Rf = 0,7 en éluant par le mélange acétone 2–eau 1.

Spectre UV (éthanol)

| | | | |
|---|---|---|---|
| Max. | 222 nm $E_1^1$ | = 403 | $\epsilon$ = 31 500 ; |
| Inflexion | 246 nm $E_1^1$ | = 236 ; | |
| Max. | 308 nm $E_1^1$ | = 250 | $\epsilon$ = 19 500. |

Spectre UV (éthanol + HCl 0,1N)

| | | | |
|---|---|---|---|
| Inflexion | 218 nm $E_1^1$ | = 340 | $\epsilon$ = 26 500 ; |
| Max. | 253 nm $E_1^1$ | = 260 | $\epsilon$ = 20 300 ; |
| Inflexion | 300 nm $E_1^1$ | = 237 ; | |
| Max. | 312 nm $E_1^1$ | = 244 | $\epsilon$ = 19 000. |

**Exemple 15 : Sel interne de 2–[[3–[7–[[(2–amino 4–thiazolyl) (méthoxy imino) acétyl] amino] 2–carboxy 8–oxo 4–thia 1–azabicyclo [4.2.0.] oct–2–èn–3–yl] 2–(e)–propényl] thio] 1–méthyl pyridinium (6S) (7S) (Z).**

**Stade A :** 7–[[2–(2–triphénylméthylaminothiazol–4–yl) 2(Z)méthoxyimino acétyl] amino] 3–(3–iodo) propèn–1(E)–yl] 8–oxo 4–thia 1–azabicyclo [4.2.0.] oct–2–èn–2–carboxylate de (1,1–diméthyl) éthyle (6S) (7S).

On mélange sous atmosphère inerte 184,5 mg de 7–[[2–(2–triphénylméthyl aminothiazol–4–yl) 2–(Z)–méthoxyiminoacétyl] amino]3–(3–hydroxy) propèn–1(E)–yl] 8–oxo 4–thia 1–azabicyclo [4.2.0.] oct–2–èn–2–carboxylate de (1,1–diméthyl) éthyle (6S) (7S), 4 cm3 de chlorure de méthylène, 120 mg 'd'iodure de tétrabutylammonium, 116 µl de 2,6–lutidine, amène le mélange réactionnel à –70¤C, introduit à cette température goutte à goutte, 620 µl d'une solution d'anhydride trifluorométhane sulfonique

dans le chlorure de méthylène titrant 0,609 mmoles/cm3, agite à −70¤C pendant 10 minutes, laisse revenir à 20¤C, élimine le chlorure de méthylène par distillation sous pression réduite et obtient un solide jaune qui correspond au dérivé iodé attendu brut. Ce produit est utilisé tel quel, immédiatement, pour le stade suivant.

**Stade B :** Iodure de 2−[[3−[7−[[(2−triphénylméthylamino 4−thiazolyl) (méthoxyimino) acétyl] amino] 2−terbutoxycarbonyl] 8−oxo 4−thia 1−azabicyclo [4.2.0.] oct−2−èn−3−yl] [2−(E)−propényl] thio] 1−méthyl pyridinium (6S) (7S) (Z).

On mélange sous atmosphère inerte 320 mg de dérivé iodé obtenu au stade A précédent, 7 cm3 d'acétonitrile, 60 mg de 1−méthyl pyridin−2−thione, agite à 20¤C pendant 1 heure et 30 minutes, note une dissolution totale, concentre à sec par distillation sous pression réduite, chromatographie le résidu sur silice en éluant par un mélange de chlorure de méthylène et de méthanol (9/1) et obtient 251 mg de produit attendu. Rf = 0,4 en éluant avec un mélange de chlorure de méthylène et de méthanol (9/1).
$[alpha]_D$ = −120¤ (c = 0,5% chloroforme).

Spectre UV (éthanol)

Inflexion 238 nm $E_1^1$ = 984 ;
Max. 315 nm $E_1^1$ = 238 $\epsilon$ = 23 200 ;

Spectre UV (éthanol + HCl 0,1N)

Inflexion 240 nm $E_1^1$ = 307 ;
Max. 275 nm $E_1^1$ = 251 $\epsilon$ = 24 400 ;
Max. 303 nm $E_1^1$ = 244 $\epsilon$ = 23 700 ;
Inflexion 320 nm $E_1^1$ = 233 $\epsilon$ = 22 700.

**Stade C :** Mélange 1:1 des iodure et trifluoroacétate de 2−[[3−[7−[[(2−amino 4−thiazolyl) (méthoxyimino) acétyl] amino] 2−carboxy] 8−oxo 4−thia 1−azabicyclo [4.2.0.] oct−2−èn−3−yl] [2(E)−(propényl) thio] 1−méthyl pyridinium (6S) (7S) (Z).

On mélange sous atmosphère inerte 218 mg de produit obtenu au stade B précédent, 1,1 cm3 d'acide trifluoroacétique à 10% d'eau, agite à 20¤C pendant 50 minutes, ajoute lentement 5 cm3 d'éther, agite pendant 1 heure jusqu'à concrétisation du precipité, isole par essorage le précipité formé, le lave, le sèche sous vide et obtient 160 mg de produit attendu. Rf = 0,35 en éluant avec un mélange d'acétone et d'eau (2/1).

**Stade D :** Sel interne de 2−[[3−[7−[[(2−amino 4−thiazolyl) (méthoxy imino) acétyl] amino] 2−carboxy 8−oxo 4−thia 1−azabicyclo [4.2.0.] oct−2−èn−3−yl] 2(E)−propényl] thio] 1−méthyl pyridinium (6S) (7S) (Z).

On mélange sous atmosphère inerte 160 mg de produit obtenu au stade C précédent, 1,5 cm3 d'acétonitrile, 1 cm3 de carbonate de triéthylamine, purifie par "H.P.L.C." sur une colonne de 67 cm de longueur et de 27 mm de diamètre (emplissage RP 18). On élue avec des mélanges suivants :

| Acétonitrile % | Eau % |
|---|---|
| 5 | 95 |
| 10 | 90 |
| 15 | 85 |
| 20 | 80 |

réunit les fractions contenant le produit attendu, les concentre à sec et obtient 45,5 mg de produit attendu.
F ≃ 200¤C avec décomposition.
Rf = 0,35 en éluant avec un mélange d'acétone et d'eau (2/1).

Spectre UV (éthanol + HCl 0,1N)

Inflexion 240 nm $E_1^1$ = 337 $\epsilon$ = 18 400 ;
Max. 280 nm $E_1^1$ = 451 $\epsilon$ = 24 650 ;
Inflexion 320 nm $E_1^1$ = 242 $\epsilon$ = 13 600.

**Exemple 16 : Mélange de iodure et de trifluoroacétate de 2-[[[7-[[(2-amino 4-thiazolyl) (méthoxyimino) acétyl amino] 2-carboxy 8-oxo 4-thia 1-azabicyclo [4.2.0.] oct-2-èn-3-yl] méthyl] thio] 1-(carboxy méthyl) pyridinium (6S) (7S) (Z).**

On opère comme au stade 2 de l'exemple 1 à partir de 85 mg de 1-terbutyloxyacétyl pyridin-2-thione et de 300 mg de 7-[[(2-tritylamino 4-thiazolyl) (méthoxyimino) acétyl] amino] 8-oxo 4-thia 1-azabicyclo [4.2.0.] oct-2-èn-3-yl] 3-iodométhyl 2-carboxylate de 1,1-diméthyléthyle et obtient 222 mg d'iodure du produit encore protégé. On traite 204 mg de ce dernier comme en 2 de l'exemple 2 et obtient 45 mg de produit attendu.

Spectre UV (éthanol)

Max. 221 nm $E_1^1$ = 380 $\epsilon$ = 30 700 ;
Max. 305 nm $E_1^1$ = 225 $\epsilon$ = 18 100.

Spectre UV (éthanol + HCl 0,1N)

Max. 220 nm $E_1^1$ = 345 $\epsilon$ = 27 800 ;
Max. 256 nm $E_1^1$ = 263 $\epsilon$ = 21 200 ;
Inflexion 284 nm $E_1^1$ = 230 ;
Inflexion 283 nm $E_1^1$ = 229 $\epsilon$ = 18 500 ;
Inflexion 308 nm $E_1^1$ = 210.

**Exemple 17 : Sel interne de 2-[[[7-[[2-(2-amino 4-thiazolyl) (méthoxy imino) acétyl] amino] 2-carboxy 8-oxo 4-thia 1-azabicyclo [4.2.0.] oct-2-èn-3-yl] méthyl] thio] 1-propyl pyridinium (6S) (7S) (Z).**

On opère comme à l'exemple 16 à partir de 78 mg de 1-propyl pyridin-2-thione et obtient 170 mg de produit que l'on purifie par chromatographie en "H.P.L.C." en le dissolvant dans 0,9 cm3 d'acétonitrile et 0,9 cm3 d'une solution (M) carbonate de triéthylamine et en éluant successivement par des solutions aqueuses à 5, 10, 15 et 20% d'acétonitrile. On lyophilise les fractions intéressantes et obtient 71 mg de produit attendu.

Spectre UV (éthanol)

Inflexion 230 nm $E_1^1$ = 388 ;
Inflexion 249 nm $E_1^1$ = 295 ;
Max. 295 nm $E_1^1$ = 300 $\epsilon$ = 16 500 ;
Inflexion 310 nm $E_1^1$ = 265.

Spectre UV (éthanol + HCl 0,1N)

Inflexion 222 nm $E_1^1$ = 328 ;
Inflexion 266 nm $E_1^1$ = 304 ;
Inflexion 273 nm $E_1^1$ = 306 ;
Max. 284 nm $E_1^1$ = 322 $\epsilon$ = 17 700 ;
Inflexion 290 nm $E_1^1$ = 318 ;
Inflexion 309 nm $E_1^1$ = 278.

**Exemple 18 : Sel interne de 2-[[[7-[[2-(2-amino 4-thiazolyl) (méthoxy imino) acétyl] amino] 2-carboxy 8-oxo 4-thia 1-azabicyclo [4.2.0.] oct-2-èn-3-yl] méthyl] thio] 1-(2-propényl) pyridinium (6S) (7S) (Z).**

On opère comme à l'exemple 17 à partir de 85 mg de 1-(2-propényl) 2-pyridin thione et obtient 60 mg de produit obtenu lyophilisé.

Spectre UV (éthanol)

Inflexion 230 nm $E_1^1$ = 399 ;
Max. 291 nm $E_1^1$ = 313 ε = 17 100 ;
Inflexion 370 nm $E_1^1$ = 35.

Spectre UV (éthanol + HCl 0,1N)

Inflexion 230 nm $E_1^1$ = 309 ;
Inflexion 270 nm $E_1^1$ = 323 ;
Max. 284 nm $E_1^1$ = 345 ε = 19 000 ;
Inflexion 310 nm $E_1^1$ = 264.

**Exemple 19 : Trifluoroacétate de 6-[[[7-[[(2-amino 4-thiazolyl) (méthoxy imino) acétyl] amino] 2-carboxy 8-oxo 4-thia 1-azabicyclo [4.2.0.] oct-2-èn-3-yl] méthyl] thio] 7-méthyl (thiéno/2,3-b) pyridinium (6S) (7S) (Z) trifluoroacétate.**

On opère comme à l'exemple 16 à partir de 7-méthyl thiéno [2,3-b] pyridin-6-thione et obtient 93 mg de produit attendu.

Spectre UV (éthanol)

Max. 239 nm $E_1^1$ = 368 ε = 29 600 ;
Max. 300 nm $E_1^1$ = 201 ε = 16 200 ;
Max. 342 nm $E_1^1$ = 141 ε = 11 400.

Spectre UV (éthanol + HCl 0,1N)

Max. 247 nm $E_1^1$ = 339 ε = 27 300 ;
Max. 286 nm $E_1^1$ = 217 ε = 17 500 ;
Max. 343 nm $E_1^1$ = 150 ε = 12 100.

**Exemple 20 : Sel interne de 2-[[[7-[[2-(2-amino 4-thiazolyl) (méthoxy imino) acétyl] amino] 2-carboxy 8-oxo 4-thia 1-azabicyclo [4.2.0.] oct-2-èn-3-yl] méthyl] thio] 6-méthylthio 1-méthyl pyridinium (6S) (7S) (Z).**

On opère comme à l'exemple 17 à partir de 109 mg de 6-méthylthio 1-méthyl pyridin-2-thione et obtient 85 mg de produit attendu.

Spectre UV (éthanol)

Max. 225 nm $E_1^1$ = 462 ε = 26 200 ;
Inflexion 260 nm $E_1^1$ = 254 ;
Max. 291 nm $E_1^1$ = 273 ε = 15 500 ;
Max. 350 nm $E_1^1$ = 214 ε = 12 100 ;
Inflexion 380 nm $E_1^1$ = 59.

Spectre UV (éthanol + HCl 0,1N)

Max. 225 nm $E_1^1$ = 370 ε = 21 000 ;
Max. 261 nm $E_1^1$ = 327 ε = 18 500 ;
Max. 283 nm $E_1^1$ = 304 ε = 17 200 ;
Max. 350 nm $E_1^1$ = 223 ε = 12 600.

**Exemple 21 : Sel interne de 2−[[[7−[[2−(2−amino 4−thiazolyl) (méthoxy imino) acétyl] amino] 2−carboxy 8−oxo 4−thia 1−azabicyclo [4.2.0.] oct−2−èn−3−yl] méthyl] thio] 1−(méthylthio) méthyl pyridinium (6S) (7S) (Z).**

On opère comme à l'exemple 17 à partir de 73 mg de 1−méthylthiométhyl 2−pyridin thione et obtient 65 mg de produit attendu. F = 200¤ ° C.

Spectre UV (éthanol)

| | | |
|---|---|---|
| Inflexion | 230 nm $E_1^1$ | = 395 ε = 22 400 ; |
| Inflexion | 250 nm $E_1^1$ | = 260 ; |
| Max. | 291 nm $E_1^1$ | = 311 ε = 17 600 ; |
| Inflexion | 330 nm $E_1^1$ | = 140 ; |
| Inflexion | 370 nm $E_1^1$ | = 44. |

Spectre UV (éthanol + HCl 0,1N)

| | | |
|---|---|---|
| Inflexion | 266 nm $E_1^1$ | = 318 ; |
| Inflexion | 275 nm $E_1^1$ | = 333 ; |
| Max. | 285 nm $E_1^1$ | = 350 ε = 19 800 ; |
| Inflexion | 315 nm $E_1^1$ | = 225 ; |
| Inflexion | 370 nm $E_1^1$ | = 45. |

**Exemple 22 : Sel interne de 4−[[[7−[2−(2−amino 4−thiazolyl) (méthoxy imino) acétyl] amino] 2−carboxy 8−oxo 4−thia 1−azabicyclo [4.2.0.] oct−2−èn−3−yl] méthyl] thio] 1−(1−méthyl) éthyle pyridinium (6S) (7S) (Z).**

On opère comme à l'exemple 17 à partir de 76 mg de 1−(1−méthyl) éthyl 4−pyridine thione et obtient 95 mg de produit attendu.

Spectre UV (méthanol)

| | | |
|---|---|---|
| Inflexion | 222 nm $E_1^1$ | = 434 ; |
| Max. | 230 nm $E_1^1$ | = 461 ε = 25 300 ; |
| Inflexion | 296 nm $E_1^1$ | = 441 ; |
| Max. | 306 nm $E_1^1$ | = 477 ε = 26 200 ; |
| Inflexion | 340 nm $E_1^1$ | = 79. |

Spectre UV (éthanol + HCl 0,1N)

| | | |
|---|---|---|
| Max. | 230 nm $E_1^1$ | = 460 ε = 25 200 ; |
| Inflexion | 270 nm $E_1^1$ | = 309 ; |
| Max. | 303 nm $E_1^1$ | = 518 ε = 28 400. |

**Exemple 23 : Iodure de 4−[[[7−[[(2−amino 4−thiazolyl) méthoxyimino) acétyl] amino] 2−carboxy 8−oxo 4−thia 1−azabicyclo [4.2.0.] oct−2−èn−3−yl] méthyl] thio] 6,7−dihydro 1−méthyl 5H 1−pyridinium (6S) (7S) (Z).**

On opère comme à l'exemple 6 à partir de 80 mg de 1−méthyl cyclopentano (b) 4−thiopyridone sans carbonate de potassium. On chromatographie sur silice, élue par un mélange de chlorure de méthylène−méthanol (95/5) puis (90/10) et obtient 370 mg de l'iodure du produit bloqué. On traite 315 mg de ce dernier par 3,15 cm3 d'acide formique à 33% d'eau 8 heures à 50¤ ° C. On filtre, concentre à sec le filtrat sous pression réduite et agite 1 heure le résidu dans 3,15 cm3 d'acétate d'éthyle. On essore et obtient 206 mg de produit attendu.

$[alpha]_D$ = −44¤ ± 1,5¤ (c = 0,6% DMSO)

Spectre UV (éthanol)

Inflexion 214 nm $E_1^1$ = 497 $\epsilon$ = 34 200 ;
Inflexion 231 nm $E_1^1$ = 422 ;
Max. 292 nm $E_1^1$ = 407 $\epsilon$ = 28 000 ;
Inflexion 330 nm $E_1^1$ = 91.

Spectre UV (éthanol + HCl 0,1N)

Max. 219 nm $E_1^1$ = 443 $\epsilon$ = 30 500 ;
Max. 299 nm $E_1^1$ = 427 $\epsilon$ = 29 400.

**Exemple 24 : Mélange de iodure et trifluoroacétate de 2-[[[7-[[(2-amino 4-thiazolyl) (méthoxyimino) acétyl] amino] 2-carboxy 8-oxo 4-thia 1-azabicyclo [4.2.0.] oct-2-èn-3-yl] méthyl] thio] 1-[(méthoxycarbonyl) méthyl] pyridinium (trifluoroacétate) (6S) (7S) (Z).**

On opère comme à l'exemple 16 à partir de 80 mg de 1-[(méthoxy carbonyl) méthyl] pyridin-2-thione et obtient 85 mg de produit attendu.

Spectre UV (éthanol)

Max. 218 nm $E_1^1$ = 342 $\epsilon$ = 28 100 ;
Max. 294 nm $E_1^1$ = 196 $\epsilon$ = 16 100 ;
Inflexion 350 nm $E_1^1$ = 70.

Spectre UV (éthanol + HCl 0,1N)

Max. 218 nm $E_1^1$ = 308 $\epsilon$ = 25 300 ;
Max. 260 nm $E_1^1$ = 232 $\epsilon$ = 19 000 ;
Max. 284 nm $E_1^1$ = 223 $\epsilon$ = 18 300 ;
Inflexion 360 nm $E_1^1$ = 43.

**Exemple 25 : Iodure de 4-[[[7-[[(2-amino 4-thiazolyl) (méthoxyimino) acétyl] amino] 2-carboxy 8-oxo 4-thia 1-azabicyclo [4.2.0.] oct-2-èn-3-yl] méthyl] thio] 1-(2-fluoroéthyl pyridinium (6S) (7S) (Z).**

On opère comme à l'exemple 23 à partir de 1-(2-fluoroéthyl) pyridin 4-thione et obtient 128 mg de produit attendu. F = 210¤C.
$[alpha]_D$ = -39,5¤ ± 1,5¤ (c = 0,6% DMSO)

Spectre UV (éthanol)

Max. 221 nm $E_1^1$ = 493 $\epsilon$ = 33 500 ;
Inflexion 230 nm $E_1^1$ = 450 ;
Max. 309 nm $E_1^1$ = 399 $\epsilon$ = 27 200.

Spectre UV (éthanol + HCl 0,1N)

Max. 222 nm $E_1^1$ = 456 $\epsilon$ = 31 000 ;
Inflexion 270 nm $E_1^1$ = 248 ;
Max. 308 nm $E_1^1$ = 421 $\epsilon$ = 28 600.

**Exemple 26 : Mélange de iodure et trifluoroacétate de 4-[[[7-[[(2-amino 4-thiazolyl) (méthoxyimino) acétyl] amino] 2-carboxy 8-oxo 4-thia 1-azabicyclo [4.2.0.] oct-2-èn-yl] méthyl] thio] 1-(carboxyméthyl) pyridinium trifluoroacétate (6S (7S) (Z).**

On opère comme à l'exemple 24 à partir de 113 mg de 1-[(méthoxy carbonyl) méthyl] pyridin-4-thione et obtient 130 mg de produit attendu.

Spectre UV (éthanol)

Max. 227 nm $E_1^1$ = 379 $\epsilon$ = 30 600 ;
Max. 309 nm $E_1^1$ = 406 $\epsilon$ = 32 700.

Spectre UV (éthanol + HCl 0,1N)

Max. 227 nm $E_1^1$ = 326 $\epsilon$ = 26 300 ;
Max. 264 nm $E_1^1$ = 234 $\epsilon$ = 18 900 ;
Max. 308 nm $E_1^1$ = 393 $\epsilon$ = 31 700.

**Exemple 27 : Iodure de 4–[[[7–[[(2–amino 4–thiazolyl) (méthoxyimino) acétyl] amino] 2–carboxy 8–oxo 4–thia 1–azabicyclo [4.2.0.] oct–2–èn–3–yl] méthyl] thio] 1–[(aminothiocarbonyl) méthyl] pyridinium (6S) (7S) (Z).**

On opère comme à l'exemple 23 à partir de 45 mg de 1–/(amino thio carbonyl) méthyl/ pyridine–4–thione et obtient 116 mg de produit attendu.
$[alpha]_D$ = −60¤ ± 1,5¤ (c = 0,6% DMSO)

Spectre UV (éthanol)

Max. 221 nm $E_1^1$ = 500 $\epsilon$ = 35 400 ;
Max. 265 nm $E_1^1$ = 263 $\epsilon$ = 24 300 ;
Inflexion 301 nm $E_1^1$ = 343 ;
Max. 309 nm $E_1^1$ = 366 $\epsilon$ = 25 900.

Spectre UV (éthanol + HCl 0,1N)

Max. 223 nm $E_1^1$ = 462 $\epsilon$ = 32 700 ;
Max. 269 nm $E_1^1$ = 348 $\epsilon$ = 24 600 ;
Inflexion 302 nm $E_1^1$ = 380 ;
Max. 309 nm $E_1^1$ = 386 $\epsilon$ = 27 300.

**Exemple 28 : Iodure de 4–[[[7–[[(2–amino 4–thiazolyl) (1–carboxy 1–méthyléthoxy) imino] acétyl] amino] 2–carboxy 8–oxo 4–thia 1–azabicyclo [4.2.0.] oct–2–èn–3–yl] méthyl] thio] 1–méthyl pyridinium (6S) (7S) (Z) (trifluoroacétate).**

On opère comme à l'exemple 2 à partir de 150 mg de 7–[[(2–amino 4–thiazolyl) (1–carboxy 1–méthyléthoxy) imino] acétyl] amino] 8–oxo 4–thia 1–azabicyclo [4.2.0.] oct–2–èn–3yl] 3–iodométhyl 2–carboxylate de 1,1–diméthyléthyle et obtient 78 mg de produit attendu.

Spectre UV (éthanol)

Max. 227 nm $E_1^1$ = 362 $\epsilon$ = 30 600 ;
Max. 305 nm $E_1^1$ = 340 $\epsilon$ = 28 800.

Spectre UV (éthanol + HCl 0,1N)

Max. 225 nm $E_1^1$ = 306 $\epsilon$ = 25 900 ;
Inflexion 267 nm $E_1^1$ = 229 ;
Max. 300 nm $E_1^1$ = 357 $\epsilon$ = 30 200.

**Exemple 29 : Iodure de 2–[[[7–[[(2–amino 4–thiazolyl) [(1–carboxy 1–méthyléthoxy) imino acétyl] amino] 2–carboxy 8–oxo 4–thia 1–azabicyclo [4.2.0.] oct–2–èn–3–yl] méthyl] thio] 1–méthyl pyridinium (6S) (7S) (Z) (trifluoroacétate).**

On opère comme à l'exemple 28 à partir de 1–méthyl pyridin–2–thione et obtient 89 mg de produit attendu.

Spectre UV (éthanol + HCl 0,1N)

Inflexion 218 nm $E_1^1$ = 265 $\epsilon$ = 22 400 ;
Max. 255 nm $E_1^1$ = 221 $\epsilon$ = 18 700 ;
Inflexion 267 nm $E_1^1$ = 209 ;
Inflexion 286 nm $E_1^1$ = 207 ;
Max. 294 nm $E_1^1$ = 209 $\epsilon$ = 17 700 ;
Inflexion 307 nm $E_1^1$ = 202.

**Exemple 30 : Trifluoroacétate de 2-[[[7-[[(2-amino 4-thiazolyl) (méthoxy imino) acétyl] amino] 2-carboxy 8-oxo 4-thia 1-azabicyclo [4.2.0.] oct-2-én-3-yl] méthyl] thio] quinolizinium (6S) (7S) (Z) (trifluoroacétate).**

On opère comme à l'exemple 16 à partir de quinolizin-4-thione et obtient 112,6 mg de produit attendu.

Spectre UV (éthanol)

Max. 216 nm $E_1^1$ = 576 $\epsilon$ = 45 000 ;
Inflexion 227 nm $E_1^1$ = 486 ;
Inflexion 240 nm $E_1^1$ = 400 ;
Inflexion 262 nm $E_1^1$ = 311 ;
Max. 298 nm $E_1^1$ = 275 $\epsilon$ = 21 600 ;
Max. 348 nm $E_1^1$ = 290 $\epsilon$ = 22 800.

Spectre UV (éthanol + HCl 0,1N)

Max. 215 nm $E_1^1$ = 520 $\epsilon$ = 40 800 ;
Inflexion 228 nm $E_1^1$ = 420 ;
Inflexion 252 nm $E_1^1$ = 384 ;
Max. 263 nm $E_1^1$ = 399 $\epsilon$ = 31 300 ;
Max. 297 nm $E_1^1$ = 294 $\epsilon$ = 23 100 ;
Max. 348 nm $E_1^1$ = 321 $\epsilon$ = 25 200 ;
Inflexion 288, 308 nm.

**Exemple 31 : Iodure de 4-[[[7-[[(2-amino 4-thiazolyl) (cyclopropyl méthoxyimino) acétyl] amino] 2-carboxy 8-oxo 4-thia 1-azabicyclo [4.2.0.] oct-2-èn-3-yl] méthyl] thio] 1-méthyl pyridinium (trifluoroacétate) (6S) (7S) (Z).**

On opère comme à l'exemple 16 à partir de 200 mg de 7-[[(2-tritylamino 4-thiazolyl) (cyclopropyl méthoxyimino) acétyl] amino] 2-carboxy 8-oxo 4-thia 1-azabicyclo [4.2.0.] oct-2-èn-3-yl] 3-iodométhyl 2-carboxylate de 1,1-diméthyléthyle et de 40 mg de 1-méthyl 4-thiopyridinone et obtient 47 mg de produit attendu.

Spectre UV (éthanol)

Max. 227 nm $E_1^1$ = 404 $\epsilon$ = 32 400 ;
Max. 305 nm $E_1^1$ = 394 $\epsilon$ = 31 600.

Spectre UV (éthanol + HCl 0,1N)

Max. 226 nm $E_1^1$ = 364 $\epsilon$ = 29 200 ;
Inflexion 260 nm $E_1^1$ = 234 ;
Inflexion 274 nm $E_1^1$ = 272 ;
Max. 300 nm $E_1^1$ = 424 $\epsilon$ = 34 000.

**Exemple 32 : Iodure de 4–[[[7–[[(2–amino 4–thiazolyl) (2–bromo 2–propényloxyimino) acétyl] amino] 2–carboxy 8–oxo 4–thia 1–azabicyclo [4.2.0.] oct–2–èn–3–yl] méthyl] thio] 1–méthyl pyridinium (trifluoroacétate) (6S) (7S) (Z).**

On opère comme à l'exemple 31 à partir de 180 mg de 7–[[(2–tritylamino 4–thiazolyl) (2–bromo 2–propényloxyimino) acétyl] amino] 8–oxo 4–thia 1–azabicyclo [4.2.0.] oct–2–èn–3–yl] 3–iodométhyl 2–carboxylate de 1,1–diméthyléthyle et obtient 75 mg de produit attendu.

Spectre UV (éthanol)

Max. 227 nm $E_1^1$ = 354 $\epsilon$ = 30 700 ;
Max. 305 nm $E_1^1$ = 322 $\epsilon$ = 27 900.

Spectre UV (éthanol + HCl 0,1N)

Max. 223 nm $E_1^1$ = 318 $\epsilon$ = 27 600 ;
Inflexion 262 nm $E_1^1$ = 223 ;
Max. 300 nm $E_1^1$ = 335 $\epsilon$ = 29 100.

**Exemple 33 : Mélange 1:1 des iodure et trifluoroacétate de 2–[[[7–[[(2–amino 4–thiazolyl) (fluoroéthoxyimino) acétyl]amino] 2–carboxy 8–oxo 4–thia 1–azabicyclo [4.2.0.] oct–2–èn–3–yl] méthyl] thio] 1–méthyl pyridinium (trifluoroacétate) (6S) (7S) Syn.**

On opère comme à l'exemple 12 à partir de 7–[[(2–tritylamino 4–thiazolyl) (fluoroéthoxyimino) acétyl]amino] 2–carboxy 8–oxo 4–thia 1–azabicyclo [4.2.0.] oct–2–èn–3–yl] 3–iodométhyl 2–carboxylate de 1,1–diméthyléthyle et obtient 81 mg de produit attendu.

Spectre UV (éthanol)

Max. 220 nm $E_1^1$ = 334 $\epsilon$ = 31 400 ;
Max. 309 nm $E_1^1$ = 226 $\epsilon$ = 21 200.

Spectre UV (éthanol + HCl 0,1N)

Inflexion 218 nm $E_1^1$ = 302 ;
Inflexion 250 nm $E_1^1$ = 228 ;
Max. 305 nm $E_1^1$ = 217 $\epsilon$ = 20 400.

**Exemple 34 : Sel interne de 4–[[[7–[[(5–amino 1,2,4–thiadiazol–3–yl) (méthoxyimino) acétyl] amino] 2–carboxy 8–oxo 4–thia 1–azabicyclo [4.2.0.] oct–2–èn–3–yl] méthyl] thio] 1–méthyl pyridinium (6 R,S) (7 R,S) (Z).**

On agite une demi–heure à température ambiante 426mg de 7–[[(5–trityl amino 1,2,4–thiadiazol–3–yl) (méthoxyimino) acétyl] amino] 3–iodométhyl 8–oxo 4–thia 1–azabicyclo [4.2.0.] oct–2–èn–3–yl] 2–carboxylate de 1,1–diméthyléthyle (6S) (7S) (Z) dans 4 cm3 d'acétonitrile sec et 65 mg de 1–méthyl 4–pyridin thione et évapore à sec à 30¤C sous pression réduite. On chromatographie sur silice le résidu, élue avec un mélange de chlorure de méthylène–méthanol (95/5) et obtient 428 mg de produit bloqué. On agite 395 mg de ce dernier avec 7,2 cm3 d'acide trifluoroacétique à 10% d'anisole pendant 10 minutes, filtre, ajoute le filtrat goutte à goutte dans 75 cm3 d'éther isopropylique sous vive agitation et obtient par centrifugation 258,5 mg de produit brut. Après une chromatographie "H.P.L.C." sur lichrosorb, une élution avec un mélange eau–acétonitrile (85/15) puis lyophilisation, on recueille 130 mg de produit attendu.

Spectre UV (éthanol)

Max. 230 nm $E_1^1$ = 485 $\epsilon$ = 25 300 ;
Inflexion 295 nm $E_1^1$ = 411 ;
Max. 305 nm $E_1^1$ = 441 $\epsilon$ = 23 000 ;

Inflexion 340 nm $E_1^1$ = 63.

Spectre UV (éthanol + HCl 0,1N)

Max. 230 nm $E_1^1$ = 522 $\epsilon$ = 27 200 ;
Max. 305 nm $E_1^1$ = 491 $\epsilon$ = 25 600.

**Exemple 35 : Sel interne de 2–[[[7–[[(5–amino 1,2,4–thiadiazol–3–yl) (méthoxyimino) acétyl] amino] 2–carboxy 8–oxo 4–thia 1–azabicyclo [4.2.0.] oct–2–èn–3–yl] méthyl] thio] 1–méthyl pyridinium (6 R,S) (7 R,S) (Z).**

On agite 1 heure à 50¤C 117,8 mg de 7–[[(5–amino 1,2,4–thiadiazol–3–[4.2.0.] oct–2–èn–3–yl] 3–chlorométhyl 2–carboxylate de 1,1–diméthyl éthyle, 54,2 g d'iodure de sodium, 30,2 mg de 1–méthyl 2–pyridin thione et 1,7 cm3 d'acétonitrile sec. On concentre à sec, chromatographie sur silice le résidu, élue avec un mélange chlorure de méthylène–méthanol (75/25) et recueille 127 mg de iodure de 2–[[[7–[[(5–amino 1,2,4–thiadiazol–3–yl) (méthoxyimino) acétyl] amino] 8–oxo 4–thia 1–azabicyclo [4.2.0.] oct–2–èn–3–yl] méthyl] thio] 1–méthyl pyridinium 2–carboxylate de 1,1–diméthyl éthyle. On agite un quart d'heure 126 mg de ce dernier avec 3 cm3 d'acide trifluoroacétique à 10% d'anisole, filtre l'insoluble, ajoute le filtrat goutte à goutte dans 30 cm3 d'éther isopropylique sous vive agitation. On obtient par centrifugation 104 mg de produit brut. On effectue une chromatographie "H.P.L.C." sur lichrosorb, élue avec mélange eau–acétonitrile (85/15), lyophilise et recueille 19 mg de produit attendu.

Spectre UV (éthanol)

Max. 230 nm $E_1^1$ = 464 $\epsilon$ = 24 200 ;
Max. 292 nm $E_1^1$ = 279 $\epsilon$ = 14 600 ;
Inflexion 308 nm $E_1^1$ = 250.

Spectre UV (éthanol + HCl 0,1N)

Max. 231 nm $E_1^1$ = 476 $\epsilon$ = 24 800 ;
Inflexion 296 nm $E_1^1$ = 266 ;
Max. 310 nm $E_1^1$ = 292 $\epsilon$ = 15 200.

**Exemple 36 : Sel interne de 4–[[[7–[[2–(2–amino 4–thiazolyl) 2–(méthoxy imino) acétyl] amino] 2–carboxy 8–oxo 4–thia 1–azabicyclo [4.2.0.] oct–2–èn–3–yl] méthyl] thio] 1–éthyl 3–méthyl pyridinium (6S) (7S) (Z).**

On opère comme à l'exemple 17 à partir de 80 mg de 1–éthyl 3–méthyl 4–pyridine thione et obtient 66 mg de produit attendu.

Spectre UV (éthanol + HCl 0,1N)

Max. 230 nm $E_1^1$ = 425 $\epsilon$ = 23 400 ;
Inflexion 276 nm $E_1^1$ = 368 ;
Max. 300 nm $E_1^1$ = 575 $\epsilon$ = 31 600.

**Exemple 37 : Trifluoroacétate de 2–[[[7–[[(2–amino 4–thiazolyl) (méthoxyimino) acétyl amino] 2–carboxy 8–oxo 4–thia 1–azabicyclo [4.2.0.] oct–2–èn–3–yl] méthyl] thio] 1–méthyl 3,4,5,6–tétrahydropyridinium (trifluoroacétate) (6S) (7S) (Z).**

On opère comme à l'exemple 5 jusqu'au 1 du stade C à partir de 26 mg de N–méthylpyridine 2–thione et obtient 104 mg de produit attendu.

Spectre UV (éthanol)

Max. 220 nm $E_1^1$ = 393 $\epsilon$ = 29 600 ;
Max. 301 nm $E_1^1$ = 180 $\epsilon$ = 13 600.

Spectre UV (éthanol + HCl 0,1N)

Max. 220 nm $E_1^1$ = 349 $\epsilon$ = 26 300 ;
Inflexion 255 nm $E_1^1$ = 183 ;
Max. 291 nm $E_1^1$ = 201 $\epsilon$ = 15 100.

**Exemple 38 : Iodure de 4-[[[7-[[2-(2-amino 4-thiazolyl) 2-(méthoxy imino) acétyl amino] 2-carboxy 8-oxo 4-thia 1-azabicyclo [4.2.0.] oct-2-èn-3-yl] méthyl] thio] 1,3-diméthyl pyridinium (6S) (7S) (Z).**

On opère comme à l'exemple 23 à partir de 39 mg de 1,3-diméthyl 4-thio pyridinone et obtient 116 mg de produit attendu.
$[alpha]_D$ = −22¤ ± 1,5¤ (c = 0,5% DMSO).

Spectre UV (éthanol)

Max. 220 nm $E_1^1$ = 530 $\epsilon$ = 35 100 ;
Max. 302 nm $E_1^1$ = 423 $\epsilon$ = 28 000.

Spectre UV (éthanol + HCl 0,1N)

Max. 221 nm $E_1^1$ = 489 $\epsilon$ = 32 400 ;
Inflexion 264 nm $E_1^1$ = 260 ;
Inflexion 270 nm $E_1^1$ = 282 ;
Max. 299 nm $E_1^1$ = 454 $\epsilon$ = 20 100.

En utilisant les techniques décrites dans les exemples précédents, on a préparé les exemples suivants :

**Exemple 39 : Sel interne de 2-[[[7-[[2-(2-amino 4-thiazolyl) 2-(méthoxy imino) acétyl] amino] 2-carboxy 8-oxo 4-thia 1-azabicyclo [4.2.0.] oct-2-èn-3-yl] méthyl] thio] quinolizinium (6S) (7S) syn.**

Spectre RMN (DMSO) 250 MHz

6,77 ppm : $H_5$ du thiazole
3,82 ppm : H du méthoxyimino

5,44 ppm : $H_7$ } isocéphène
3,90 ppm : $H_6$ }

4,55-4,73 ppm : H du $CH_2$ en 3 en alpha de S
8,83 ppm : $H_1$ de la quinoziline
7,85 à 8,23 ppm : autres protons aromatiques.

**Exemple 40 : (6S) (7S) (Z) Iodure de 2-[[[7-[[2-(2-amino 4-thiazolyl) [[2-[(difluorométhyl) thio] éthoxy] imino] acétyl] amino] 2-carboxy 8-oxo 4-thia 1-azabicyclo [4.2.0.] oct-2-èn-3-yl] méthyl] thio] 1-méthyl pyridinium (trifluoroacétate).**

Spectre UV (EtOH)

Inflexion 217 nm
Max. 302 nm $E_1^1$ = 221 $\epsilon$ = 19 000 ;
Inflexion 360 nm ;

Spectre UV (EtOH + HCl 0,1N)

Inflexion 217 nm ;
Max. 255 nm $E_1^1$ = 225 $\epsilon$ = 19 300 ;
Max. 294 nm $E_1^1$ = 218 $\epsilon$ = 18 700 ;
Inflexion 310 nm.

**Exemple 41 : (6S) (7S) (Z) Iodure de 4-[[[7-[[(2-amino 4-thiazolyl) [(difluorométhoxy) imino] acétyl] amino] 2-carboxy 8-oxo 4-thia 1-azabicyclo [4.2.0.] oct-2-èn-3-yl] méthyl] thio] 1,3-diméthyl pyridinium (trifluoroacétate).**

Spectre UV (EtOH)

Max. 223 nm $E_1^1$ = 392 $\epsilon$ = 31 900 ;
Max. 305 nm $E_1^1$ = 365 $\epsilon$ = 29 700.

Spectre UV (EtOH + HCl 0,1N)

Max. 226 nm $E_1^1$ = 317 $\epsilon$ = 25 800 ;
Max. 257 nm $E_1^1$ = 209 $\epsilon$ = 17 000 ;
Max. 300 nm $E_1^1$ = 384 $\epsilon$ = 31200.

**Exemple 42 : Iodure de 3-[[[7-[[2-(2-amino 4-thiazolyl) 2-(méthoxy imino) acétyl] amino] 2-carboxy 8-oxo 4-thia 1-azabicyclo [4.2.0.] oct-2-èn-3-yl] méthyl] thio] 1-méthyl pyridinium (6S) (7S) (Z).**
F≃220¤C.
$[alpha]_D$ = −17¤ ± 2¤ (c = 0,6% DMSO).

**Exemple 43 : (6S) (7S) (Z) Iodure de 4-[[[7-[[(2-amino 4-thiazolyl) [(fluorométhoxy) imino] acétyl] amino] 2-carboxy 8-oxo 4-thia 1-azabicyclo [4.2.0.] oct-2-èn-3-yl] méthyl] thio] 1,3-diméthyl pyridinium (trifluoroacétate).**

Spectre UV (EtOH)

Max. 226 nm $E_1^1$ = 385 $\epsilon$ = 30 600 ;
Max. 303 nm $E_1^1$ = 357 $\epsilon$ = 28 400.

Spectre UV (EtOH + HCl 0,1N)

Max. 226 nm $E_1^1$ = 312 $\epsilon$ = 24 800 ;
Inflexion 263 nm ;
Max. 299 nm $E_1^1$ = 372 $\epsilon$ = 29 600.

**Exemple 44 : (6S) (7S) (Z) 2-[[[7-[[2-(2-amino 4-thiazolyl) 2-(méthoxy imino) acétyl] amino] 2-carboxy 8-oxo 4-thia 1-azabicyclo [4.2.0.] oct-2-èn-3-yl] méthyl] thio] 5-aminocarbonyl 1-methyl pyridinium.**

F≃190¤C.
$[alpha]_D$ = −17¤ ± 1¤ (c = 0,6% DMSO).

**Exemple 45 : Iodure de 4-[[[7-[[2-(2-amino 4-thiazolyl) 2-(méthoxyimino) acétyl] amino] 2-carboxy 8-oxo 4-thia 1-azabicyclo [4.2.0.] oct-2-èn-3-yl] méthyl] thio] 3-méthyl 1-[-(méthoxycarbonyl) méthyl] pyridinium (6S) (7S) (Z).**

F≃220¤C.
$[alpha]_D$ = −19,5¤ ± 1¤ (c = 0,6% DSMO).

**Exemple 46 : Selinterne de 4-[[[7-[[2-(2-amino 4-thiazolyl) (méthoxy imino) acétyl] amino] 2-carboxy 8-oxo 4-thia 1-azabicyclo [4.2.0.] oct-2-èn-3-yl] méthyl] thio] 1-méthyl 5,6-dihydro pyridinium (6S) (7S) (Z).**

Spectre UV (EtOH)

Inflexion 223 nm ;
Max. 228 nm $E_1^1$ = 427 $\epsilon$ = 32 300 ;
Max. 303 nm $E_1^1$ = 420 $\epsilon$ = 21 900 ;
Inflexion 356 nm.

Spectre UV (EtOH + HCl 0,1N)

Max. 227 nm $E_1^1$ = 357 $\epsilon$ = 18 700 ;
Inflexion 266 nm ;
Inflexion 274 nm ;
Max. 298 nm $E_1^1$ = 457 $\epsilon$ = 23 900.

**Exemple 47 : Sel interne de (6S) (7S) (Z) 4-[[[7-[[2-(2-amino 4-thiazolyl) (méthoxyimino) acétyl] amino] 2-carboxy 8-oxo 4-thia 1-azabicyclo [4.2.0.] oct 2-èn-3-yl] méthyl] thio] 7-méthyl thiéno [2,3-b] pyridinium.**

Spectre UV (EtOH + HCl 0,1N)

Max. 236 nm $E_1^1$ $\epsilon$ = 31 700 ;
Inflexion 247 nm ;
Max. 295 nm $E_1^1$ $\epsilon$ = 19 500 ;
Max. 330 nm $E_1^1$ $\epsilon$ = 20 400 ;
Inflexion 258, 288, 304 nm.

**Exemple 48 : Sel interne de 2-[[[7-[[2-(2-amino 4-thiazolyl) 2-(méthoxy imino) acétyl] amino] 2-carboxy 8-oxo 4-thia 1-azabicyclo [4.2.0.] oct-2-èn-3-yl] méthyl] thio] 1-bromo quinolizinium.**

Spectre UV (EtOH)

Max. 224 nm $E_1^1$ = 591 $\epsilon$ = 37 600 ;
Max. 272 nm $E_1^1$ = 354 $\epsilon$ = 22 500 ;
Max. 294 nm $E_1^1$ = 252 $\epsilon$ = 16 000 ;
Inflexion 301 nm ;
Inflexion 319 nm ;
Max. 353 nm $E_1^1$ = 304 $\epsilon$ = 19 300 ;
Inflexion 360 nm $E_1^1$ = 301.

Spectre UV (EtOH + HCl 0,1N)

Max. 226 nm $E_1^1$ = 529 $\epsilon$ = 33 600 ;
Max. 270 nm $E_1^1$ = 451 $\epsilon$ = 26 700 ;
Max. 320 nm $E_1^1$ = 293 $\epsilon$ = 18 600 ;
Max. 353 nm $E_1^1$ = 354 $\epsilon$ = 22 500 ;
Inflexion 264, 292, 316, 358 nm.

**Exemple 49 : Mélange de iodure et trifluoroacetate de 2-[[[7-[[(2-amino 4-thiazolyl) (méthoxyimino) acétyl] amino] 2-carboxy 8-oxo 4-thia 1-azabicyclo [4.2.0.] oct-2-èn-3-yl] méthyl] thio] pyridino [1,2-a] pyrimidinium (6S) (7S) (Z) (trifluoroacétate).**

Spectre UV (EtOH)

Inflexion 220 nm ;
Inflexion 260 nm ;
Max. 310 nm $E_1^1$ = 316 $\epsilon$ = 25 300.

Spectre UV (EtOH + HCl 0,1N)

Inflexion 220 nm ;
Max. 262 nm $E_1^1$ = 373 $\epsilon$ = 29 800 ;
Max. 290 nm $E_1^1$ = 317 $\epsilon$ = 25 400 ;
Max. 316 nm $E_1^1$ = 311 $\epsilon$ = 24 900.

**Exemple 50 :**

On a réalisé des préparations pour injections de formule :

| | | |
|---|---|---|
| A. | Produit de l'exemple 2<br>Excipient q.s.p. | 500 mg<br>5 cm3 |
| B. | Produit de l'exemple 3<br>Excipient q.s.p. | 500 mg<br>5 cm3 |
| C. | Produit de l'exemple 14<br>Excipient q.s.p. | 500 mg<br>5 cm3. |

**ETUDE PHARMACOLUGIOUE DES PRODUITS DE L'INVENTION**

Activité in vitro, méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de 24 heures à l'étuve à 37¤C, l'inhibition de la croissance est apprécíéd par transillumination ce qui permet de déterminer les concentrtions minimales inhibitrices (C.M.I.) exprimées en ug/cm3.

Les résultats suivants sont obtenus :

| SOUCHES | Prod. Ex. 1 | Prod. Ex. 2 | Prod. Ex.3 | Prod. Ex. 4 | Prod. Ex. 5 | Prod. Ex. 6 |
|---|---|---|---|---|---|---|
| STAPHYLOCOCCUS AUREUS SG511 | 10.000 | 0.300 | 1.200 | 1.200 | 0.600 | 10.000 |
| STAPHYLOCOCCUS AUREUS SG511 S | 5.000 | 0.300 | 0.600 | 2.500 | 1.200 | 20.000 |
| STAPHYLOCOCCUS AUREUS 285 | 10.000 | 0.150 | 0.300 | 0.600 | 0.600 | 10.000 |
| STAPHYLOCOCCUS AUREUS 54146 | 5.000 | 0.300 | 0.600 | 1.200 | 1.200 | 10.000 |
| STREPTOCOCCUS PYOGENES A 561 | <= 0.010 | <= 0.010 | 0.020 | <= 0.010 | 0.040 | 0.020 |
| STREPTOCOCCUS PYOGENES 77 A | 0.080 | 0.020 | <= 0.010 | <= 0.010 | <= 0.010 | 0.020 |
| STREPTOCOCCUS FAECIUM M 78 L | > 20.000 | 2.500 | 5.000 | 2.500 | > 20.000 | 20.000 |
| ESCHERICHIA COLI UC 1894 | 0.080 | <= 0.010 | <= 0.010 | <= 0.010 | <= 0.010 | 0.040 |
| ESCHERICHIA COLI 0 78 | 0.300 | <= 0.010 | <= 0.010 | 0.040 | 0.020 | 0.300 |
| ESCHERICHIA COLI TEM | 0.600 | 0.040 | 0.020 | 0.080 | 0.040 | 0.600 |
| ESCHERICHIA COLI 1507 E | 0.150 | <= 0.010 | <= 0.010 | 0.020 | 0.020 | 0.080 |
| ESCHERICHIA COLI DC 0 | 1.200 | 0.040 | 0.040 | 0.080 | 0.150 | 1.200 |
| ESCHERICHIA COLI DC 2 | 0.300 | <= 0.010 | <= 0.010 | 0.020 | 0.020 | 0.080 |
| SALMONELLA TYPHIMURIUM MZ 11 | 0.300 | 0.020 | <= 0.010 | 0.040 | 0.040 | 0.600 |
| KLEBSIELLA PNEUMONIAE 52145 | 1.200 | 0.150 | 0.020 | 0.080 | 0.040 | 0.600 |
| KLEBSIELLA AEROGENES 1082 E | 20.000 | 0.300 | 0.300 | 1.200 | 1.200 | 20.000 |
| KLEBSIELLA AEROGENES 1522 E | 5.000 | 0.150 | 0.080 | 0.080 | 0.150 | 1.200 |
| ENTEROBACTER CLOACAE P 99 | > 20.000 | 5.000 | 10.000 | > 20.000 | 20.000 | > 20.000 |
| ENTEROBACTER CLOACAE 1321 E | 0.600 | 0.020 | <= 0.010 | 0.020 | 0.040 | 0.600 |
| SERRATIA RG 2532 | 10.000 | 0.300 | 0.300 | 1.200 | 0.600 | 20.000 |
| PROTEUS MIRABILIS A 235 | 0.600 | 0.040 | 0.020 | 0.080 | 0.150 | 0.080 |
| PROTEUS VULGARIS A 232 | 0.600 | 0.040 | 0.020 | 0.080 | 0.300 | 0.300 |
| PROVIDENCIA DU 48 | 20.000 | 1.200 | 0.600 | 1.200 | 1.200 | 2.500 |

| SOUCHES | Prod. Ex. 7 | Prod. Ex. 9 | Prod. Ex. 10 | Prod. Ex. 11 | Prod. Ex. 12 |
|---|---|---|---|---|---|
| STAPHYLOCOCCUS AUREUS SG511 | 10.000 | 0.600 | 1.200 | 0.300 | 0.600 |
| STAPHYLOCOCCUS AUREUS SG511 S | 20.000 | 0.600 | 2.500 | 0.300 | 1.200 |
| STAPHYLOCOCCUS AUREUS 285 | 10.000 | 0.600 | 0.600 | 0.150 | 0.600 |
| STAPHYLOCOCCUS AUREUS 54146 | 10.000 | 0.600 | 1.200 | 0.600 | 1.200 |
| STREPTOCOCCUS PYOGENES A 561 | 0.020 | 0.020 | <= 0.010 | 0.040 | <= 0.010 |
| STREPTOCOCCUS PYOGENES 77 A | 0.080 | <= 0.010 | <= 0.010 | 0.020 | 0.020 |
| STREPTOCOCCUS FAECIUM M 78 L | > 20.000 | 1.200 | 10.000 | 1.200 | 10.000 |
| ESCHERICHIA COLI UC 1894 | 0.040 | 0.020 | 0.020 | <= 0.010 | <= 0.010 |
| ESCHERICHIA COLI O 78 | 0.150 | 0.600 | 0.040 | 0.020 | 0.020 |
| ESCHERICHIA COLI TEM | 0.300 | 0.600 | 0.150 | 0.040 | 0.150 |
| ESCHERICHIA COLI 1507 E | 0.080 | <= 0.010 | 0.020 | <= 0.010 | <= 0.010 |
| ESCHERICHIA COLI DC O | 0.600 | 0.300 | 0.080 | 0.080 | 0.150 |
| ESCHERICHIA COLI DC 2 | 0.080 | 0.600 | 0.020 | <= 3.010 | <= 0.010 |
| SALMONELLA TYPHIMURIUM MZ 11 | 0.150 | 0.600 | 0.080 | 0.020 | 0.040 |
| KLEBSIELLA PNEUMONIAE 52145 | 0.600 | 0.600 | 0.080 | 0.040 | 0.080 |
| KLEBSIELLA AEROGENES 1082 E | 10.000 | 0.600 | 0.600 | 0.600 | 0.300 |
| KLEBSIELLA AEROGENES 1522 E | 1.200 | 0.600 | 0.300 | 0.080 | 0.300 |
| ENTEROBACTER CLOACAE P 99 | > 20.000 | 5.000 | 20.000 | 20.000 | 10.000 |
| ENTEROBACTER CLOACAE 1321 E | 0.300 | 0.150 | 0.150 | 0.040 | 0.040 |
| SERRATIA RG 2532 | 2.500 | 1.200 | 0.300 | 0.150 | 0.600 |
| PROTEUS MIRABILIS A 235 | 0.600 | 0.300 | 0.150 | 0.040 | 0.150 |
| PROTEUS VULGARIS A 232 | 0.600 | 0.300 | 0.150 | 0.080 | 0.080 |
| PROVIDENCIA DU 48 | 10.000 | 2.500 | 1.200 | 0.600 | 2.500 |

| SOUCHES | Prod. Ex. 13 | Prod. Ex. 14 | Prod. Ex. 15 | Prod. Ex. 16 | Prod. Ex. 17 | Prod. Ex. 18 |
|---|---|---|---|---|---|---|
| STAPHYLOCOCCUS AUREUS SG511 | 0.600 | 0.600 | 10.000 | 20.000 | 1.200 | 2.500 |
| STAPHYLOCOCCUS AUREUS SG511 S | 0.600 | 2.500 | 10.000 | 20.000 | 1.200 | 2.500 |
| STAPHYLOCOCCUS AUREUS 285 | 0.150 | 0.300 | 10.000 | 20.000 | 1.200 | 0.600 |
| STAPHYLOCOCCUS AUREUS 54146 | 0.600 | 1.200 | 20.000 | > 20.000 | 2.500 | 2.500 |
| STREPTOCOCCUS PYOGENES A 561 | 0.020 | 0.020 | 0.080 | 0.020 | <= 0.010 | 0.020 |
| STREPTOCOCCUS PYOGENES 77 A | 0.040 | <= 0.010 | 0.040 | 0.080 | <= 0.010 | 0.020 |
| STREPTOCOCCUS FAECIUM M 78 L | 20.000 | 2.500 | > 20.000 | > 20.000 | 5.000 | 10.000 |
| ESCHERICHIA COLI UC 1894 | <= 0.010 | <= 0.010 | 0.300 | 0.020 | <= 0.010 | <= 0.010 |
| ESCHERICHIA COLI O 78 | 0.080 | <= 0.010 | 0.600 | 0.150 | 0.020 | 0.080 |
| ESCHERICHIA COLI TEM | 0.300 | 0.020 | 1.200 | 0.080 | 0.300 | 0.150 |
| ESCHERICHIA COLI 1507 E | <= 0.010 | <= 0.010 | 0.150 | 0.020 | 0.020 | 0.040 |
| ESCHERICHIA COLI DC O | 0.300 | 0.040 | 1.200 | 0.080 | 0.300 | 0.150 |
| ESCHERICHIA COLI DC 2 | <= 0.010 | <= 0.010 | 0.300 | 0.080 | 0.010 | 0.020 |
| SALMONELLA TYPHIMURIUM MZ 11 | 0.150 | 0.040 | 0.600 | 0.040 | 0.040 | 0.080 |
| KLEBSIELLA PNEUMONIAE 52145 | 0.300 | 0.020 | 1.200 | 0.150 | 0.600 | 0.150 |
| KLEBSIELLA AEROGENES 1082 E | 1.200 | 1.200 | 5.000 | 10.000 | 0.600 | 2.500 |
| KLEBSIELLA AEROGENES 1522 E | 0.600 | 0.150 | 1.200 | 0.600 | 0.600 | 0.600 |
| ENTEROBACTER CLOACAE P 99 | 20.000 | 2.500 | > 20.000 | > 20.000 | 20.000 | 20.000 |
| ENTEROBACTER CLOACAE 1321 E | 0.300 | 0.020 | 0.600 | 0.040 | 0.040 | 0.150 |
| SERRATIA RG 2532 | 2.500 | 0.080 | 10.000 | 0.300 | 0.300 | 0.300 |
| PROTEUS MIRABILIS A 235 | 0.300 | 0.040 | 0.600 | 0.040 | 0.150 | 0.150 |
| PROTEUS VULGARIS A 232 | 0.150 | 0.040 | 1.200 | 0.150 | 0.150 | 0.300 |
| PROVIDENCIA DU 48 | 5.000 | 0.600 | 20.000 | 1.200 | 2.500 | 2.500 |

| SOUCHES | Prod. Ex. 19 | Prod. Ex. 20 | Prod. Ex. 21 | Prod. Ex. 22 | Prod. Ex. 23 | Prod. Ex. 24 |
|---|---|---|---|---|---|---|
| STAPHYLOCOCCUS AUREUS SG511 | 1.200 | 0.600 | 2.500 | 0.600 | 0.300 | 20.000 |
| STAPHYLOCOCCUS AUREUS SG511 S | 1.200 | 0.600 | 2.500 | 0.600 | 0.300 | 20.000 |
| STAPHYLOCOCCUS AUREUS 285 | 0.600 | 0.300 | 1.200 | 0.600 | 0.300 | 20.000 |
| STAPHYLOCOCCUS AUREUS 54146 | 1.200 | 0.600 | 2.500 | 1.200 | 0.600 | > 20.000 |
| STREPTOCOCCUS PYOGENES A 561 | 0.020 | 0.020 | <= 0.010 | <= 0.010 | <= 0.010 | 0.080 |
| STREPTOCOCCUS PYOGENES 77 A | 0.020 | 0.020 | <= 0.010 | <= 0.010 | <= 0.010 | 0.040 |
| STREPTOCOCCUS FAECIUM M 78 L | 2.500 | 2.500 | 20.000 | 5.000 | 2.500 | > 20.000 |
| ESCHERICHIA COLI UC 1894 | <= 0.010 | <= 0.010 | <= 0.010 | <= 0.010 | <= 0.010 | 0.040 |
| ESCHERICHIA COLI O 78 | 0.020 | 0.020 | 0.040 | 0.020 | 0.040 | 0.150 |
| ESCHERICHIA COLI TEM | 0.150 | 0.080 | 0.150 | 0.040 | 0.150 | 0.300 |
| ESCHERICHIA COLI 1507 E | <= 0.010 | <= 0.010 | <= 0.010 | <= 0.010 | <= 0.010 | 0.080 |
| ESCHERICHIA COLI DC O | 0.080 | 0.080 | 0.150 | 0.040 | 0.150 | 0.300 |
| ESCHERICHIA COLI DC 2 | 0.020 | <= 0.010 | 0.020 | <= 0.010 | <= 0.010 | 0.080 |
| SALMONELLA TYPHIMURIUM MZ 11 | 0.150 | 0.080 | 0.040 | 0.020 | 0.040 | 0.300 |
| KLEBSIELLA PNEUMONIAE 52145 | 0.080 | 0.080 | 0.150 | 0.040 | 0.300 | 0.150 |
| KLEBSIELLA AEROGENES 1082 E | 2.500 | 0.600 | 1.200 | 0.300 | 1.200 | > 20.000 |
| KLEBSIELLA AEROGENES 1522 E | 0.150 | 0.300 | 0.300 | 0.150 | 0.300 | 0.300 |
| ENTEROBACTER CLOACAE P 99 | 10.000 | 10.000 | 20.000 | 20.000 | 10.000 | > 20.000 |
| ENTEROBACTER CLOACAE 1321 E | 0.080 | 0.080 | 0.080 | 0.020 | 0.080 | 0.600 |
| SERRATIA RG 2532 | 1.250 | 0.600 | 0.300 | 0.600 | 1.200 | 1.200 |
| PROTEUS MIRABILIS A 235 | 0.300 | 0.150 | 0.150 | 0.080 | 0.300 | 0.600 |
| PROTEUS VULGARIS A 232 | 0.300 | 0.300 | 0.150 | 0.080 | 0.150 | 0.300 |
| PROVIDENCIA DU 48 | 2.500 | 1.200 | 5.000 | 1.200 | 2.500 | 2.500 |

| SOUCHES | Prod. Ex. 25 | Prod. Ex. 26 | Prod. Ex. 27 | Prod. Ex. 28 | Prod. Ex. 29 | Prod. Ex. 30 |
|---|---|---|---|---|---|---|
| STAPHYLOCOCCUS AUREUS SG511 | 0.600 | 2.500 | 0.300 | 5.000 | 2.500 | 0.300 |
| STAPHYLOCOCCUS AUREUS SG511 S | 1.200 | 5.000 | 0.300 | 5.000 | 5.000 | 0.300 |
| STAPHYLOCOCCUS AUREUS 285 | 0.600 | 2.500 | 0.150 | 5.000 | 2.500 | 0.300 |
| STAPHYLOCOCCUS AUREUS 54146 | 1.200 | 5.000 | 0.300 | 5.000 | 5.000 | 0.600 |
| STREPTOCOCCUS PYOGENES A 561 | 0.020 | 0.080 | <= 0.010 | 0.300 | 0.300 | <= 0.010 |
| STREPTOCOCCUS PYOGENES 77 A | <= 0.010 | 0.040 | <= 0.010 | 0.300 | 0.300 | <= 0.010 |
| STREPTOCOCCUS FAECIUM M 78 L | 2.500 | 20.000 | 1.250 | > 20.000 | > 20.000 | 2.500 |
| ESCHERICHIA COLI UC 1894 | <= 0.010 | <= 0.010 | <= 0.010 | 0.040 | 0.080 | <= 0.010 |
| ESCHERICHIA COLI O 78 | 0.020 | 0.080 | 0.020 | 0.600 | 0.150 | 0.040 |
| ESCHERICHIA COLI TEM | 0.080 | 0.040 | 0.080 | 0.300 | 0.300 | 0.150 |
| ESCHERICHIA COLI 1507 E | <= 0.010 | 0.020 | <= 0.010 | 0.080 | 0.080 | <= 0.010 |
| ESCHERICHIA COLI DC O | 0.150 | 0.040 | 0.040 | 0.150 | 0.150 | 0.300 |
| ESCHERICHIA COLI DC 2 | <= 0.010 | 0.080 | <= 0.010 | 0.150 | 0.040 | <= 0.010 |
| SALMONELLA TYPHIMURIUM MZ 11 | 0.040 | 0.150 | 0.020 | 0.150 | 0.150 | 0.080 |
| KLEBSIELLA PNEUMONIAE 52145 | 0.080 | 0.020 | 0.040 | 0.300 | 0.150 | 0.150 |
| KLEBSIELLA AEROGENES 1082 E | 5.000 | > 20.000 | 5.000 | 1.200 | 0.600 | 2.500 |
| KLEBSIELLA AEROGENES 1522 E | 0.150 | 0.150 | 0.300 | 0.300 | 0.300 | 0.150 |
| ENTEROBACTER CLOACAE P 99 | > 20.000 | > 20.000 | 10.000 | > 20.000 | > 20.000 | 20.000 |
| ENTEROBACTER CLOACAE 1321 E | 0.150 | 0.080 | 0.020 | 0.150 | 0.150 | 0.080 |
| SERRATIA RG 2532 | 1.200 | 1.200 | 1.200 | 0.600 | 0.600 | 1.200 |
| PROTEUS MIRABILIS A 235 | 0.150 | 0.040 | 0.080 | 0.080 | 0.080 | 0.300 |
| PROTEUS VULGARIS A 232 | 0.300 | 0.600 | 0.600 | 0.020 | 0.040 | 0.300 |
| PROVIDENCIA DU 48 | 1.200 | 1.200 | 1.200 | 2.500 | 2.500 | 1.200 |

| SOUCHES | Prod. Ex. 31 | Prod. Ex. 32 | Prod. Ex. 33 | Prod. Ex. 34 | Prod. Ex. 35 | Prod. Ex. 36 |
|---|---|---|---|---|---|---|
| STAPHYLOCOCCUS AUREUS SG511 | 0.150 | 0.150 | 1.200 | 0.600 | 1.200 | 0.150 |
| STAPHYLOCOCCUS AUREUS SG511 S | 0.300 | 0.300 | 1.200 | 0.600 | 0.600 | 0.300 |
| STAPHYLOCOCCUS AUREUS 285 | 0.150 | 0.150 | 0.600 | 0.600 | 1.200 | 0.150 |
| STAPHYLOCOCCUS AUREUS 54146 | 0.300 | 0.300 | 1.200 | 1.200 | 1.200 | 0.300 |
| STREPTOCOCCUS PYOGENES A 561 | <= 0.010 | 0.020 | 0.040 | 0.020 | 0.020 | <= 0.010 |
| STREPTOCOCCUS PYOGENES 77 A | <= 0.010 | <= 0.010 | 0.040 | <= 0.010 | 0.020 | <= 0.010 |
| STREPTOCOCCUS FAECIUM M 78 L | 20.000 | 5.000 | 20.000 | 5.000 | > 20.000 | 1.200 |
| ESCHERICHIA COLI UC 1894 | 0.020 | 0.040 | <= 0.010 | 0.080 | 0.020 | <= 0.010 |
| ESCHERICHIA COLI O 78 | 0.300 | 0.300 | 0.040 | 0.150 | 0.080 | 0.020 |
| ESCHERICHIA COLI TEM | 0.600 | 1.200 | 0.080 | 0.300 | 0.150 | 0.040 |
| ESCHERICHIA COLI 1507 E | <= 0.010 | <= 0.010 | 0.020 | 0.150 | 0.040 | <= 0.010 |
| ESCHERICHIA COLI DC O | 1.200 | 1.200 | 0.080 | 0.300 | 0.150 | 0.080 |
| ESCHERICHIA COLI DC 2 | <= 0.010 | <= 0.010 | 0.020 | 0.300 | 0.080 | <= 0.010 |
| SALMONELLA TYPHIMURIUM MZ 11 | 0.600 | 0.600 | 0.080 | 0.150 | 0.040 | <= 0.010 |
| KLEBSIELLA PNEUMONIAE 52145 | 1.200 | 2.500 | 0.080 | 0.300 | 0.150 | 0.150 |
| KLEBSIELLA AEROGENES 1082 E | 2.500 | 20.000 | 0.600 | > 20.000 | 10.000 | 1.200 |
| KLEBSIELLA AEROGENES 1522 E | 2.500 | 2.500 | 0.300 | 0.300 | 0.600 | 0.150 |
| ENTEROBACTER CLOACAE P 99 | 20.000 | 20.000 | 10.000 | > 20.000 | > 20.000 | 10.000 |
| ENTEROBACTER CLOACAE 1321 E | 0.300 | 0.300 | 0.080 | 0.600 | 0.300 | 0.040 |
| SERRATIA RG 2532 | 2.500 | 5.000 | 0.600 | 1.200 | 1.200 | 0.300 |
| PROTEUS MIRABILIS A 235 | 0.600 | 0.600 | 0.150 | 1.200 | 0.600 | 0.080 |
| PROTEUS VULGARIS A 232 | 0.600 | 1.200 | 0.150 | 10.000 | 1.200 | 0.150 |
| PROVIDENCIA DU 48 | 5.000 | 5.000 | 2.500 | 2.500 | 2.500 | 1.200 |

| SOUCHES | Prod. Ex. 38 | Prod. Ex. 46 |
|---|---|---|
| STAPHYLOCOCCUS AUREUS SG511 | 0.300 | 0.300 |
| STAPHYLOCOCCUS AUREUS SG511 S | 0.300 | 0.300 |
| STAPHYLOCOCCUS AUREUS 285 | 0.300 | 0.300 |
| STAPHYLOCOCCUS AUREUS 54146 | 0.300 | 0.600 |
| STREPTOCOCCUS PYOGENES A 561 | <= 0.010 | 0.020 |
| STREPTOCOCCUS PYOGENES 77 A | <= 0.010 | 0.020 |
| STREPTOCOCCUS FAECIUM M 78 L | 1.200 | 1.200 |
| ESCHERICHIA COLI UC 1894 | <= 0.010 | ≤ 0.010 |
| ESCHERICHIA COLI O 78 | 0.020 | 0.020 |
| ESCHERICHIA COLI TEM | 0.080 | 0.080 |
| ESCHERICHIA COLI 1507 E | <= 0.010 | ≤ 0.010 |
| ESCHERICHIA COLI DC 0 | 0.150 | 0.080 |
| ESCHERICHIA COLI DC 2 | <= 0.010 | ≤ 0.010 |
| SALMONELLA TYPHIMURIUM MZ 11 | 0.080 | 0.040 |
| KLEBSIELLA PNEUMONIAE 52145 | 0.150 | 0.080 |
| KLEBSIELLA AEROGENES 1082 E | 5.000 | 5.000 |
| KLEBSIELLA AEROGENES 1522 E | 0.300 | 0.150 |
| ENTEROBACTER CLOACAE P 99 | 20.000 | 10.000 |
| ENTEROBACTER CLOACAE 1321 E | 0.040 | 0.080 |
| SERRATIA RG 2532 | 0.600 | 0.300 |
| PROTEUS MIRABILIS A 235 | 0.080 | 0.080 |
| PROTEUS VULGARIS A 232 | 0.300 | 0.150 |
| PROVIDENCIA DU 48 | 1.200 | 1.200 |

## Revendications

### Revendications pour les Etats contractants suivants : AT, BE, CH, DE, FR, GB, IT, LI, NL, SE

**1.** Les produits répondant à la formule générale (I')

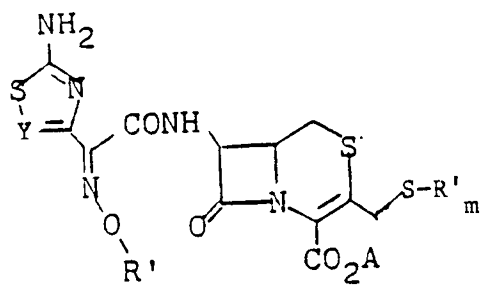

(I')

isomère syn

dans laquelle R' représente un atome d'hydrogène ou un radical alkyle, alkényle, alkynyle ou cycloakyle ayant au plus 6 atomes de carbone, éventuellement substitué par les radicaux halogène, cyano, carbamoyle, nitro, amino, hydroxy, mercapto, alkylthio, oxo, alkoxy, carboxyle libre, estérifié ou salifié,

$R'_m$ représente soit un radical pyridinium éventuellement substitué par un ou plusieurs radicaux choisis parmi les radicaux alkyle ayant de 1 à 4 atomes de carbone, méthoxy, éthoxy, les atomes d'halogènes, les radicaux carboxy alkyle éventuellement estérifiés, le radical carbamoyle, les radicaux thioacétylal-kyle, thiocarbamoylalkyle, haloalkyle, cyanoalkyle, alkenyle ayant au plus 4 atomes de carbone, alkylthio alkyle et thioalkyle, ledit radical pyridinium pouvant éventuellement être partiellement hydrogéné

soit un radical quinoléinyle, isoquinoléinyle, cinnolinyle, quinazolinyle, pyridino [1,2-a] pyrimidinyle

soit un radical choisi parmi les radicaux :

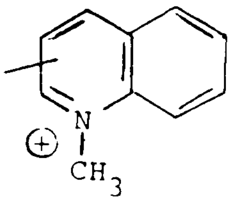 ; 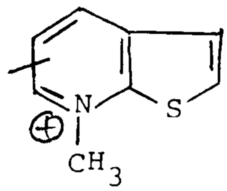 ; 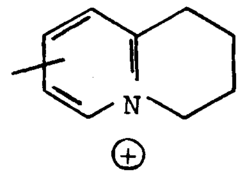 ; 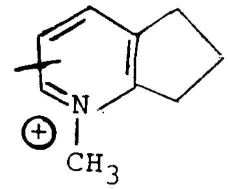

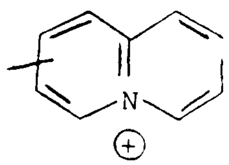

ces radicaux étant liés à l'atome de soufre par un atome de carbone, Y représente un radical méthine ou un atome d'azote,
A représente un atome d'hydrogène, un équivalent de métal alcalin, alcalino-terreux, de magnésium, d'ammonium ou d'une base organique aminée ou A représente un groupement ester ou $CO_2A$ représente un groupement $CO_2^-$, aussi que les sels des produits de formule I' avec les acides minéraux ou organiques.

**2.** Les produits tels que définis à la revendication 1 et répondant à la formule (I") :

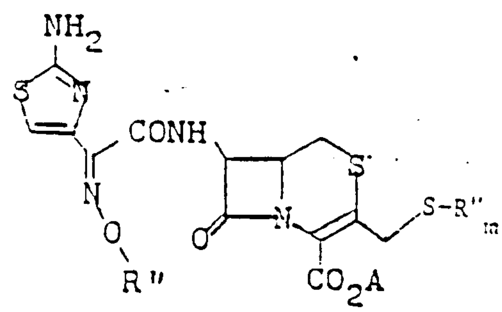

(I")

dans laquelle R" représente un radical méthyle, fluorométhyle ou difluorométhyle, $R"_m$ représente un radical

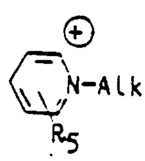

dans lequel Alk représente un radial alkyle ayant de 1 à 4 atomes de carbone, $R_5$ représente un atome d'hydrogène, un radical alkyle ou alkylhio ayant de 1 à 4 atomes de carbone, ledit radical $R"_m$ étant lié à l'atome de soufre par les positions 2 ou 4 et A a la signification indiquée à la revendication 1.

**3.** Les produits tels que définis à l'une des revendications 1 ou 2 dont les noms suivent :
- Le (6S) (7S) (Z) 4-[[[7-[[(2-amino 4-thiazolyl) [(difluorométhoxy) imino] acétyl amino] 2-carboxy 8-oxo 4-thia 1-azabicyclo [4.2.0.] oct-2-èn-3-yl] méthyl] thio] 1-méthyl pyridinium,
- Le (6S) (7S) (Z) 4-[[[7-[[(2-amino 4-thiazolyl) [fluorométhoxy) imino] acétyl] amino] 2-carboxy 8-oxo 4-thia 1-azabicyclo [4.2.0.] oct-2-èn-3-yl] méthyl] thio] 1-méthyl pyridinium,

- Le (6S) (7S) (Z) 2 – [[[7 – [[(2 – amino 4 – thiazolyl) [fluorométhoxy) imino] acétyl] amino] 2 – carboxy 8 – oxo 4 – thia 1 – azabicyclo [4.2.0.] oct – 2 – èn – 3 – yl] méthyl] thio] 1 – méthyl pyridi – nium,
- Le 4 – [[7 – [[2 – (2 – amino 4 – thiazolyl 2 – (méthoxyimino) acétyl] amino] 2 – carboxy 8 – oxo 4 – thia 1 – azabicyclo [4.2.0.] oct – 2 – èn – 3 – yl] méthyl] thio] 1 – éthyl 3 – méthyl pyridinium (6S) (7S) (Z),
- Le 4 – [[[7 – [[2 – (2 – amino 4 – thiazolyl 2 – (méthoxyimino) acétyl] amino] 2 – carboxy 8 – oxo 4 – thia 1 – azabicyclo [4.2.0.] oct – 2 – èn – 3 – yl] méthyl] thio] 1,3 – diméthyl pyridinium (6S) (7S) (Z),
- Le 4 – [[[7 – [[2 – (2 – amino 4 – thiazolyl) (méthoxyimino) acétyl] amino] 2 – carboxy 8 – oxo 4 – thia 1 – azabicyclo [4.2.0.] oct – 2 – èn – 3 – yl] méthyl] thio] 1 – méthyl pyridinium (6S) (7S) (Z),
- Le 4 – [[[7 – [[2 – (2 – amino 4 – thiazolyl) (méthoxyimino) acétyl] amino] 2 – carboxy 8 – oxo 4 – thia 1 – azabicyclo [4.2.0.] oct – 2 – èn – 3 – yl] méthyl] thio] 1 – méthyl 5,6 – dihydro pyridinium (6S) (7S) (Z),

sous forme de sel interne ou de sels avec les acides minéraux ou organiques.

**4.** Le produit tel que défini à l'une des revendications 1 à 3 dont le nom suit :

- Le (6S) (7S) (Z) 4 – [[[7 – [[(2 – amino 4 – thiazolyl [(difluorométhoxy) imino] acétyl amino] 2 – carboxy 8 – oxo 4 – thia 1 – azabicyclo [4.2.0.] oct – 2 – èn – 3 – yl] méthyl] thio] 1 – méthyl pyridi – nium, sous forme de sel interne ou de sels avec les acides minéraux ou organiques.

**5.** Procédé de préparation des produits de formule générale (I') telle que définie ci – dessus caractérisé en ce que l'on traite un produit de formule (VI') :

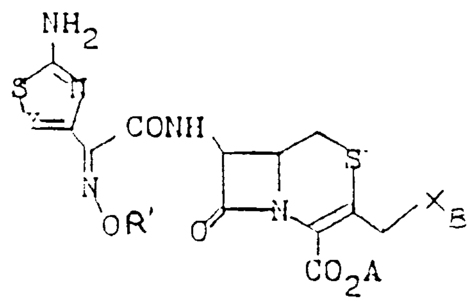

(VI')

dans laquelle Y, A et R' ont la signification indiquée à la revendication 1 et $X_B$ représente un atome d'halogène ou un pseudo halogène par un produit de formule VIII :

$R_{AM}$ CS (VIII)

dans laquelle $R_{AM}$ représente le reste du radical $R'_m$ dans lequel l'atome d'azote est lié par des liaisons simples et non polaires et soumet le produit obtenu si nécessaire ou si désiré, à l'une quelconque ou à plusieurs des réactions suivantes, dans un ordre quelconque :

a) coupure par hydrolyse, hydrogénolyse ou par action de la thiourée de tout ou partie du ou des groupements protecteurs;
b) estérification ou salification par une base du ou des groupements carboxy ou sulfo;
c) salification par un acide du ou des groupements amino;
d) dédoublement de la molécule pour obtenir un produit optiquement actif;
e) oxydation de l'atome de soufre en position 2 du cycle isocéphème.

**6.** A titre de médicaments, les produits de formule (I') telle que définie à la revendication 1, ainsi que leurs sels d'acides pharmaceutiquement acceptables.

**7.** A titre de médicaments, les produits de formule (I") telle que définie à l'une des revendications 2 à 4, ainsi que leurs sels d'acides pharmaceutiquement acceptables.

**8.** Les compositions pharmaceutiques contenant, à titre de principe actif, l'un au moins des médicaments selon l'une des revendications 6 ou 7.

**Revendications pour l'Etat contractant suivant : ES**

**1.** Procédé de préparation des produits répondant à la formule générale (I')

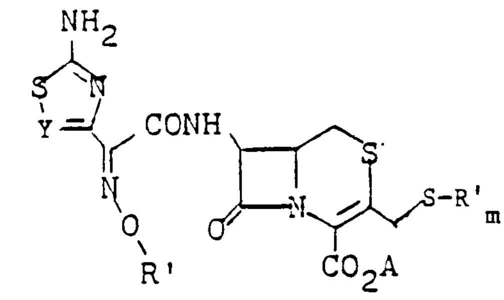

(I')

isomère syn

dans laquelle R' représente un atome d'hydrogène ou un radical alkyle, alkényle, alkynyle ou cycloakyle ayant au plus 6 atomes de carbone, éventuellement substitué par les radicaux halogène, cyano, carbamoyle, nitro, amino, hydroxy, mercapto, alkylthio, oxo, alkoxy, carboxyle libre, estérifié ou salifié,

$R'_m$ représente soit un radical pyridinium éventuellement substitué par un ou plusieurs radicaux choisis parni les radicaux alkyle ayant de 1 à 4 atomes de carbone, méthoxy, éthoxy, les atomes d'halogènes, les radicaux carboxy alkyle éventuellement estérifiés, le radical carbamoyle, les radicaux thioacétylalkyle, thiocarbamoylalkyle, haloalkyle, cyanoalkyle, alkenyle ayant au plus 4 atomes de carbone, alkylthio alkyle et thioalkyle, ledit radical pyridinium pouvant éventuellement être partiellement hydrogéné

soit un radical quinoléinyle, isoquinoléinyle, cinnolinyle, quinazolinyle, pyridino [1,2-a] pyrimidinyle

soit un radical choisi parmi les radicaux :

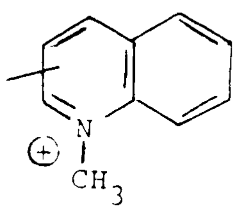 ; 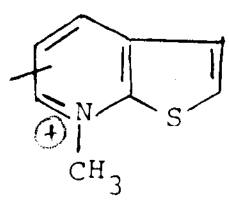 ; 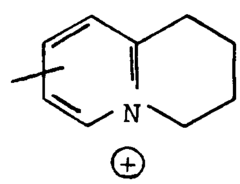 ; 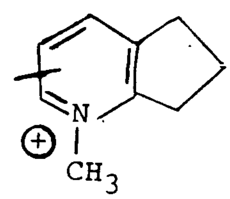

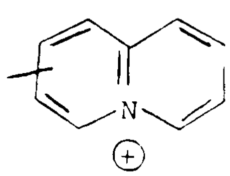

ces radicaux étant liés à l'atome de soufre par un atome de carbone, Y représente un radical méthine ou un atome d'azote,

A représente un atome d'hydrogène, un équivalent de métal alcalin, alcalino-terreux, de magnésium, d'ammonium ou d'une base organique aminée ou A représente un groupement ester ou $CO_2A$ représente un groupement $CO_2^-$, aussi que les sels des produits de formule I' avec les acides minéraux ou organiques, caractérisé en ce que l'on traite un produit de formule (VI'):

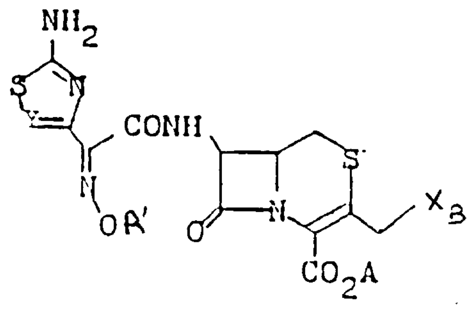

(VI')

dans laquelle Y, A et R' ont la signification indiquée précédemment et $X_B$ représente un atome d'halogène ou un pseudo halogène par un produit de formule VIII :

$R_{AM}$ CS (VIII)

dans laquelle $R_{AM}$ représente le reste du radical $R'_m$ dans lequel l'atome d'azote est lié par des liaisons simples et non polaires et soumet le produit obtenu si nécessaire ou si désiré, à l'une quelconque ou à plusieurs des réactions suivantes, dans un ordre quelconque :

a) coupure par hydrolyse, hydrogénolyse ou par action de la thiourée de tout ou partie du ou des groupements protecteurs ;
b) estérification ou salification par une base du ou des groupements carboxy ou sulfo;
c) salification par un acide du ou des groupements amino;
d) dédoublement de la molécule pour obtenir un produit optiquement actif;
e) oxydation de l'atome de soufre en position 2 du cycle isocéphème.

**2.** Procédé selon la revendication 1 pour la préparation des produits, répondant à la formule (I") :

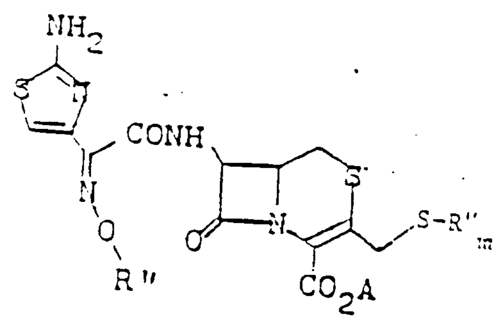

(I")

dans laquelle R" représente un radical méthyle, fluorométhyle ou difluorométhyle, $R''_m$ représente un radical

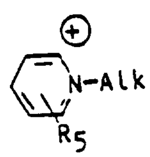

dans lequel Alk représente un radical alkyle ayant de 1 à 4 atomes de carbone, $R_5$ représente un atome d'hydrogène, un radical alkyle ou alkylthio ayant de 1 à 4 atomes de carbone, ledit radial $R''_m$ étant lié à l'atome de soufre par les positions 2 ou 4 et A a la signification indiquée à la revendication 1, caractérisé en ce que l'on traite un produit de formule (VI') dans laquelle A et X3 ont les significations indiquées à la revendication 1 et R' à la valeur de R", R" représentant un radical méthyle, fluorométhyle ou difluorométhyle, par un produit de formule (VIII) dans laquelle $R_{AM}$ représente le reste du radical $R''_m$ défini comme précédemment, radical dans lequel l'atome d'azote est lié par des liaisons simples et non polaires.

## Claims

**Claims for the following Contracting States : AT, BE, CH, DE, FR, GB, IT, LI, NL, SE**

**1.** The products corresponding to general formula (I'):

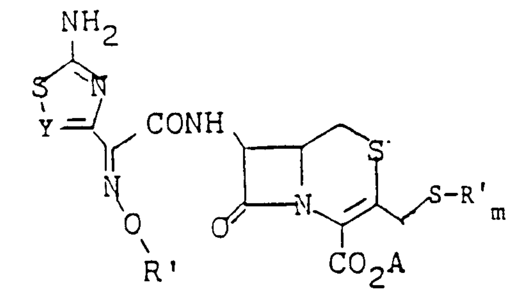

(I')

syn isomer
in which R' represents a hydrogen atom or an alkyl, alkenyl, alkynyl or cycloalkyl radical having at most 6 carbon atoms, optionally substituted by the following radicals: halogen, cyano, carbamoyl, nitro, amino, hydroxy, mercapto, alkylthio, oxo, alkoxy, free, esterified or salified carboxyl,
$R'_m$ represents either a pyridinium radical optionally substituted by one or more radicals chosen from alkyl radicals having 1 to 4 carbon atoms, methoxy, ethoxy radicals, halogen atoms, optionally esterified alkyl carboxy radicals, the carbamoyl radical, the following radicals: thioacetylalkyl, thiocarbamoylalkyl, haloalkyl, cyanoalkyl, alkenyl having at most 4 carbon atoms, alkylthio alkyl and thioalkyl, the said pyridinium radical optionally being able to be partially hydrogenated
or a quinolinyl, isoquinolinyl, cinnolinyl, quinazolinyl, pyridino [1,2-a] pyrimidinyl radical
or a radical chosen from the radicals:

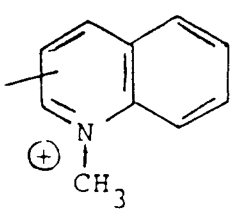 ; 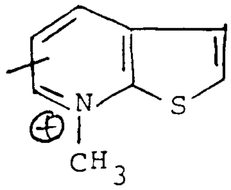 ; 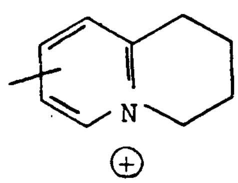 ; 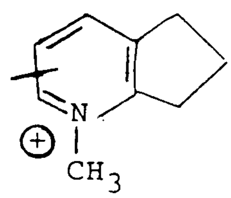

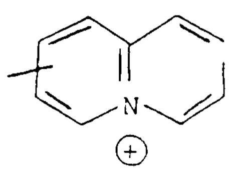

these radicals being linked to the sulphur atom by a carbon atom, Y represents a methine radical or a nitrogen atom,
A represents a hydrogen atom, an equivalent of an alkali or alkaline-earth metal, of magnesium, ammonium or of an amino organic base or A represents an ester group or $CO_2A$ represents a $CO_2^-$ group, as well as the salts of the products of formula I' with mineral or organic acids.

**2.** The products as defined in claim 1 and corresponding to formula (I"):

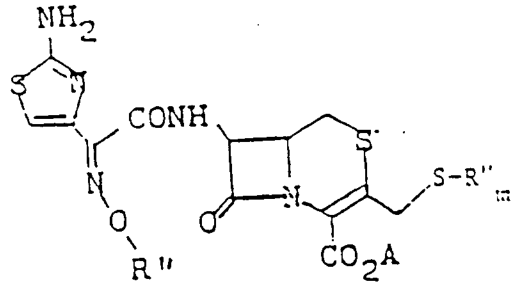

(I")

in which R" represents a methyl fluoromethyl or difluoromethyl radical, $R"_m$ represents a

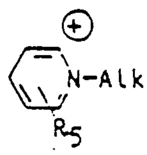

radical in which Alk represents an alkyl radical having 1 to 4 carbon atoms, $R_5$ represents a hydrogen atom, an alkyl or alkylthio radical having 1 to 4 carbon atoms, the said $R"_m$ radical being linked to the sulphur atom by positions 2 or 4 and A has the meaning indicated in claim 1.

**3.** The products as defined in one of claims 1 or 2 of which the names follow:

- (6S) (7S) (Z) 4-[[[7-[[(2-amino 4-thiazolyl) [(difluoromethoxy) imino] acetyl amino] 2-carboxy 8-oxo 4-thia 1-azabicyclo [4.2.0] oct-2-en-3-yl] methyl] thio] 1-methyl pyridinium,
- (6S) (7S) (Z) 4-[[[7-[[(2-amino 4-thiazolyl) (fluoromethoxy) imino] acetyl] amino] 2-carboxy 8-oxo 4-thia 1-azabicyclo [4.2.0.] oct-2-en-3-yl] methyl] thio] 1-methyl pyridinium,
- (6S) (7S) (Z) 2-[[[7-[[(2-amino 4-thiazolyl) (fluoromethoxy) imino] acetyl] amino] 2-carboxy 8-oxo 4-thia 1-azabicyclo [4.2.0.] oct-2-en-3-yl] methyl] thio] 1-methyl pyridinium,
- 4-[[7-[[2-(2-amino 4-thiazolyl 2-(methoxyimino) acetyl] amino] 2-carboxy 8-oxo 4-thia 1-azabicyclo [4.2.0.] oct-2-en-3-yl] methyl] thio] 1-ethyl 3-methyl pyridinium (6S) (7S) (Z),
- 4-[[[7-[[2-(2-amino 4-thiazolyl 2-(methoxyimino) acetyl] amino] 2-carboxy 8-oxo 4-thia 1-azabicyclo [4.2.0.] oct-2-en-3-yl] methyl] thio] 1,3-dimethyl pyridinium (6S) (7S) (Z),
- 4-[[[7-[[2-(2-amino 4-thiazolyl) (methoxyimino) acetyl] amino] 2-carboxy 8-oxo 4-thia 1-azabicyclo [4.2.0.] oct-2-en-3-yl] methyl] thio] 1-methyl pyridinium (6S) (7S) (Z),
- 4-[[[7-[[2-(2-amino 4-thiazolyl) (methoxyimino) acetyl] amino] 2-carboxy 8-oxo 4-thia 1-azabicyclo [4.2.0.] oct-2-en-3-yl] methyl] thio] 1-methyl 5,6-dihydro pyridinium (6S) (7S) (Z),

in the form of the internal salt or of salts with mineral or organic acids.

**4.** The product as defined in one of claims 1 to 3 of which the name follows:

- (6S) (7S) (Z) 4-[[[7-[[(2-amino-4-thiazolyl-[(difluoromethoxy) imino] acetyl amino] 2-carboxy 8-oxo 4-thia 1-azabicyclo [4.2.0.] oct-2-en-3-yl] methyl] thio] 1-methyl pyridinium, in the form of the internal salt or of salts with mineral or organic acids.

**5.** Preparation process for the products of general formula (I') as defined above characterized in that a product of formula (VI'):

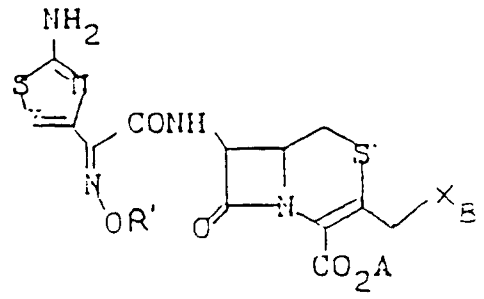

(VI')

in which Y, A and R' have the meaning indicated in claim 1 and $X_B$ represents a halogen atom or a pseudo halogen, is treated with a product of formula (VIII):

$R_{AM}$ CS (VIII)

in which $R_{AM}$ represents the remainder of the $R'_m$ radical in which the nitrogen atom is linked by single and non polar bonds, and the product obtained is subjected if necessary or if desired to any one or to several of the following reactions, in any order:

a) cutting by hydrolysis, hydrogenolysis or by the action of thiourea of all or part of the protective group or groups;
b) esterification or salification of the carboxy or sulpho groups by a base;
c) salification of the amino group or groups by an acid;
d) resolution of the molecule to obtain an optically active product;
e) oxidation of the sulphur atom in position 2 of the isocepheme ring.

**6.** As medicaments, the products of formula (I') as defined in claim 1, as well as their pharmaceutically acceptable acid salts.

**7.** As medicaments, the products of formula (I") as defined in one of claims 2 to 4, as well as their pharmaceutically acceptable acid salts.

**8.** The pharmaceutical compositions containing, as active ingredient, at least one of the medicaments according to one of claims 6 or 7.

**Claims for the following Contracting State : ES**

**1.** Preparation process for the products corresponding to general formula (I'):

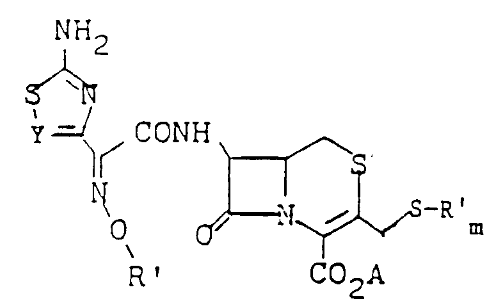

(I')

syn isomer
in which R' represents a hydrogen atom or an alkyl, alkenyl, alkynyl or cycloalkyl radical having at most 6 carbon atoms, optionally substituted by the following radicals: halogen, cyano, carbamoyl, nitro, amino, hydroxy, mercapto, alkylthio, oxo, alkoxy, free, esterified or salified carboxyl,
$R'_m$ represents either a pyridinium radical optionally substituted by one or more radicals chosen from alkyl radicals having 1 to 4 carbon atoms, methoxy, ethoxy radicals, halogen atoms, optionally esterified

alkyl carboxy radicals, the carbamoyl radical, the following radicals: thioacetylalkyl, thiocarbamoylalkyl, haloalkyl, cyanoalkyl, alkenyl having at most 4 carbon atoms, alkylthio alkyl and thioalkyl, the said pyridinium radical optionally being able to be partially hydrogenated
or a quinolinyl, isoquinolinyl, cinnolinyl, quinazolinyl, pyridino [1,2 – a] pyrimidinyl radical
or a radical chosen from the radicals:

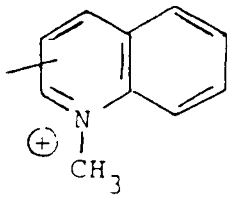 ; 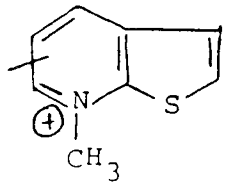 ; 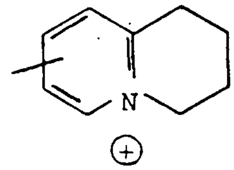 ; 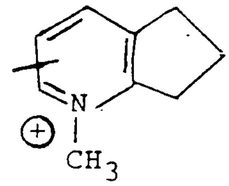

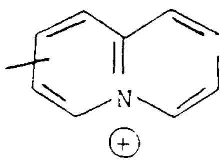

these radicals being linked to the sulphur atom by a carbon atom, Y represents a methine radical or a nitrogen atom,
A represents a hydrogen atom, an equivalent of an alkali or alkaline – earth metal, of magnesium, ammonium or of an amino organic base or A represents an ester group or $CO_2A$ represents a $CO_2^-$ group, as well as the salts of the products of formula I' with mineral or organic acids, characterized in that a product of formula (VI'):

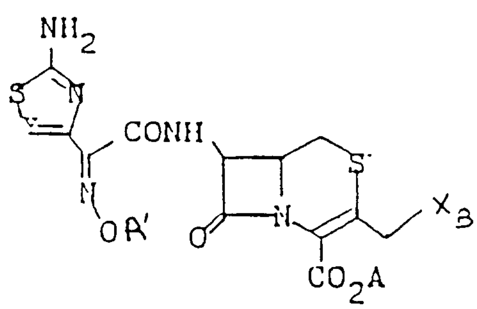

(VI')

in which Y, A and R' have the meaning indicated previously and $X_B$ represents a halogen atom or a pseudo halogen, is treated with a product of formula (VIII):

$R_{AM}$ CS (VIII)

in which $R_{AM}$ represents the remainder of the $R'_m$ radical in which the nitrogen atom is linked by single and non polar bonds, and the product obtained is subjected if necessary or if desired to any one or to several of the following reactions, in any order:

a) cutting by hydrolysis, hydrogenolysis or by the action of thiourea of all or part of the protective group or groups;
b) esterification or salification of the carboxy or sulpho groups by a base;
c) salification of the amino group or groups by an acid;
d) resolution of the molecule to obtain an optically active product;
e) oxidation of the sulphur atom in position 2 of the isocepheme ring.

2. Process according to claim 1 for the preparation of products corresponding to formula (I"):

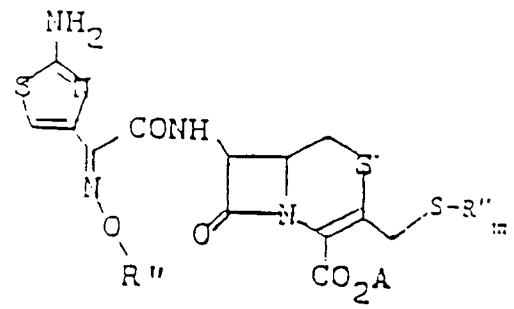

(I")

in which R" represents a methyl fluoromethyl or difluoromethyl radical, $R"_m$ represents a

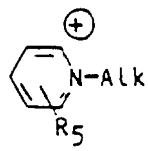

radical in which Alk represents an alkyl radical having 1 to 4 carbon atoms, $R_5$ represents a hydrogen atom, an alkyl or alkylthio radical having 1 to 4 carbon atoms, the said $R"_m$ radicalbeing linked to the sulphur atom by positions 2 or 4 and A has the meaning indicated in claim 1, characterized in that a product of formula (VI') in which A and X3 have the meanings indicated in claim 1 and R' has the value of R", R" representing a methyl, fluoromethyl or difluoromethyl radical, is treated with a product of formula (VIII) in which $R_{AM}$ represents the remainder of the $R"_m$ radical defined as previously, in which radical the nitrogen atom is linked by single and non polar bonds.

**Patentansprüche**
**Patentansprüche für folgende Vertragsstaaten : AT, BE, CH, DE, FR, GB, IT, LI, NL, SE**

1. Produkte der allgemeinen Formel (I')

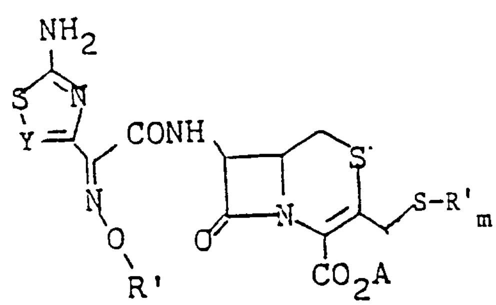

(I')

syn – Isomer,
worin R' ein Wasserstoffatom oder einen Alkyl –, Alkenyl –, Alkinyl – oder Cycloalkylrest mit maximal 6 Kohlenstoffatomen darstellt, der eventuell mit einer Halogen –, Cyano –, Carbamoyl –, Nitro –, Amino – , Hydroxy –, Mercapto –, Alkylthio –, Oxo – oder Alkoxygruppe oder mit einer freien, veresterten oder in die Salzform überführten Carboxylgruppe substituiert ist,
$R'_m$ entweder eine Pyridiniumgruppe darstellt, die eventuell mit einer oder mehreren Gruppen substi – tuiert ist, die ausgewählt werden unter Alkylgruppen mit 1 bis 4 Kohlenstoffatomen, Methoxy – und Ethoxygruppen, Halogenatomen, eventuell veresterte Carboxyalkylgruppen, Carbamoyl –, Thioacetylalkyl –, Thiocarbamoylalkyl –, Haloalkyl – und Cyanoalkylgruppen, Alkenylgruppen mit ma – ximal 4 Kohlenstoffatomen und Alkylthioalkyl – und Thioalkylgruppen, wobei die Pyridiniumgruppe eventuell partiell hydriert sein kann,

oder eine Chinolinyl‑, Isochinolinyl‑, Cinnolinyl‑, Chinazolinyl‑, Pyridino[1,2‑a]‑pyrimidinylgruppe oder eine unter den folgenden Gruppen ausgewählte Gruppe darstellt:

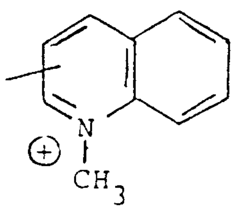 ; 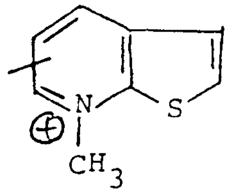 ; 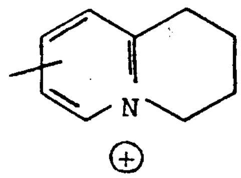 ; 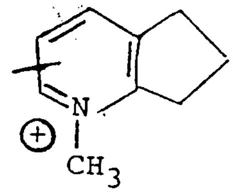

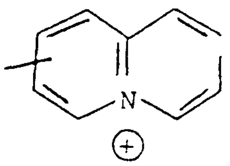

wobei diese Gruppen über ein Kohlenstoffatom an das Schwefelatom gebunden sind, Y eine Methingruppe oder ein Stickstoffatom darstellt,
A ein Wasserstoffatom, ein Äquivalent eines Alkalimetalls, eines Erdalkalimetalls, von Magnesium, von Ammonium oder einer organischen Aminbase oder eine Estergruppe, wobei $CO_2A$ auch eine $CO_2^-$‑Gruppe darstellen kann, und die Salze der Produkte der Formel I' mit anorganischen oder organischen Säuren.

**2.** Produkte nach Anspruch 1 der Formel (I"):

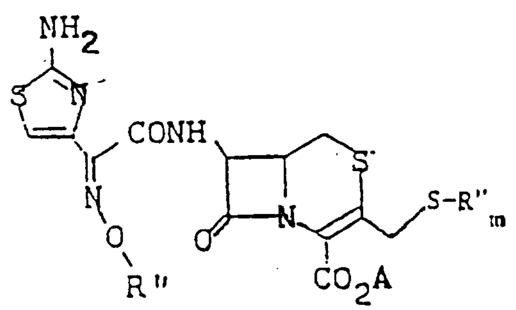

(I")

und R" eine Methyl‑, Fluormethyl‑ oder Difluormethylgruppe und $R"_m$ eine Gruppe

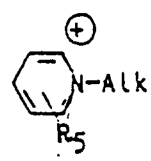

bedeutet, worin Alk eine Alkylgruppe mit 1 bis 4 Kohlenstoffatomen, $R_5$ ein Wasserstoffatom oder eine Alkyl‑ oder Alkylthiogruppe mit 1 bis 4 Kohlenstoffatomen bedeuten, wobei die Gruppe $R"_m$ über die Positionen 2 oder 4 an das Schwefelatom gebunden ist und A die in Anspruch 1 angegebene Bedeutung besitzt.

**3.** Produkte nach einem der Ansprüche 1 oder 2 mit den folgenden Bezeichnungen:
- (6S) (7S) (Z) 4‑[[[7‑[2‑(2‑Amino‑4‑thiazolyl)‑2‑(difluormethoximino)‑acetamido]‑2‑carboxy‑8‑oxo‑4‑thia‑1‑azabicyclo [4.2.0.]oct‑2‑en‑3‑yl]‑methyl]‑thio]‑1‑methylpyridinium,

- (6S) (7S) (Z) 4-[[[7-[2-(2-Amino-4-thiazolyl)-2-(fluormethoximino)-acetamido]-2-carboxy-8-oxo-4-thia-1-azabicyclo [4.2.0.]oct-2-en-3-yl]-methyl]-thio]-1-methylpyridinium,
- (6S) (7S) (Z) 2-[[[7-[2-(2-Amino-4-thiazolyl)-2-(fluormethoximino)-acetamido]-2-carboxy-8-oxo-4-thia-1-azabicyclo [4.2.0.]oct-2-en-3-yl]-methyl]-thio]-1-methylpyridinium,
- (6S) (7S) (Z) 4-[[[7-[2-(2-Amino-4-thiazolyl)-2-(methoximino)-acetamido]-2-carboxy-8-oxo-4-thia-1-azabicyclo [4.2.0.]oct-2-en-3-yl]-methyl]-thio]-1-ethyl-3-methylpyridinium,
- (6S) (7S) (Z) 4-[[[7-[2-(2-Amino-4-thiazolyl)-2-(methoximino)-acetamido]-2-carboxy-8-oxo-4-thia-1-azabicyclo [4.2.0.]oct-2-en-3-yl]-methyl]-thio]-1,3-dimethylpyridinium,
- (6S) (7S) (Z) 4-[[[7-[2-(2-Amino-4-thiazolyl)-2-(methoximino)-acetamido]-2-carboxy-8-oxo-4-thia-1-azabicyclo [4.2.0-]oct-2-en-3-yl]-methyl]-thio]-1-methylpyridinium,
- (6S) (7S) (Z) 4-[[[7-[2-(2-Amino-4-thiazolyl)-2-(methoximino)-acetamido]-2-carboxy-8-oxo-4-thia-1-azabicyclo [4.2.0.]oct-2-en-3-yl]-methyl]-thio]-1-methyl-5,6-dihydropyridinium,

in Form des inneren Salzes oder der Salze mit organischen oder anorganischen Säuren.

**4.** Produkt nach einem der Ansprüche 1 bis 3, mit der folgenden Bezeichnung:
- (6S) (7S) (Z) 4-[[[7-[2-(2-Amino-4-thiazolyl)-2-difluormethoximinoacetamido]-2-carboxy-8-oxo-4-thia-1-azabicyclo [4.2.0.]oct-2-en-3-yl]-methyl]-thio]-1-methylpyridinium

in Form des inneren Salzes oder der Salze mit anorganischen oder organischen Säuren.

**5.** Verfahren zur Herstellung von Produkten der allgemeinen Formel (I'), wie oben definiert, dadurch gekennzeichnet, daß man ein Produkt der Formel (VI')

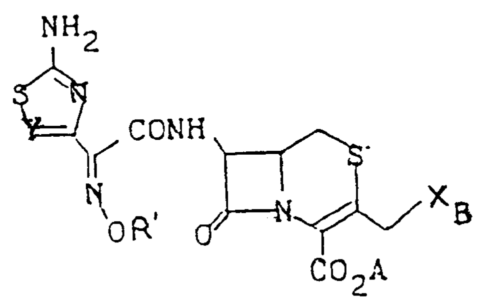

(VI')

worin Y, A und R' die in Anspruch 1 angegebene Bedeutung besitzen und $X_B$ ein Halogenatom oder ein Pseudohalogen bedeutet, mit einem Produkt der Formel VIII:

$R_{AM}$ CS (VIII)

behandelt, worin $R_{AM}$ den Rest der Gruppe $R'_m$ darstellt, worin das Stickstoffatom über nichtpolare Einfachbindungen gebunden ist, und das erhaltene Produkt, wenn nötig oder wenn gewünscht, einer oder mehrerer der folgenden Reaktionen in irgendeiner Reihenfolge unterzieht:

a) teilweise oder vollständige Abspaltung der Schutzgruppe(n) durch Hydrolyse, Hydrogenolyse oder Einwirkung von Thioharnstoff,
b) Veresterung oder Überführung in das Salz der Carboxyoder Sulfogruppe(n) mit einer Base,
c) Überführung der Aminogruppe(n) in das Salz mit einer Säure,
d) Trennung des Moleküls, um ein optisch aktives Produkt zu erhalten,
e) Oxidation des Schwefelatoms in Position 2 des Isocephemcyclus.

**6.** Produkte der Formel (I) nach Anspruch 1, sowie ihre pharmazeutisch akzeptablen Salze als Medikamente.

7. Produkte der Formel (I") nach einem der Ansprüche 2 bis 4, sowie ihre pharmazeutische akzeptablen Salze als Medikamente.

8. Pharmazeutische Zusammensetzungen, die als Wirkstoff mindestens eines der Medikamente nach einem der Ansprüche 6 oder 7 enthalten.

**Patentansprüche für folgenden Vertragsstaat : ES**

1. Verfahren zur Herstellung von Produkten der allgemeinen Formel (I')

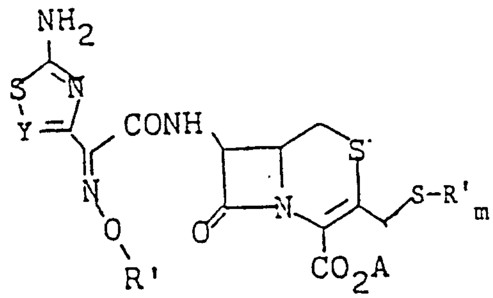

(I')

syn – Isomer,
worin R' ein Wasserstoffatom oder einen Alkyl – , Alkenyl – , Alkinyl – oder Cycloalkylrest mit maximal 6 Kohlenstoffatomen darstellt, der eventuell mit einer Halogen – , Cyano – , Carbamoyl – , Nitro – , Amino – , Hydroxy – , Mercapto – , Alkylthio – , Oxo – oder Alkoxygruppe oder mit einer freien, veresterten oder in die Salzform überführten Carboxylgruppe substituiert ist,
$R'_m$ entweder eine Pyridiniumgruppe darstellt, die eventuell mit einer oder mehreren Gruppen substi – tuiert ist, die ausgewählt werden unter Alkylgruppen mit 1 bis 4 Kohlenstoffatomen, Methoxy – und Ethoxygruppen, Halogenatomen, eventuell veresterte Carboxyalkylgruppen, Carbamoyl – , Thioacetylalkyl – , Thiocarbamoylalkyl – , Haloalkyl – und Cyanoalkylgruppen, Alkenylgruppen mit ma – ximal 4 Kohlenstoffatomen und Alkylthioalkyl – und Thioalkylgruppen, wobei die Pyridiniumgruppe eventuell partiell hydriert sein kann,
oder eine Chinolinyl – , Isochinolinyl – , Cinnolinyl – , Chinazolinyl – , Pyridino[1,2 – a] – pyrimidinylgruppe oder eine unter den folgenden Gruppen ausgewählte Gruppe darstellt:

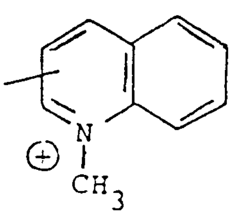 ; 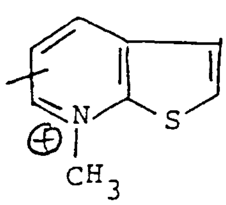 ; 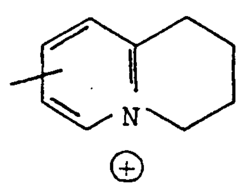 ; 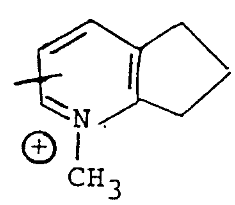

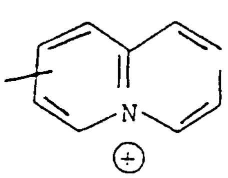

wobei diese Gruppen über ein Kohlenstoffatom an das Schwefelatom gebunden sind, Y eine Methin – gruppe oder ein Stickstoffatom darstellt,
A ein Wasserstoffatom, ein Äquivalent eines Alkalimetalls, eines Erdalkalimetalls, von Magnesium, von Ammonium oder einer organischen Aminbase oder eine Estergruppe, wobei $CO_2A$ auch eine $CO_2^-$ – Gruppe darstellen kann, und die Salze der Produkte der Formel I' mit anorganischen oder organischen Säuren,
dadurch gekennzeichnet, daß man ein Produkt der Formel (VI')

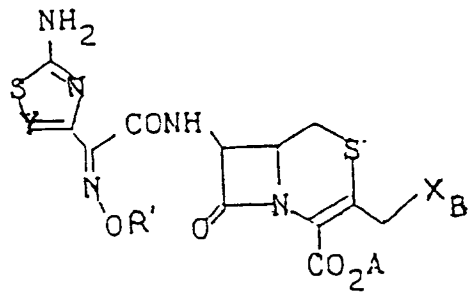

(VI')

worin Y, A und R' die vorher angegebene Bedeutung besitzen und $X_B$ ein Halogenatom oder ein Pseudohalogen bedeutet, mit einem Produkt der Formel VIII:

$R_{AM}$ CS (VIII)

behandelt, worin $R_{AM}$ den Rest der Gruppe $R'_m$ darstellt, worin das Stickstoffatom über nichtpolare Einfachbindungen gebunden ist, und das erhaltene Produkt, wenn nötig oder wenn gewünscht, einer oder mehrerer der folgenden Reaktionen in irgendeiner Reihenfolge unterzieht:

a) teilweise oder vollständige Abspaltung der Schutzgruppe(n) durch Hydrolyse, Hydrogenolyse oder Einwirkung von Thioharnstoff,
b) Veresterung oder Überführung in das Salz der Carboxy- oder Sulfogruppe(n) mit einer Base,
c) Überführung der Aminogruppe(n) in das Salz mit einer Säure,
d) Trennung des Moleküls, um ein optisch aktives Produkt zu erhalten,
e) Oxidation des Schwefelatoms in Position 2 des Isocephemcyclus.

**2.** Verfahren nach Anspruch 1 zur Herstellung von Produkten der Formel (I"):

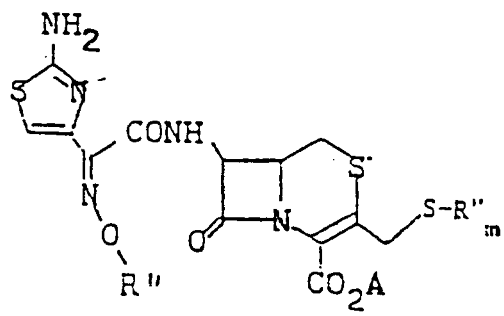

(I")

worin R" eine Methyl-, Fluormethyl- oder Difluormethylgruppe und $R''_m$ eine Gruppe

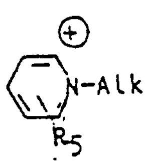

bedeutet, worin Alk eine Alkylgruppe mit 1 bis 4 Kohlenstoffatomen, $R_5$ ein Wasserstoffatom oder eine Alkyl- oder Alkylthiogruppe mit 1 bis 4 Kohlenstoffatomen bedeuten, wobei die Gruppe $R''_m$ über die Positionen 2 oder 4 an das Schwefelatom gebunden ist und A die in Anspruch 1 angegebene Bedeutung besitzt, dadurch gekennzeichnet, daß man ein Produkt der Formel VI', worin A und $X_B$ die in Anspruch 1 angegebene Bedeutung besitzen und R' R" bedeutet, wobei R" eine Methylfluormethyl- oder Difluormethylgruppe ist, mit einem Produkt der Formel VIII behandelt, worin $R_{AM}$ den Rest der Gruppe $R''_m$ wie vorher definiert darstellt, worin das Stickstoffatom über nichtpolare Einfachbindungen gebunden ist.